# EUROPEAN PATENT APPLICATION

(11) **EP 2 264 026 A1**
(43) Date of publication of application: **22.12.2010**
(21) Application number: 09718266.1
(22) Date of filing: 04.03.2009
(51) Int. Cl.: C07D 401/14, A61K 31/4545, A61K 31/46, A61K 31/5377, A61K 31/5386, A61K 31/541, A61K 31/55, A61P 3/04, A61P 3/10, A61P 43/00, C07D 413/14, C07D 417/14, C07D 451/02, C07D 498/08

(54) **ALKYLAMINOPYRIDINE DERIVATIVE**

(30) Priority: 06.03.2008 JP 2008055861
(71) Applicant: Banyu Pharmaceutical Co., Ltd., Chiyoda-ku Tokyo 102-8667 (JP)
(72) Inventor: ANDO, Makoto, Tsukuba-shi Ibaraki 300-2611 (JP); ITO, Hirokatsu, Tsukuba-shi Ibaraki 300-2611 (JP); KAMEDA, Minoru, Tsukuba-shi Ibaraki 300-2611 (JP); KAWAMOTO, Hiroshi, Tsukuba-shi Ibaraki 300-2611 (JP); KOBAYASHI, Kensuke, Tsukuba-shi Ibaraki 300-2611 (JP); MIYAZOE, Hiroshi, Tsukuba-shi Ibaraki 300-2611 (JP); NAKAMA, Chisato, Tsukuba-shi Ibaraki 300-2611 (JP); SATO, Nagaaki, Tsukuba-shi Ibaraki 300-2611 (JP); TSUJINO, ToshiakI, Tsukuba-shi Ibaraki 300-2611 (JP)
(74) Representative: Buchan, Gavin MacNicol
(86) International application number: PCT/JP2009/054077
(87) International publication number: WO 2009/110510

(57) **Abstract**

The present invention relates to a compound that is useful for treatment of, for example, hypertension, arteriosclerosis, bulimia and obesity because of having an antagonistic action to a neuropeptide Y receptor and is represented by formula (I) 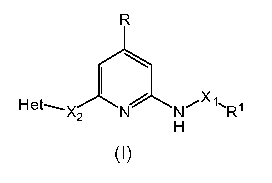 [wherein R¹ represents hydrogen, cyano, or the like;
R represents a group represented by formula (II); 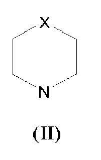 X₁ represents C₁₋₄ lower alkylene or the like; X₂ represents lower alkylene or the like; and Het represents a 5-membered heteroaromatic ring that has at least one nitrogen atom and, in addition, one or two hetero atoms selected from the group consisting of nitrogen, sulfur and oxygen atoms]
or to a pharmaceutically acceptable salt thereof.

## Description

### TECHNICAL FIELD

The present invention relates to neuropeptide Y receptor antagonists which contain an alkylaminopyridine derivative as an active ingredient. Furthermore, the present invention relates to a novel alkylaminopyridine derivative.

### BACKGROUND Art

Neuropeptide Y (hereinafter referred to as NPY), which is a peptide containing 36 amino acids, was first isolated in 1982 from porcine brain by Tatemoto et al. (for example, see Non-Patent Document 1). NPY is widely distributed in the central and peripheral nervous systems and has a variety of *in vivo* actions as one of the peptides most abundantly present in the nervous system. That is, in the central nervous system, NPY acts as an aperitive and significantly promotes a fat accumulation via secretion of various hormones and actions of the nervous system. A continuous intracerebroventricular administration of NPY has been known to induce obesity and insulin resistance based on the above actions. NPY is also associated with emotional control and the actions of the central autonomic nervous system. In addition, in the peripheral nervous system, NPY is present together with norepinephrine in the sympathetic nerve ending and associated with the tonicity of the sympathetic nervous system. A peripheral administration of NPY has been known to cause vasoconstriction and enhance actions of other vasoconstrictors including norepinephrine (for example, see Non-Patent Documents 2, 3, 4 and 5). The action of NPY is expressed when it is bonded to an NPY receptor present in the central or peripheral nervous system. Therefore, inhibition of the binding of NPY to the NPY receptor allows prevention of the expression of the action of NPY. Consequently, compounds antagonizing the binding of NPY to the NPY receptor can be expected to be useful in the prevention or treatment of various diseases associated with NPY, for example, cardiovascular diseases such as hypertension, arteriosclerosis, nephropathy, cardiac diseases and angiospasm; diseases of central nervous system such as bulimia, depression, epilepsy, anxiety, alcoholism and dementia; metabolic diseases such as obesity, diabetes mellitus and hormone abnormality, or glaucoma (for example, see Non-Patent Document 6).
In addition, derivatives structurally similar to NPY are described to be bonded to an NPY receptor and antagonize the activity of NPY (for example, see Patent Documents 1 and 2 and Non-Patent Document 7).
In addition, certain peptides have been recently found to inhibit binding of NPY to an NPY receptor (see Patent Documents 3 and 4).

However, such peptidic compounds have big problems in developing the compounds as medicaments. That is, such high-molecular-weight peptides generally have *in vivo* instability and are also short-acting.
Furthermore, such compounds are included in a compound group that can be hardly expected to have oral absorption and brain penetrability..

In contrast, certain nonpeptidic compounds have been recently found to inhibit binding of NPY to an NPY receptor and antagonize the activity of NPY (for example, see Patent Documents 5 and 6).

However, such nonpeptidic NPY antagonists are structurally different from a compound according to the present invention completely and give no suggestion to the present invention.

As a compound associated with an alkylaminopyridine derivative according to the present invention, for example, a compound represented by 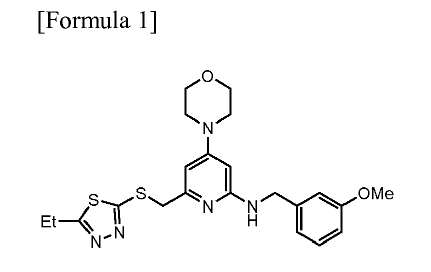 is disclosed in Patent Document 7.

However, phenyl substituted with methoxy is bonded to methylamino bonded to a pyridine ring in the compound disclosed in Patent Document 7, whereas trifluoromethyl is bonded to methylamino of a compound according to the present invention, which has no substituted phenyl.

For example, a compound represented by 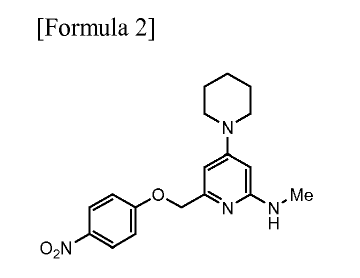 is disclosed in Patent Document 8. The above compound has nitrophenoxymethyl, whereas a compound according to the present invention has no nitrophenoxymethyl. Furthermore, the above compound has no substituent on methyl of methylamino. In addition, Patent Document 8 discloses the compound, which relates to an NPY Y5 antagonist, but does not describe nor suggest that the compound has NPY-Y1 antagonist activity.
[Patent Document 1] European Patent No. 355794
[Patent Document 2] Danish Patent No. 3811193
[Patent Document 3] WO 94/00486
[Patent Document 4] Japanese Patent Laid-Open No. 6-116284
[Patent Document 5] Japanese Patent Laid-Open No. 6-293794
[Patent Document 6] German Patent DE4301452-A1
[Patent Document 7] WO 97/34873
[Patent Document 8] WO 98/40356
[Non-Patent Document 1] Nature, vol. 296, p.659 (1982)
[Non-Patent Document 2] International Journal of Obesity, vol.19, p.517 (1995)
[Non-Patent Document 3] Endocrinology, vol.133, p.1753 (1993)
[Non-Patent Document 4] British Journal of Pharmacology, vol.95, p.419 (1988)
[Non-Patent Document 5] Frontiers in Neuroendocrinology, vol. 27, p.308 (2006)
[Non-Patent Document 6] Trends in Pharmacological Sciences, vol.15, p.153 (1994)
[Non-Patent Document 7] J. Med. Chem., vol. 37, p. 811 (1994)

### DISCLOSURE OF INVENTION

### PROBLEM TO BE SOLVED BY THE INVENTION

An object of the present invention is to provide a novel compound having an antagonistic action to NPY.

### MEANS FOR SOLVING THE PROBLEMS

As a result of extensive research, the present inventors found a novel alkylaminopyridine compound and the invention was thus accomplished. Specifically, the present invention relates to a compound represented by the formula (I) 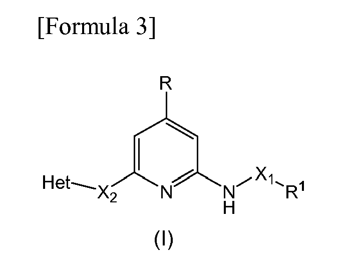 or a pharmaceutically acceptable salt thereof, wherein:
R¹ represents a group selected from the group consisting of:
   hydrogen,
   cyano,
   trifluoromethyl,
   halogen,
   alkoxycarbonyl,
   alkoxycarbonylamino,
   lower alkylsulfonyl,
   lower alkylsulfanilyl,
   lower alkoxy, and
   hydroxy;
R is a group represented by formula (II): 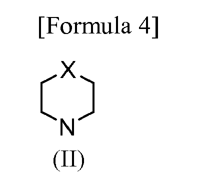
wherein X represents O, S or SO₂, or
a group represented by formula (III): 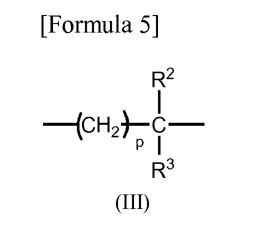 wherein R² and R³ are same or different hydrogen or halogen atoms; and p is an integer of 0 or 1, and
the group represented by the formula (II) is optionally crosslinked via a methylene or ethylene chain;
said R is optionally substituted with a group selected from the group consisting of: phenyl,
lower alkyl,
lower alkoxy,
hydroxy, and
halogen;
X₁ represents lower (C₁₋₄) alkylene,
said lower alkylene is optionally substituted with one or two same or different lower alkyl groups,
when the two same or different lower alkyl groups are bonded to the same carbon atom in the lower alkylene, these may together form a 3-7 membered cycloalkyl group, and when R¹ is lower alkoxycarbonylamino, nitrogen constituting the lower alkoxycarbonylamino and X₁ may together form a 5- or 6-membered aliphatic ring containing nitrogen;
X₂ represents C₂₋₆ lower alkylene, and one of carbon atoms constituting said lower alkylene is optionally substituted with an oxygen or sulfur atom, and the lower alkylene may be substituted with hydroxy;
Het represents a 5-membered heteroaromatic ring which has at least one nitrogen atom and, in addition, one or two hetero atoms selected from the group constituting of nitrogen, sulfur and oxygen, and
when said Het group is substituted with one or two same or different lower alkyl optionally substituted with halogen or lower alkoxy, or cycloalkyl groups, or when said Het is substituted with two alkyl groups, the two alkyl groups together may form 5-7 membered cycloalkyl.

### EFFECTS OF THE INVENTION

Since the compound (I) according to the present invention has an antagonistic action to NPY, it is useful for treatment and/or prevention of various diseases associated with NPY, for example, cardiovascular diseases such as hypertension, arteriosclerosis, nephropathy, cardiac diseases and angiospasm; diseases of central nervous system such as bulimia, depression, epilepsy, anxiety, alcoholism and dementia; metabolic diseases such as obesity, diabetes mellitus and hormone abnormality, or glaucoma.

Since the compound (I) according to the present invention has low affinity for human ERG (also referred to as hERG) compared to aminopyridine derivatives in related art, it is also more useful as a medicament.

### BEST MODE FOR CARRYING OUT THE INVENTION

The meanings of terms as used herein are described below, and a compound according to the present invention is described in further detail.

The term "lower alkyl" means linear or branched C₁₋₆ alkyl and includes, for example, methyl, ethyl, propyl, isopropyl, butyl, isobutyl, sec-butyl, tert-butyl, pentyl, isoamyl, neopentyl, isopentyl, 1,1-dimethylpropyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,2,2-trimethylpropyl and 1-ethyl-2-methylpropyl.

The term "lower alkoxy" means a group, in which a hydrogen atom of hydroxy is substituted with the lower alkyl, and includes, for examples, methoxy, ethoxy, propoxy, isopropoxy, butoxy, sec-butoxy, tert-butoxy, pentyloxy, isopentyloxy, hexyloxy and isohexyloxy.

The term "halogen atom" includes, for example, fluorine, chlorine, bromine and iodine atoms.

The term "alkoxycarbonyl" means a group in which the lower alkoxy and carbonyl are bonded and includes, for example, methoxycarbonyl, ethoxycarbonyl and propoxycarbonyl.

The term "lower alkylsulfonyl" means a group in which the lower alkyl and sulfonyl are bonded and includes, for example, methylsulfonyl, ethylsulfonyl, propylsulfonyl and isopropylsulfonyl.

The term "lower alkylsulfanilyl" means a group in which the lower alkyl and sulfanilyl are bonded and includes, for example, methylsulfanilyl, ethylsulfanilyl, propylsulfanilyl and isopropylsulfanilyl.

The term "alkoxycarbonylamino" means a group in which the alkoxycarbonyl and amino are bonded and includes, for example, methoxycarbonylamino, ethoxycarbonylamino and propoxycarbonylamino.

The term "cycloalkyl" means a cycloalkyl group having 3 to 8 carbon atoms and means cyclopropyl, cyclobutyl, cyclopentyl, cyclohexyl, cycloheptyl and cyclooctyl.

In order to further specifically disclose a compound represented by the formula (I) in accordance with the present invention 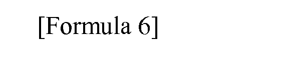 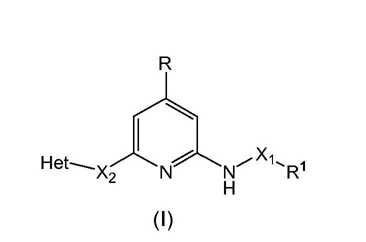 (wherein each symbol has the same definition specified above)
each symbol used in the formula (I) is described referring to specific examples.

R¹ means a group selected from the group consisting of hydrogen, cyano, trifluoromethyl, halogen, alkoxycarbonyl, alkoxycarbonylamino, lower alkylsulfonyl, lower alkylsulfanilyl, lower alkoxy and hydroxy.

R¹ is preferably cyano, trifluoromethyl, alkoxycarbonyl, lower alkylsulfonyl or lower alkoxy.

R means a group represented by the formula (II)

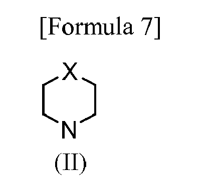

wherein X represents O, S or SO₂, or a group represented by the formula (III) 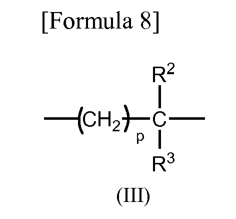 (wherein R² and R³ are same or different hydrogen or halogen atoms; and p is an integer of 0 or 1; and
the group represented by the formula (II) may be crosslinked via a methylene or ethylene chain).

A group represented by the formula (II) crosslinked via a methylene or ethylene chain is preferably a group crosslinked via an ethylene chain.

R may also has 1-3 identical or different groups selected from the group consisting of phenyl, lower alkyl, lower alkoxy, hydroxy and halogen. Among said phenyl, lower alkyl, lower alkoxy, hydroxy and halogen, lower alkyl, lower alkoxy, hydroxy or halogen is preferred.

Consequently, as a group represented by R, for example, a group selected from the group consisting of the formula (II-1) 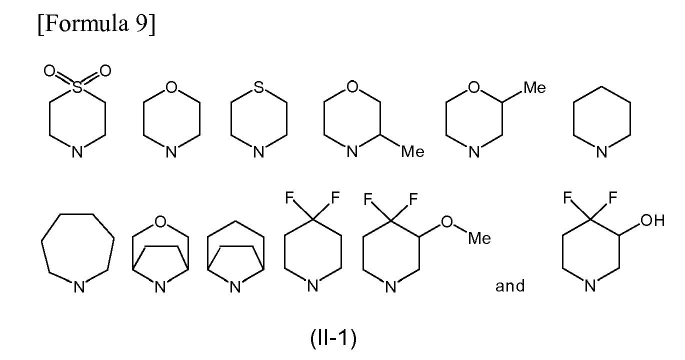 is preferred, and for example, a group selected from the group consisting of the formula (II-2)

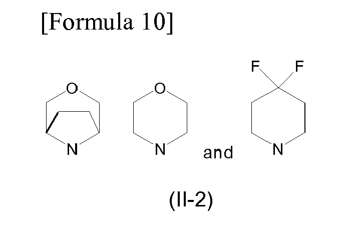

is more preferred.

X₁ represents lower (C₁₋₄) alkylene.

Lower alkylene groups represented by X₁ include, for example, methylene, ethylene, trimethylene and tetramethylene groups, among which methylene or ethylene is preferred, and methylene is more preferred.

The lower alkylene is optionally substituted with one or two identical or different lower alkyl group. When the two identical or different lower alkyl groups are bonded to the same carbon atom in the lower alkylene, these may together form 3-7 membered aliphatic rings.

In addition, when R¹ is lower alkoxycarbonylamino, X₁, together with nitrogen constituting the lower alkoxycarbonylamino and carbon in X₁, may form a 5- or 6-membered aliphatic ring containing nitrogen.

When R¹ is lower alkoxycarbonylamino, for example, groups represented by

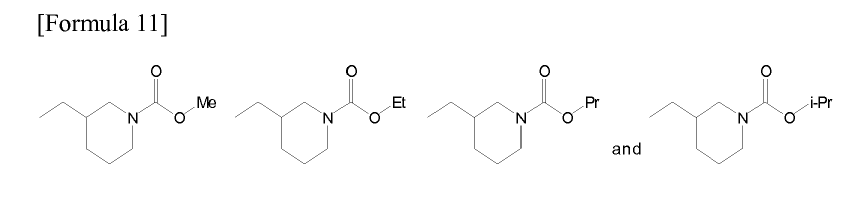

are encompassed by a group -X₁-R¹, together with nitrogen constituting the lower alkoxycarbonylamino and carbon in X₁, forming a 5- or 6-membered aliphatic ring containing nitrogen.

X₂ represents C₂₋₆ lower alkylene, and one of carbon atoms constituting the lower alkylene is optionally substituted with an oxygen or sulfur atom; and the lower alkylene is optionally substituted with hydroxy.

X₂ is preferably C₂ or C₃ lower alkylene optionally substituted with hydroxy or preferably C₁ or C₂ lower alkylene constituted by carbon atoms of which one is substituted with an sulfur atom.

X₂ is preferably, for example, -CH₂, -CH₂-, -S-CH₂- or -CH₂-CH(OH)-.

Het represents a 5-membered heteroaromatic ring which has at least one nitrogen atom and, in addition, one or two hetero atoms selected from the group consisting of nitrogen, sulfur and oxygen.

When the Het group is substituted with one or two identical or different lower alkyl optionally substituted with halogen or lower alkoxy, or cycloalkyl groups or is substituted with two lower alkyl groups, the Het group, together with the two lower alkyl groups, may form 5-7 membered cycloalkyl.

Examples of 5-membered heteroaromatic rings represented by Het include thiazolyl, imidazolyl, pyrazolyl, oxazolyl and triazolyl groups.

Consequently, Het groups include, for example, 4,5-dimethyl-1,3-thiazol-2-yl, 4,5-dimethyl-1,3-oxazol-2-yl, 1,5-dimethyl-1H-pyrazol-3-yl, 1,5-dimethyl-1H-1,2,4-triazol-3-yl, 5-ethyl-1-methyl-1H-1,2,4-triazol-3-yl, 1,2-dimethyl-1H-imidazol-4-yl, 5-cyclopropyl-1-methyl-1H-1,2,4-triazol-3-yl, 4,5,6,7-tetrahydro-1,3-benzoxazol-2-yl, 5-ethyl-4-methyl-1,3-oxazol-2-yl, 1,4,5-trimethyl-1H-imidazol-2-yl, 5-ethyl-4-methyl-1,3-thiadiazol-2-yl, 5-methoxymethyl-4-methyl-1,3-oxazol-2-yl, 1-fluoromethyl-5-methyl-1H-1,2,4-triazol-3-yl, 1-ethyl-5-methyl-1H-1,2,4-triazol-3-yl and 1-cyclopropyl-5-methyl-1H-triazol-3-yl.

In accordance with a preferred embodiment, any aspects of R, R¹, X, X₁, X₂, Het and m as described above may be combined.

Compounds represented by the formula (I) specifically include, for example,
{[6-{[(4,5-dimethyl-1,3-thiazol-2-yl)thio]methyl}-4-(3-oxa-8-azabicyclo[3,2,1]octo-8-y 1)-2-pyridinyl]amino}acetonitrile,
6-{[(4,5-dimethyl-1,3-oxazol-2-yl)thio]methyl}-4-(3-oxa-8-azabicyclo[3,2,1]octo-8-yl)-N-(2,2,2-trifluoroethyl)-2-pyridinamine,
6-{[(1,5-dimethyl-1H-pyrazol-3-yl)thio]methyl}-4-(3-oxa-8-azabicyclo[3,2,1]octo-8-yl) -N-(2,2,2-trifluoroethyl)-2-pyridinamine,
6-{[(4,5-dimethyl-1,3-thiazol-2-yl)thio]methyl}-4-(3-oxa-8-azabicyclo[3,2,1]octo-8-yl)-N-(2,2,2-trifluoroethyl)-2-pyridinamine,
4-(4,4-difluoro-1-piperidinyl)-6-{[(1,5-dimethyl-1H-1,2,4-triazol-3-yl)thio]methyl}-N-( 2,2,2-trifluoroethyl)-2-pyridinamine,
4-(4,4-difluoro-1-piperidinyl)-6-{[(5-ethyl-1-methyl-1H-1,2,4-triazol-3-yl)thio]methyl} -N-(2,2,2-trifluoroethyl)-2-pyridinamine,
4-(4,4-difluoro-1-piperidinyl)-6-{[(1,2-dimethyl-1H-imidazol-4-yl)thiol]methyl}-N-(2,2 ,2-trifluoroethyl)-2-pyridinamine,
6-{[(5-cyclopropyl-1-methyl-1H-1,2,4-triazol-3-yl)thio]methyl}-4-(4,4-difluoro-1-piper idinyl)-N-(2,2,2-trifluoroethyl)-2-pyridinamine,
4-(4,4-difluoro-1-piperidinyl)-6-{[(1,5-dimethyl-1H-pyrazol-3-yl)thio]methyl}-N-(2,2,2 -trifluoroethyl)-2-pyridinamine,
[(4-(4,4-difluoro-1-piperidinyl)-6-{[(1,2-dimethyl-1H-imidazol-4-yl)thio]methyl}-2-per idinyl)amino]acetic acid ethyl ester,
6-{[(4,5-dimethyl-1,3-oxazol-2-yl)thio]methyl}-4-(1,1-dioxide-4-thiomorpholinyl)-N-(2 ,2,2-trifluoroethyl)-2-pyridinamine,
4-(1,1-dioxide-4-thiomorpholinyl)-6-[(4,5,6,7-tetrahydro-1,3-benzoxazol-2-ylthio)meth yl]-N-(2,2,2-trifluoroethyl)-2-pyridinamine,
4-(1-azepanyl)-6-{[(1,2-dimethyl-1H-imidazol-4-yl)thio]methyl}-N-(2,2,2-trifluoroethy 1)-2-pyridinamine,
6-[2-(5-ethyl-4-methyl-1,3-oxazol-2-yl)ethyl]-N-(2-methoxyethyl)-4-(3-methyl-4-morp ho linyl)-2-pyridinamine,
6-[2-(5-ethyl-4-methyl-1,3-oxazol-2-yl)ethyl]-N-(2-methoxyethyl)-4-(2-methyl-4-morp ho linyl)-2-pyridinamine,
1-{2-[2-(5-ethyl-4-methyl-1,3-oxazol-2-yl)ethyl]-6-[(2-methoxyethyl)amino]-4-pyridin yl-4,4-difluoro-piperidinol,
4-(4,4-difluoro-3-methoxy-1-piperidinyl)-6-[2-(5-ethyl-4-methyl-1,3-oxazol-2-yl)ethyl] -N-(2-methoxyethyl)-2-pyridinamine,
4-(8-azabicyclo[3,2,1]octo-8-yl)-6-[2-(5-ethyl-4-methyl-1,3-oxazol-2-yl)ethyl]-N-(2-me thoxyethyl)-2-pyridinamine,
1-{4-(4,4-difluoro-1-piperidinyl)-6-[(2,2,2-trifluoroethyl)amino]-2-pyridinyl}-2-(4,5-di methyl-1,3-oxazol-2-yl)ethanol,
1-{4-(4,4-difluoro-1-piperidinyl)-6-[(2,2,2-trifluoroethyl)amino]-2-pyridinyl}-2-(5-ethy 1-4-methyl-1,3-oxazol-2-yl)ethanol,
2-(5-ethyl-4-methyl-1,3-oxazol-2-yl)-1-{4-(3-oxa-8-azabicydo[3,2,1]octo-8-yl)-6-[(2,2, 2-trifluoroethyl)amino]-2-pyridinyl}ethanol,
2-(4,5-dimethyl-1,3-thiazol-2-yl)-1-{4-(3-oxa-8-azabicydo[3,2,1]octo-8-yl)-6-[(2,2,2-tr ifluoroethyl)amino]-2-pyridinyl}ethanol,
2-(5-ethyl-4-methyl-1,3-oxazol-2-yl)-1-{4-(4-morpholinyl)-6-[(2,2,2-trifluoroethyl)ami no]-2-pyridinyl}ethanol,
2-(4,5-dimethyl-1,3-oxazol-2-yl)-1-{4-(4-thiomorpholinyl)-6-[(2,2,2-trifluoroethyl)ami no]-2-pyridinyl}ethanol,
{[6-{[(4,5-dimethyl-1,3-oxazol-2-yl)thio]methyl}-4-(3-oxa-8-azabicyclo[3,2,1]octo-8-y 1)-2-pyridinyl]amino}acetonitrile,
({4-(3-oxa-8-azabicyclo[3,2,1]octo-8-yl)-6-[(4,5,6,7-tetrahydro-1,3-benzoxazol-2-ylthio )methyl]-2-pyridinyl}amino)acetonitrile,
{[6-[2-(5-ethyl-4-methyl-1,3-thiazol-2-yl)ethyl]-4-(3-oxa-8-azabicyclo[3,2,1]octo-8-yl)-2-pyridinyl]amino}acetonitrile,
[(4-(4-morpholinyl)-6-{[(1,4,5-trimethyl-1H-imidazol-2-yl)thio]methyl)-2-pyridinyl)a mino]acetonitrile,
{[6-{[(4,5-dimethyl-1,3-thiazol-2-yl)thio]methyl}-4-(4-morpholinyl)-2-pyridinyl]amino }cetonitrile,
{[6-{[(4,5-dimethyl-1,3-oxazol-2-yl)thio]methyl}-4-(4-morpholinyl)-2-pyridinyl]amino }acetonitrile,
{[6-{[(5-ethyl-4-methyl-1,3-oxazol-2-yl)thio]methyl}-4-(4-morpholinyl)-2-pyridinyl]a mino}acetonitrile,
({4-(4,4-difluoro-1-piperidinyl)-6-[2-(1,5-dimethyl-1H-pyrazol-3-yl)ethyl]-2-pyridinyl} amino)acetonitrile,
({4-(4,4-difluoro-l-piperidinyl)-6-[2-(4,5-dimethyl-l,3-oxazol-2-yl)ethyl]-2-pyridinyl}a mino)acetonitrile,
[(4-(4-thiomorpholinyl)-6-{[(1,4,5-trimethyl-1H-imidazol-2-yl)thio]methyl}-2-pyridiny 1)amino]acetonitrile,
2-{[6-[2-(5-ethyl-4-methyl-1,3-oxazol-2-yl)ethyl]-4-(4-morpholinyl)-2-piperidinyl]ami no}propanenitrile,
3-{[6-[2-(5-ethyl-4-methyl-1,3-oxazol-2-yl)ethyl]-4-(4-morpholinyl)-2-pyridinyl]amino }propanenitrile,
6-{[(4,5-dimethyl-1,3-oxazol-2-yl)thio]methyl}-N-(3-methoxypropyl)-4-(4-morpholinyl )-2-pyridinamine,
{[6-{[(4,5-dimethyl-1,3-thiazol-2-yl)thio]methyl}-4-(3-oxa-8-azabicyclo[3,2,1]octo-8-y 1)-2-pyridinyl]amino} acetic acid methyl ester,
4-(4-morpholinyl)-N-(2,2,2-trifluoroethyl)-6-{[(1,4,5-trimethyl-1H-imidazol-2-yl)thio] methyl}-2-pyridinamine,
6-{[(4,5-dimethyl-1,3-oxazol-2-yl)thio]methyl}-4-(4-morpholinyl)-N-(2,2,2-trifluoroeth yl)-2-pyridinamine,
6-{[(1,2-dimethyl-1H-imidazol-4-yl)thio]methyl}-N-(2-methoxyethyl)-4-(4-morpholiny 1)-2-pyridinamine,
N-(2-methoxyethyl)-6-({[5-(methoxymethyl)-4-methyl-1,3-oxazol-2-yl]thio}methyl)-4-(4-morpholinyl)-2-pyridinamine,
4-(4,4-difluoro-1-piperidinyl)-6-({[1-(fluoromethyl)-5-methyl-1H-1,2,4-triazol-3-yl]thi o}methyl)-N-(2,2,2-trifluoroethyl)-2-pyridinamine,
4-(4,4-difluoro-1-piperidinyl)-6-{[(1-ethyl-5-methyl-1H-1,2,4-triazol-3-yl)thio]methyl} -N-(2,2,2-trifluoroethyl)-2-pyridinamine,
6-{[(1,5-dimethyl-1H-1,2,4-triazol-3-yl)thio]methyl}-4-(4-thiomorpholinyl)-N-(2,2,2-tri fluoroethyl)-2-pyridinamine,
6-{[(1,2-dimethyl-1H-imidazol-4-yl)thio]methyl}-4-(4-thiomorpholinyl)-N-(2,2,2-triflu oroethyl)-2-pyridinamine,
6-{[(1-cyclopropyl-5-methyl-1H-1,2,4-triazol-3-yl)thio]methyl}-4-(4,4-difluoro-1-piper idinyl)-N-(2,2,2-trifluoroethyl)-2-pyridinamine,
4-(4,4-difluoro-l-piperidinyl)-6-{[(4,5-dimethyl-l,3-oxazol-2-yl)thio]methyl}-N-[2-(me thylsulfonyl)ethyl]-2-pyridinamine,
6-{[(4,5-dimethyl-1,3-oxazol-2-yl)thio]methyl}-N-[2-(methylsulfonyl)ethyl]-4-(4-thiom orpholinyl)-2-pyridinamine,
6-{[(4,5-dimethyl-1,3-thiazol-2-yl)thio]methyl}-4-(1,1-dioxide)-4-thiomorpholinyl)-N-( 2,2,2-trifluoroethyl)-2-pyridinamine,
6-{[(1,5-dimethyl-1H-pyrazol-3-yl)thio]methyl}-4-(1,1-dioxide-4-thiomorpholinyl)-N-( 2,2,2-trifluoroethyl)-2-pyridinamine,
4-(1,1-dioxide-4-thiomorpholinyl)-6-{[(5-ethyl-4-methyl-1,3-oxazol-2-yl)thio]methyl}-N-(2,2,2-trifluoroethyl)-2-pyridinamine,
N-cyclopropyl-6-{(4,5-dimethyl-1,3-oxazol-2-yl)thio]methyl}-4-(4-morpholinyl)-2-pyr idinamine,
2-{[6-{[(5-ethyl-4-methyl-1,3-oxazol-2-yl)thio]methyl}-4-(4-morpholinyl)-2-pyridinyl] amino}ethanol,

3-({[6-{[(4,5-dimethyl-1,3-oxazol-2-yl)thio]methyl}-4-(4-morpholinyl)-2-pyridinyl]ami no}methyl)-1-piperidine carboxylic acid methyl ester,
6-[2-(5-ethyl-4-methyl-1,3-oxazol-2-yl)ethyl]-N-[(methylthio)methyl]-4-(4-morpholinyl )-2-pyridinamine and
2-{[6-{[(4,5-dimethyl-1,3-oxazol-2-yl)thio]methyl}-4-(4-morpholinyl)-2-pyridinyl]ami no}-2-methylpropanenitrile.

Furthermore, among the compounds represented by the formula (I),
{[6-{[(4,5-dimethyl-1,3-thiazol-2-yl)thio]methyl}-4-(3-oxa-8-azabicyclo[3,2,1]octo-8-y 1)-2-pyridinyl]amino}acetonitrile,
4-(4,4-difluoro-1-piperidinyl)-6-{[(1,5-dimethyl-1H-1,2,4-triazol-3-yl)thio]methyl}-N-( 2,2,2-trifluoroethyl)-2-pyridinamine,
4-(4,4-difluoro-1-piperidinyl)-6-{[(5-ethyl-1-methyl-1H-1,2,4-triazol-3-yl)thio]methyl} -N-(2,2,2-trifluoroethyl)-2-pyridinamine,
4-(4,4-difluoro-1-piperidinyl)-6-{[(1,5-dimethyl-1H-pyrazol-3-yl)thio]methyl}-N-(2,2,2 -trifluoroethyl)-2-pyridinamine,
6-{[(4,5-dimethyl-1,3-oxazo1-2-yl)thio]methyl}-4-(1,1-dioxide-4-thiomorpholinyl)-N-(2 ,2,2-trifluoroethyl)-2-pyridinamine,
1-{4-(4,4-difluoro-1-piperidinyl)-6-[(2,2,2-trifluoroethyl)amino]-2-pyridinyl}-2-(4,5-di methyl-1,3-oxazol-2-yl)ethanol,
2-(5-ethyl-4-methyl-1,3-oxazol-2-yl)-1-{4-(3-oxa-8-azabicyclo[3,2,1]octo-8-yl)-6-[(2,2, 2-trifluoroethyl)amino]-2-pyridinyl}ethanol,
2-(5-ethyl-4-methyl-1,3-oxazol-2-yl)-1-{4-(4-morpholinyl)-6-[(2,2,2-trifluoroethyl)ami no]-2-pyridinyl}ethanol,
{[6-{[(4,5-dimethyl-1,3-oxazol-2-yl)thio]methyl}-4-(3-oxa-8-azabicyclo[3,2,1]octo-8-y 1)-2-pyridinyl]amino}acetonitrile,
({4-(3-oxa-8-azabicyclo[3,2,1]octo-8-yl)-6-[(4,5,6,7-tetrahydro-1,3-benzoxazol-2-ylthio )methyl]-2-pyridinyl}amino)acetonitrile,
{[6-{[(4,5-dimethyl-1,3-thiazol-2-yl)thio]methyl}-4-(4-morpholinyl)-2-pyridinyl]amino }acetonitrile,
{[6-{[(4,5-dimethyl-1,3-oxazol-2-yl)thio]methyl}-4-(4-morpholinyl)-2-pyridinyl]amino }acetonitrile,
({4-(4,4-difluoro-1-piperidinyl)-6-[2-(1,5-dimethyl-1H-pyrazol-3-yl)ethyl]-2-pyridinyl} amino)acetonitrile,
4-(4,4-difluoro-1-piperidinyl)-6-({[1-(fluoromethyl)-5-methyl-1H-1,2,4-triazol-3-yl]thi o}methyl)-N-(2,2,2-trifluoroethyl)-2-pyridinamine,
4-(4,4-difluoro-1-piperidinyl)-6-{[(1-ethyl-5-methyl-1H-1,2,4-triazol-3-yl)thio]methyl} -N-(2,2,2-trifluoroethyl)-2-pyridinamine,
6-{[(1,5-dimethyl-1H-1,2,4-triazol-3-yl)thio]methyl}-4-(4-thiomorpholinyl)-N-(2,2,2-tri fluoroethyl)-2-pyridinamine,
6-{[(1-cyclopropyl-5-methyl-1H-1,2,4-triazol-3-yl)thio]methyl}-4-(4,4-difluoro-1-piper idinyl)-N-(2,2,2-trifluoroethyl)-2-pyridinamine,
6-{[(4,5-dimethyl-1,3-thiazo1-2-yl)thio]methyl}-4-(1,1-dioxide-4-thiomorpholinyl)-N-( 2,2,2-trifluoroethyl)-2-pyridinamine or
2-{[6-{[(4,5-dimethyl-1,3-oxazol-2-yl)thio]methyl}-4-(4-morpholinyl)-2-pyridinyl]ami no}-2-methylpropanenitrile, or a pharmaceutically acceptable salt thereof is preferred.
A process for producing a compound according to the present invention will now be described. A compound according to the present invention can be produced by a process illustrated below or processes described in Reference Examples or Examples. However, a process for producing a compound according to the present invention is not limited to such reaction examples.
The compound (I) according to the present invention can be produced, for example, by the following process:

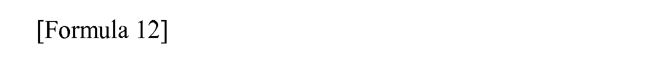

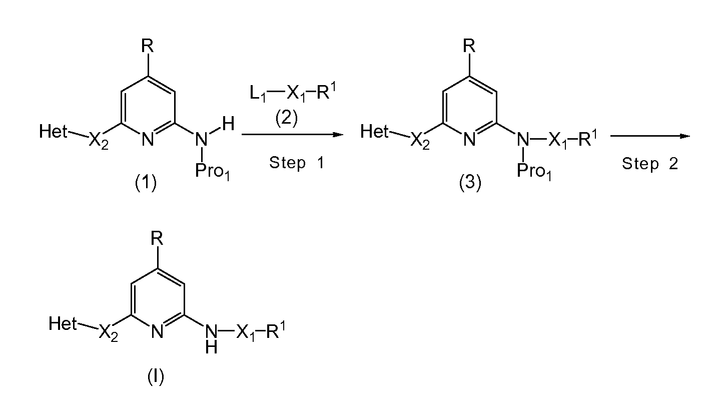

(wherein Pro₁ represents a protective group for amino; L₁ represents a leaving group; and the other symbols have the same definitions specified above).

### (Step 1)

This step is a process of producing a compound (3) by reacting a compound (1) with a compound (2) in the presence of a base.

Examples of bases as used in this step include sodium hydride, potassium hydride, N-butyllithium, potassium-tert-butoxide and sodium methoxide.

An amount of the base is typically 1.0-5.0 equivalents, preferably 1.0-2.0 equivalents, relative to 1 equivalent of the compound (1).

An amount of the compound (2) as used is typically 1.0-5.0 equivalents, preferably 1.0-2.0 equivalents, relative to 1 equivalent of the compound (1).

L₁ in the compound (2) includes, for example, halogen, trifluoromethanesulfonyloxy, p-toluenesulfonyloxy and methanesulphonyloxy.

Instead of the compound (2), a compound represented by the compound (2-1)

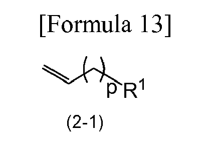

[wherein p represents an integer of from 0 to 2; and the other symbols have the same definitions specified above]
may be also used.

Compounds represented by the compound (2-1) include methyl vinyl sulfone. The reaction temperature is typically 0-100°C, preferably from 0°C to room temperature.
The reaction time is typically 10 minutes to 48 hours, preferably 10 minutes to 24 hours.

Unless interfering with the reaction, any reaction solvent may be used, examples of which include tetrahydrofuran (sometimes abbreviated as THF), N,N-dimethylformamide (sometimes abbreviated as DMF), N,N-dimethylacetamide (sometimes abbreviated as DMA), dimethylsulfoxide (sometimes abbreviated as DMSO), dimethoxyethane (sometimes abbreviated as DME), chloroform, methylene chloride and diethyl ether.

The compound (3) thus obtained may be isolated and purified in well-known separation and purification method such as concentration, concentration under reduced pressure, reprecipitation, solvent extraction, crystallization and chromatography, or subjected to the next step without isolation and purification.

### (Step 2)

This step is a process of producing a compound (I) according to the present invention by removing a protective group Pro₁ for amino of the compound (3) obtained in Step 1 described above.

The reaction in this step can be carried out by a method as described in the document (T.W. Green: Protective Groups in Organic Synthesis, Second Edition, John Wiley & Sons (1991)), methods equivalent thereto or combinations of these with usual methods. For example, when a Boc group is used as a protective group Pro₁ for amino, the Boc group can be removed by using trifluoroacetic acid.

The compound (I) obtained in such a manner may be isolated and purified by well-known separation and purification measures such as concentration, concentration under reduced pressure, reprecipitation, solvent extraction, crystallization and chromatography.
A compound (I-1) according to the present invention

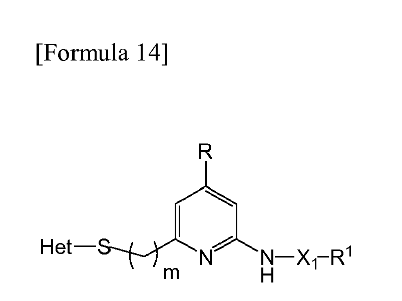

(wherein m represents an integer of 1 or 2; and the other symbols have the same definitions specified above) can be also produced, for example, by the following process:

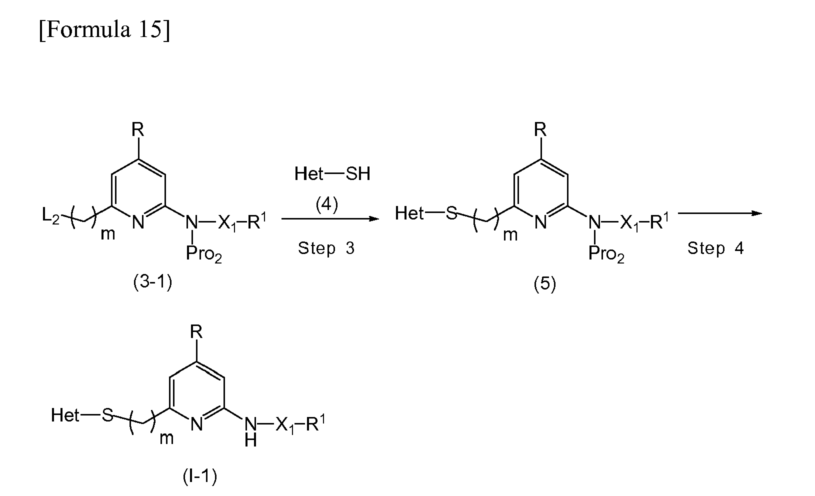

(wherein L₂ represents a leaving group; Pro₂ represents a protective group for amino; and the other symbols have the same definitions specified above).

### (Step 3)

This step is a process of producing a compound (5) by reacting a compound (3-1) with a compound (4) or a salt thereof in the presence of a base.

L₂ includes, for example, halogen, methanesulfonyloxy, trifluoromethanesulfonyloxy, p-toluenesulfonyloxy and p-nitrobenzenesulfonyloxy.

Bases as used in this step include, for example, potassium carbonate, sodium carbonate, cesium carbonate, sodium hydrogencarbonate, potassium hydrogencarbonate, lithium carbonate, trimethylamine, triethylamine, diisopropylethylamine, pyridine and sodium hydride.

An amount of the base is typically 1.0-5.0 equivalents, preferably 1.0-2.0 equivalents, relative to 1 equivalent of the compound (3-1).

Examples of the compound (4) as used in this step include 4,5-dimethyl-2-mercaptothiazole, 4,5-dimethyl-oxazol-2-thiol, 4,5,6,7-tetrahydro-1,3-benzoxazol-2-thiol, 5-ethyl-4-methyl-1,3-oxazol-2-thiol, 5-ethyl-1-methyl-1H-1,2,4-triazol-3-thiol hydrochloride, 1,5-dimethyl-1H-1,2,4-triazol-3-thiol hydrochloride, 5-cyclopropyl-1-methyl-1H-1,2,4-triazol-3-thiol, 1,4,5-trimethyl-1H-imidazol-2-thiol, 1,2-dimethyl-1H-imidazol-4-thiol hydrochloride, 5-methoxymethyl-4-methyl-1,3-oxazol-2-thiol and 1,5-dimethyl-1H-pyrazol-3-thiol.

An amount of the compound (4) is typically 1.0-5.0 equivalents, preferably 1.0-2.0 equivalents, relative to 1 equivalent of the compound (3-1).

The reaction temperature is typically 0-100°C, preferably from 0°C to room temperature.

The reaction time is typically 10 minutes to 48 hours, preferably 10 minutes to 24 hours.

Unless interfering with the reaction, any solvent may be used in this step, examples of which include tetrahydrofuran, N,N-dimethylformamide, N,N-dimethylacetamide, dimethylsulfoxide, dimethoxyethane, chloroform, methylene chloride and diethyl ether.
The compound (5) thus obtained may be isolated and purified in well-known separation and purification method such as concentration, concentration under reduced pressure, reprecipitation, solvent extraction, crystallization and chromatography, or subjected to the next step without isolation and purification.

### (Step 4)

This step is a process for producing a compound (I-1) according to the present invention by removing a protective group Pro₂ for amino of the compound (5).

A group Pro₂ includes the same groups as the group Pro₁, such as a Boc group.

The reaction in this step can be carried out by a method as described in the document (T.W. Green: Protective Groups in Organic Synthesis, Second Edition, John Wiley & Sons (1991)), methods equivalent thereto or combinations of these with usual methods.

The compound (I-1) obtained in such a manner may be isolated and purified by well-known separation and purification measures such as concentration, concentration under reduced pressure, reprecipitation, solvent extraction, crystallization and chromatography.
A compound (1-2) according to the present invention

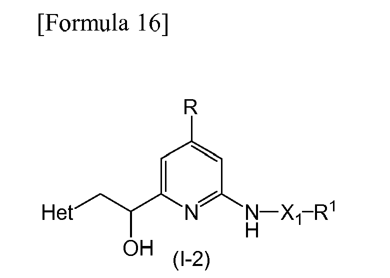

(wherein each symbol has the same definition specified above) can be also produced, for example, by the following process:

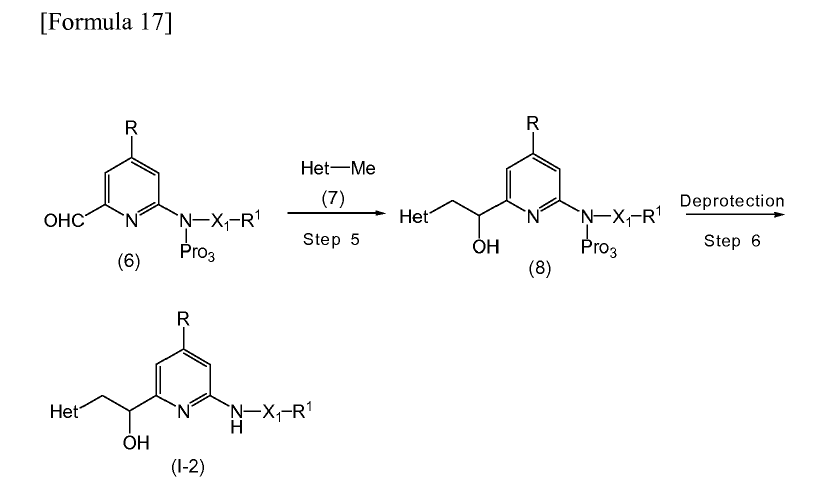

(wherein Pro₃ represents a protective group for amino; and the other symbols have the same definitions specified above).

### (Step 5)

This step is a process of producing a compound (8) by reacting a compound (6) with a compound (7) in the presence of a base.

Bases as used in this step include, for example, lithium diisopropylamide (hereinafter sometimes abbreviated as LDA), lithium hexamethyldisilazide, sodium hexamethyldisilazide, n-butyllithium and tert-butyllithium.

LDA can be also freshly prepared from n-butyllithium and diisopropylamine.

An amount of the base is typically 1.0-3.0 equivalents, preferably 1.0-2.0 equivalents, relative to 1 equivalent of the compound (6).

The compound (7) as used in this step includes, for example, 2,4,5-trimethyloxazole, 2,4-dimethyl-5-ethyloxazole and 2,4,5-trimethylthiazole.

An amount of the compound (7) is typically 1.0-3.0 equivalents, preferably 1.0-2.0 equivalents, relative to 1 equivalent of the compound (6).

The reaction temperature is typically from -78°C to room temperature, preferably from -78°C to 0°C.

The reaction time is typically 10 minutes to 48 hours, preferably 10 minutes to 24 hours.

Unless interfering with the reaction, any solvent may be used in this step, examples of which include THF, DME, chloroform, methylene chloride, diethyl ether and toluene.

The compound (8) thus obtained may be isolated and purified in well-known separation and purification method such as concentration, concentration under reduced pressure, reprecipitation, solvent extraction, crystallization and chromatography, or subjected to the next step without isolation and purification.

### (Step 6)

This step is a process for producing a compound (1-2) according to the present invention by removing a protective group Pro₃ for amino of the compound (8).

A group Pro₃ includes the same groups as the group Pro₁ or Pro₂ such as a Boc group.
The reaction in this step can be carried out by a method as described in the document (T.W. Green: Protective Groups in Organic Synthesis, Second Edition, John Wiley & Sons (1991)), methods equivalent thereto or combinations of these with usual methods.

The compound (1-2) obtained in such a manner may be isolated and purified by well-known separation and purification measures such as concentration, concentration under reduced pressure, reprecipitation, solvent extraction, crystallization and chromatography.

A compound (1-3) according to the present invention 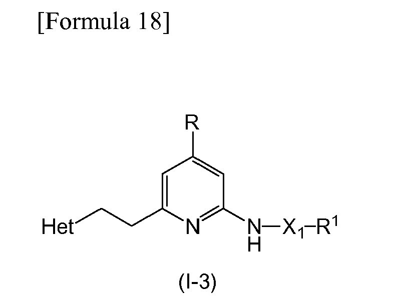 can be also produced, for example, by the following process: 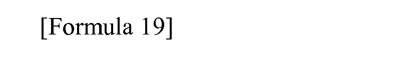 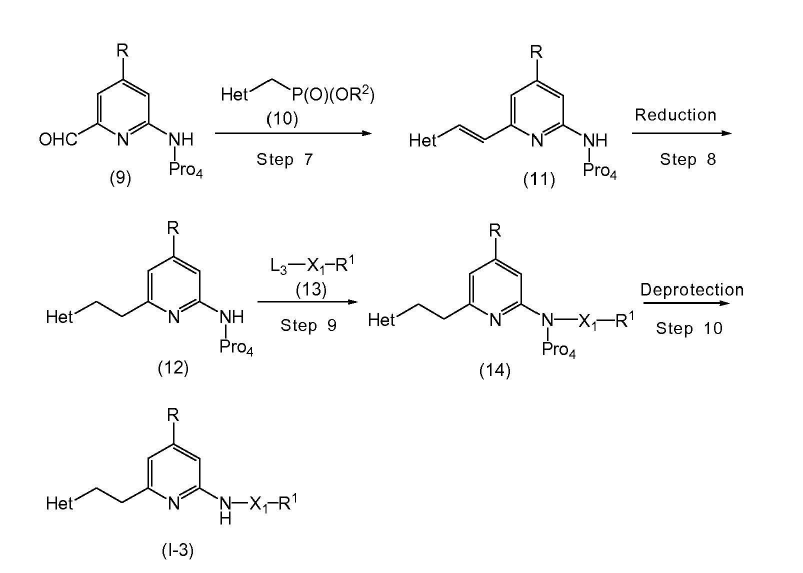 (wherein Pro₄ represents a protective group for amino; R² represents lower alkyl; L₃ represents a leaving group; and the other symbols have the same definitions specified above).

### (Step 7)

This step is a process of producing a compound (11) by reacting a compound (9) with a compound (10) in the presence of a base.

Bases as used in this step include, for example, sodium hydride, n-butyllithium and LDA.

An amount of the base is typically 1.0-3.0 equivalents, preferably 1.0-2.0 equivalents, relative to 1 equivalent of the compound (9).

The compound (10) as used in this step includes, for example, [(5-ethyl-4-methyl-1,3-oxazol-2-yl)methyl]phosphonic acid diethyl ester, [(5-ethyl-4-methyl-1,3-thiazol-2-yl)methyl]phosphonic acid diethyl ester, [(4,5-dimethyl-1,3-oxazol-2-yl)methyl]phosphonic acid diethyl ester and [(1,5-dimethyl-1H-pyrazol-3-yl)methyl]phosphonic acid dimethyl ester.

An amount of the compound (10) is typically 1.0-5.0 equivalents, preferably 1.0-2.0 equivalents, relative to 1 equivalent of the compound (9).

The reaction temperature is typically from 0°C to 50°C, preferably from 0°C to room temperature.

The reaction time is typically 10 minutes to 48 hours, preferably 10 minutes to 24 hours.

Unless interfering with the reaction, any solvent may be used in this step, examples of which include THF, DMF, DMA, DMSO, DME, chloroform, methylene chloride and diethyl ether.

The compound (11) thus obtained may be isolated and purified in well-known separation and purification method such as concentration, concentration under reduced pressure, reprecipitation, solvent extraction, crystallization and chromatography, or subjected to the next step without isolation and purification.

### (Step 8)

This step is a process of producing a compound (12) by reducing the compound (11).

The reaction in this step can be carried out using a palladium catalyst under hydrogen atmosphere.

Palladium catalysts as used include, for example, palladium carbon and palladium hydride.
An amount of the palladium catalyst used is typically 0.1-0.5 equivalent, preferably 0.1-0.2 equivalent, relative to 1 equivalent of the compound (11).

The reaction temperature is typically from 0°C to 50°C, preferably from 0°C to room temperature.

The reaction time is typically 2 hours to 72 hours, preferably 2 hours to 24 hours.

Unless interfering with the reaction, any solvent may be used in this step, examples of which include THF, methanol, ethanol and ethyl acetate. The compound (12) thus obtained may be isolated and purified in well-known separation and purification method such as concentration, concentration under reduced pressure, reprecipitation, solvent extraction, crystallization and chromatography, or subjected to the next step without isolation and purification.

### (Step 9)

This step is a process of producing a compound (14) by reacting a compound (12) with a compound (13) in the presence of a base.
The reaction in this step can be carried out by the same method as in Step 1 described above, methods equivalent thereto or combinations of these with usual methods.
A compound (13) as used in this step is an identical compound as the compound (2) used in Step 1, or a salt thereof.
The compound (14) thus obtained may be isolated and purified in well-known separation and purification method such as concentration, concentration under reduced pressure, reprecipitation, solvent extraction, crystallization and chromatography, or subjected to the next step without isolation and purification.

### (Step 10)

This step is a process for producing a compound (1-3) according to the present invention by removing a protective group Pro₄ for amino of the compound (14). The reaction in this step can be carried out by a method as described in the document (T.W. Green: Protective Groups in Organic Synthesis, Second Edition, John Wiley & Sons (1991)), methods equivalent thereto or combinations of these with usual methods.
The compound (1-3) obtained in such a manner may be isolated and purified by well-known separation and purification measures such as concentration, concentration under reduced pressure, reprecipitation, solvent extraction, crystallization and chromatography.

The compound (1-3) according to the present invention can be also produced, for example, by the following process:

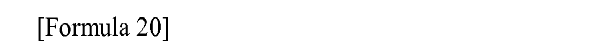

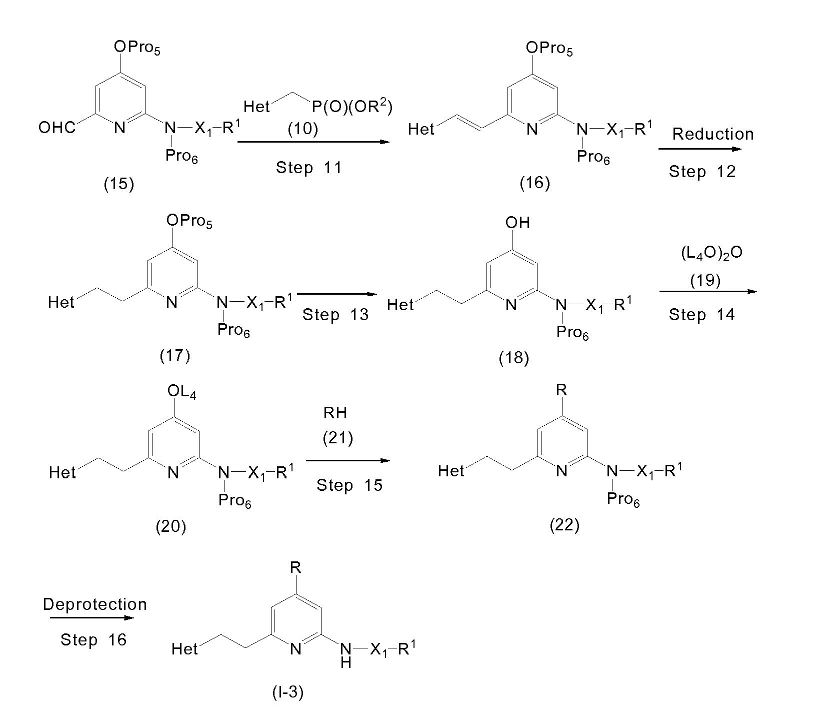

(wherein Pro₅ represents a protective group for phenolic OH; Pro₆ represents a protective group for amino; OL₄ represents a leaving group; and the other symbols have the same definitions specified above).

### (Step 11)

This step is a process of producing a compound (16) by reacting a compound (15) with the compound (10) in the presence of a base.

The reaction in this step can be carried out by the same method as in Step 7 described above, methods equivalent thereto or combinations of these with usual methods.

Protective groups Pro₅ include protective groups described in the document (T.W. Green: Protective Groups in Organic Synthesis, Second Edition, John Wiley & Sons (1991)), such as benzyl.

The compound (16) thus obtained may be isolated and purified in well-known separation and purification method such as concentration, concentration under reduced pressure, reprecipitation, solvent extraction, crystallization and chromatography, or subjected to the next step without isolation and purification.

### (Step 12)

This step is a process of producing a compound (17) by reducing the compound (16).

The reaction in this step can be carried out by the same method as in Step 8 described above, methods equivalent thereto or combinations of these with usual methods.

The compound (17) thus obtained may be isolated and purified in well-known separation and purification method such as concentration, concentration under reduced pressure, reprecipitation, solvent extraction, crystallization and chromatography, or subjected to the next step without isolation and purification.

### (Step 13)

This step is a process for producing a compound (18) by removing a protective group Pro₅ of the compound (17).

The reaction in this step can be carried out by a method as described in the document (T.W. Green: Protective Groups in Organic Synthesis, Second Edition, John Wiley & Sons (1991)), methods equivalent thereto or combinations of these with usual methods.

For example, when Pro₅ is a benzyl group, a compound (18) can be produced by reaction at room temperature to 100°C for 10 minutes to 10 hours using 10% palladium carbon and cyclohexene in methanol.

The compound (18) thus obtained may be isolated and purified in well-known separation and purification method such as concentration, concentration under reduced pressure, reprecipitation, solvent extraction, crystallization and chromatography, or subjected to the next step without isolation and purification.

### (Step 14)

This step is a process of producing a compound (20) by reacting the compound (18) with a compound (19) in the presence of a base.

Bases as used in this step include, for example, triethylamine, potassium carbonate, cesium carbonate and diisopropylethylamine.

An amount of the base used is typically 1.0-5.0 equivalents, preferably 1.0-2.0 equivalents, relative to 1 equivalent of the compound (18).

An amount of the compound (19) used is typically 1.0-5.0 equivalents, preferably 1.0-2.0 equivalents, relative to 1 equivalent of the compound (18).

Compounds (19) include, for example, trifluoromethanesulfonic anhydride and p-toluenesulfonic anhydride.
Instead of the compound (19), L₄Cl may be used. Examples of L₄Cl include trifluoromethanesulfonyl chloride, methanesulfonyl chloride, p-toluenesulphonyl chloride and p-nitrobenzenesulfonyl chloride.

The reaction temperature is typically from 0°C to 50°C, preferably from 0°C to room temperature.

The reaction time is typically 10 minutes to 48 hours, preferably 10 minutes to 24 hours.

Unless interfering with the reaction, any solvent may be used in this step, examples of which include THF, DMF, DMA, DMSO, DME, chloroform, methylene chloride and diethyl ether.

The compound (20) thus obtained may be isolated and purified in well-known separation and purification method such as concentration, concentration under reduced pressure, reprecipitation, solvent extraction, crystallization and chromatography, or subjected to the next step without isolation and purification.

### (Step 15)

This step is a process for producing a compound (22) by reacting the compound (20) with a compound (21) in the presence of a palladium catalyst, a phosphine ligand and a base.

Palladium catalysts as used in this step include, for example, tris(dibenzylideneacetone)palladium(0), palladium acetate(II), palladium trifluoroacetate(II), palladium chloride(II), 1,1'-bis(diphenylphosphino)ferrocenedichloropalladium(II) and dichlorobis(triphenylphosphine)palladium(II).

An amount of the palladium catalyst used is typically 0.1-1.0 equivalent, preferably 0.1-0.5 equivalent, relative to 1 equivalent of the compound (20).

Phosphine ligands as used in this step include, for example, 2-(di-tert-butylphosphino)biphenyl, 4,5-bis(diphenylphosphino)-9,9-dimethylxanthene, 1,1'-bis(diphenylphosphino)ferrocene and 2,2'-bis(diphenylphosphino)-1,1'-binaphthyl.
An amount of the phosphine ligands used is typically 0.1-2.0 equivalents, preferably 0.1-0.5 equivalent, relative to 1 equivalent of the compound (20).

Bases as used in this step include , for example, potassium phosphate, potassium carbonate, cesium carbonate, tert-butoxypotassium and tert-butoxysodium.

An amount of the base used is typically 1.0-5.0 equivalents, preferably 1.0-3.0 equivalents, relative to 1 equivalent of the compound (20).
An amount of the compound (21) used is typically 1.0-5.0 equivalents, preferably 1.0-2.0 equivalents, relative to 1 equivalent of the compound (20).
The compound (21) RH denotes a group represented by 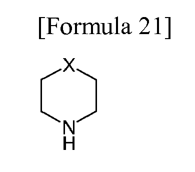 and is a compound in which a hydrogen atom is bonded to a nitrogen atom of R described above.

The reaction temperature is typically from 0°C to 100°C, preferably from 50°C to 100°C.

The reaction time is typically 10 minutes to 48 hours, preferably 10 minutes to 24 hours.

Unless interfering with the reaction, any solvent may be used in this step, examples of which include ethylene glycol dimethyl ether, 1,4-dioxane, THF, DMF and toluene.

The compound (22) thus obtained may be isolated and purified in well-known separation and purification method such as concentration, concentration under reduced pressure, reprecipitation, solvent extraction, crystallization and chromatography, or subjected to the next step without isolation and purification.

### (Step 16)

This step is a process for producing the compound (1-3) according to the present invention by removing a protective group Pro₆ for amino of the compound (22).

The reaction in this step can be carried out by a method as described in the document (T.W. Green: Protective Groups in Organic Synthesis, Second Edition, John Wiley & Sons (1991)), methods equivalent thereto or combinations of these with usual methods.

The compound (1-3) obtained in such a manner may be isolated and purified by well-known separation and purification measures such as concentration, concentration under reduced pressure, reprecipitation, solvent extraction, crystallization and chromatography.

In addition, a compound represented the formula (1-4)

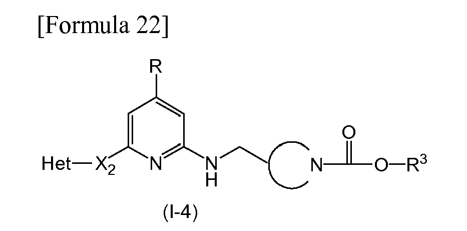

(wherein R³ represents lower alkyl; 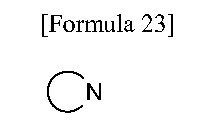 represents 5- or 6-membered aliphatic ring containing nitrogen formed together by a nitrogen atom constituting a lower alkoxycarbonylamino group (-NC(O)OR³) and a carbon atom in X₁; and the other symbols have the same definitions specified above) can be produced by the following process:

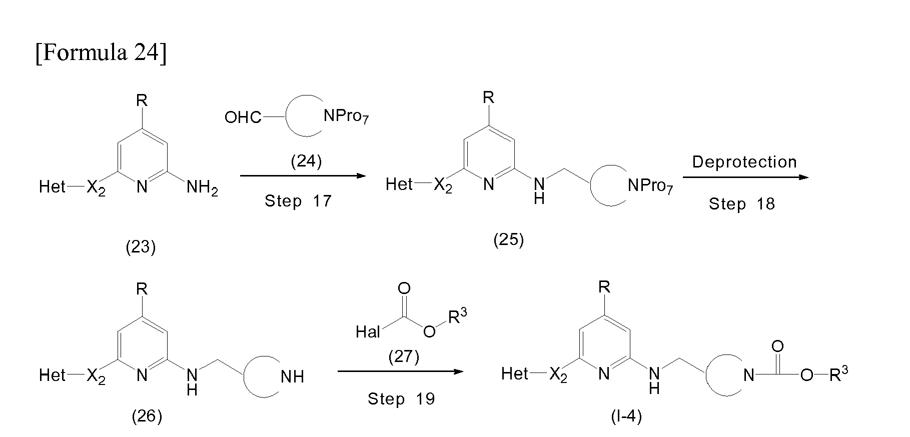

(wherein Pro₇ represents a protective group for amino; and the other symbols have the same definitions specified above).

### (Step 17)

This step is a process of producing a compound (25) by reacting a compound (23) with a compound (24) in the presence of a reducing agent.

An amount of the compound (24) used is typically 1.0-5.0 equivalents, preferably 1.0-2.0 equivalents, relative to 1 equivalent of the compound (23).

Reducing agents as used include, for example, sodium borohydride, sodium cyanoborohydride and sodium triacetoxyborohydride, among which sodium triacetoxyborohydride is preferred.

An amount of the reducing agent used is typically 1.0-5.0 equivalents, preferably 1.0-3.0 equivalents, relative to 1 equivalent of the compound (23).

An additive such as acetic acid or zinc chloride may be also added to a reaction system.
Unless interfering with the reaction, reaction solvents as used in this step include, but are not limited to, for example, methanol, ethanol, chloroform, methylene chloride, THF and 1,4-dioxane, among which THF is preferred.

The reaction temperature is typically from 0°C to 100°C, preferably from 0°C to 50°C.

The reaction time is typically 10 minutes to 48 hours, preferably 10 minutes to 24 hours.

The compound (25) thus obtained may be isolated and purified in well-known separation and purification method such as concentration, concentration under reduced pressure, reprecipitation, solvent extraction, crystallization and chromatography, or subjected to the next step without isolation and purification.

### (Step 18)

This step is a process for producing a compound (26) by removing a protective group Pro₇ for amino of the compound (25).

A group Pro₇ includes a Boc group.

The reaction in this step can be carried out by a method as described in the document (T.W. Green: Protective Groups in Organic Synthesis, Second Edition, John Wiley & Sons (1991)), methods equivalent thereto or combinations of these with usual methods.

The compound (26) thus obtained may be isolated and purified in well-known separation and purification method such as concentration, concentration under reduced pressure, reprecipitation, solvent extraction, crystallization and chromatography, or subjected to the next step without isolation and purification.

### (Step 19)

This step is a process for producing a compound (1-4) according to the present invention by reacting the compound (26) with a compound (27) in the presence of a base.

Bases as used in this step include, for example, triethylamine, diisopropylethylamine, sodium carbonate, potassium carbonate and cesium carbonate, among which potassium carbonate is preferred.
An amount of the base used is typically 1.0-5.0 equivalents, preferably 1.0-3.0 equivalents, relative to 1 equivalent of the compound (26).

Hal includes, for example, a chlorine atom.

An amount of the compound (27) used is typically 1.0-3.0 equivalents, preferably 1.0-2.0 equivalents, relative to 1 equivalent of the compound (26).
Unless interfering with the reaction, reaction solvents as used in this step include, but are not limited to, for example, DMF, DMA, DME, chloroform, methylene chloride, THF and 1,4-dioxane, among which THF is preferred.
The reaction temperature is typically from 0°C to 100°C, preferably from 0°C to 50°C.

The reaction time is typically 10 minutes to 48 hours, preferably 10 minutes to 24 hours.

The compound (1-4) obtained in such a manner may be isolated and purified by well-known separation and purification measures such as concentration, concentration under reduced pressure, reprecipitation, solvent extraction, crystallization and chromatography.

A compound (5-1) encompassed by the compound (5) can be also produced, for example, by the following process:

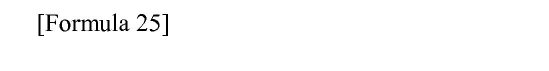

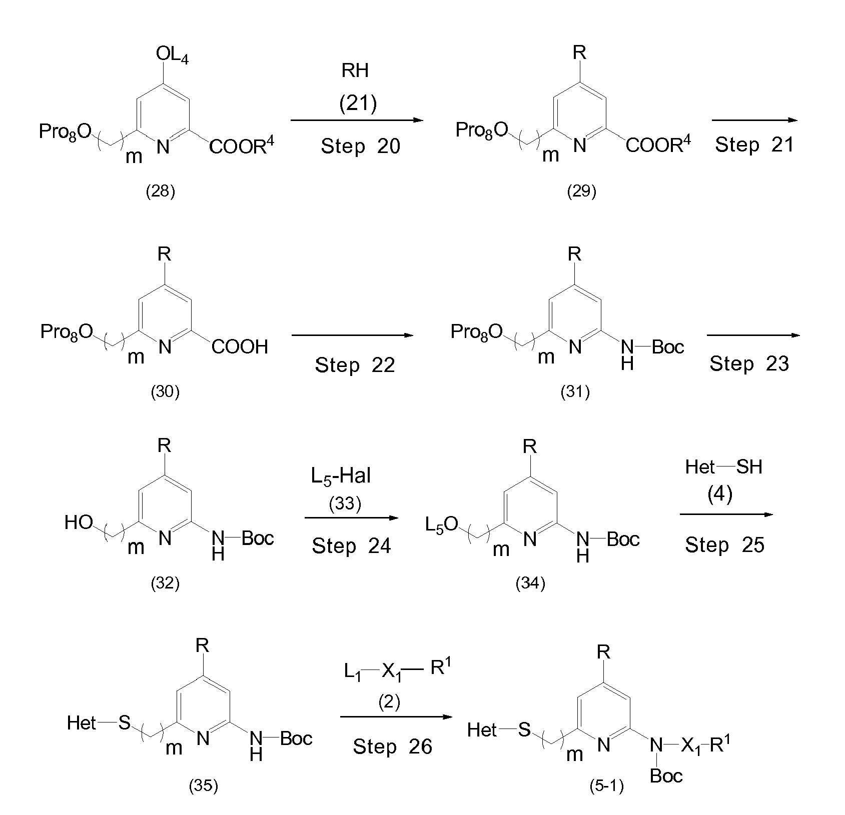

(wherein Prog represents a protective group for hydroxy; R⁴ represents lower alkyl; Hal represents halogen; and the other symbols have the same definitions specified above).

### (Step 20)

This step is a process for producing a compound (29) by reacting a compound (28) with the compound (21) in the presence of a base.

A protective group Pro₈ for hydroxy in the compound (28) includes protective groups as described in, for example, the document (T.W. Green: Protective Groups in Organic Synthesis, Second Edition, John Wiley & Sons (1991)), and protective groups for hydroxy group which are commonly used by those skilled in the art, specifically, for example, tetrahydropyranyl, tetrahydrofuranyl, triethylsilyl and tert-butyldimethylsilyl.

L₅ includes methanesulfonyl, trifluoromethanesulfonyl, p-toluenesulfonyl and p-nitrobenzenesulfonyl.

In addition, R⁴ is preferably ethyl or methyl.

Bases as used in this step include, for example, diisopropylethylamine, triethylamine, potassium carbonate, sodium carbonate, cesium carbonate, sodium hydrogencarbonate, potassium hydrogencarbonate, lithium carbonate, trimethylamine and pyridine.

An amount of the base used is typically 1.0-5.0 equivalents, preferably 1.0-2.0 equivalents, relative to 1 equivalent of the compound (28).

An amount of the compound (21) used is typically 1.0-10.0 equivalents, preferably 1.0-5.0 equivalents, relative to 1 equivalent of the compound (28).

The reaction temperature is typically from room temperature to 150°C, preferably from room temperature to 100°C.

The reaction time is typically 1 hour to 48 hours, preferably 1 hour to 24 hours.

Additives such as molecular sieves may be also added to a reaction system.

Unless interfering with the reaction, any solvent may be used in this step, examples of which include DMF, THF, toluene, chloroform, DMA and DME.

The compound (29) thus obtained may be isolated and purified in well-known separation and purification method such as concentration, concentration under reduced pressure, reprecipitation, solvent extraction, crystallization and chromatography, or subjected to the next step without isolation and purification.

### (Step 21)

This step is a process for producing a compound (30) by removing a protective group R⁴ for carboxyl of the compound (29).

The reaction in this step can be carried out by a method as described in the document (T.W. Green: Protective Groups in Organic Synthesis, Second Edition, John Wiley & Sons (1991)), methods equivalent thereto or combinations of these with usual methods. For example, the compound (30) can be produced by reacting the compound (29) with sodium hydroxide in a methanol solvent.

The compound (30) thus obtained may be isolated and purified in well-known separation and purification method such as concentration, concentration under reduced pressure, reprecipitation, solvent extraction, crystallization and chromatography, or subjected to the next step without isolation and purification.

### (Step 22)

This step is a process for producing a compound (31) by reacting the compound (30), tert-butanol and diphenylphosphoryl azide in the presence of a base.
Bases as used in this step include, for example, trimethylamine, triethylamine and pyridine.

An amount of the base used is typically 1.0-5.0 equivalents, preferably 1.0-2.0 equivalents, relative to 1 equivalent of the compound (30).

The reaction may be carried out in tert-butanol or a mixed solution of tert-butanol and an organic solvent.

Unless interfering with the reaction, any organic solvent may be used, examples of which include toluene, THF, 1,4-dioxane and diethyl ether.

An amount of diphenylphosphoryl azide used is typically 1.0-5.0 equivalents, preferably 1.0-2.0 equivalents, relative to 1 equivalent of the compound (30).

An amount of tert-butanol used is typically from 1.0 equivalent to greatly excessive amount, relative to 1 equivalent of the compound (30).

The reaction temperature is typically from room temperature to 150°C, preferably from room temperature to 100°C.

The reaction time is typically 10 minutes to 24 hours, preferably 10 minutes to 5 hours.

The compound (31) thus obtained may be isolated and purified in well-known separation and purification method such as concentration, concentration under reduced pressure, reprecipitation, solvent extraction, crystallization and chromatography, or subjected to the next step without isolation and purification.

### (Step 23)

This step is a process for producing a compound (32) by removing a protective group Pro₈ for hydroxy of the compound (31).

The reaction in this step can be carried out by a method as described in the document (T.W. Green: Protective Groups in Organic Synthesis, Second Edition, John Wiley & Sons (1991)), methods equivalent thereto or combinations of these with usual methods.

When a tetrahydropyranyl group is used as Pro₈, a compound (32) can be obtained by reacting the compound (31) with p-toluenesulfonic acid or a hydrate thereof in a solvent such as ethanol or methanol.

The compound (32) thus obtained may be isolated and purified in well-known separation and purification method such as concentration, concentration under reduced pressure, reprecipitation, solvent extraction, crystallization and chromatography, or subjected to the next step without isolation and purification.

### (Step 24)

This step is a process for producing a compound (34) by reacting the compound (32) with a compound (33) in the presence of a base.

Bases as used in this step include, for example, trimethylamine, triethylamine, diisopropylethylamine, pyridine, potassium carbonate, cesium carbonate, sodium carbonate, sodium hydrogencarbonate, potassium hydrogencarbonate and lithium carbonate.

An amount of the base used is typically 1.0-5.0 equivalents, preferably 1.0-3.0 equivalents, relative to 1 equivalent of the compound (32).

An amount of the compound (33) used is typically 1.0-5.0 equivalents, preferably 1.0-3.0 equivalents, relative to 1 equivalent of the compound (32).

A compound (33) as used includes, for example, methanesulfonyl chloride, trifluoromethanesulfonyl chloride, p-toluenesulphonyl chloride and p-nitrobenzenesulfonyl chloride.

The reaction temperature is typically from 0°C to 100°C, preferably 0°C to 50°C.

The reaction time is typically 10 minutes to 48 hours, preferably 10 minutes to 24 hours.

Unless interfering with the reaction, any organic solvent may be used, examples of which include THF, DMF, chloroform and methylene chloride.

The compound (34) thus obtained may be isolated and purified in well-known separation and purification method such as concentration, concentration under reduced pressure, reprecipitation, solvent extraction, crystallization and chromatography, or subjected to the next step without isolation and purification.

### (Step 25)

This step is a process for producing a compound (35) by reacting the compound (34) with the compound (4) in the presence of a base.

The reaction in this step can be carried out by the same method as in Step 3 described above, methods equivalent thereto or combinations of these with usual methods.

The compound (35) thus obtained may be isolated and purified in well-known separation and purification method such as concentration, concentration under reduced pressure, reprecipitation, solvent extraction, crystallization and chromatography, or subjected to the next step without isolation and purification.

### (Step 26)

This step is a process for producing a compound (5-1) by reacting the compound (35) with the compound (2) in the presence of a base.

The reaction in this step can be carried out by the same method as in Step 1 described above, methods equivalent thereto or combinations of these with usual methods.

The compound (5-1) thus obtained may be isolated and purified in well-known separation and purification method such as concentration, concentration under reduced pressure, reprecipitation, solvent extraction, crystallization and chromatography, or subjected to the next step without isolation and purification.

The compound (I) according to the present invention can be also produced by the following process.

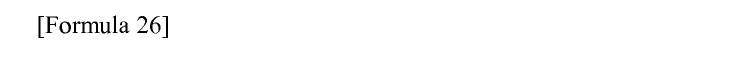

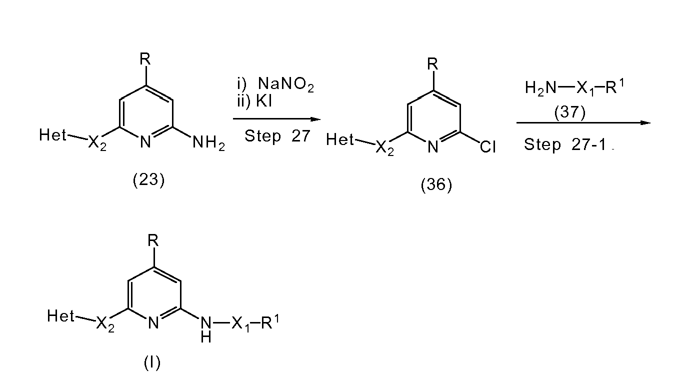

### (Step 27)

This step is a process for producing a compound (36) by reacting the compound (23) with sodium nitrite and then with potassium iodide in an aqueous solution such as hydrochloric acid.

An amount of sodium nitrite used in this step is typically 1.0-5.0 equivalents, preferably 1.0-2.0 equivalents, relative to 1 equivalent of the compound (23).
An amount of potassium iodide used in this step is typically 1.0-5.0 equivalents, preferably 1.0-2.0 equivalents, relative to 1 equivalent of the compound (23).
The reaction temperature is typically from -40°C to room temperature, preferably -10°C to room temperature.
The reaction time is typically 1 hour to 48 hours, preferably 1 hour to 24 hours.
Solvents as used in this step include an aqueous hydrochloric acid solution and an aqueous sulfuric acid solution.

The compound (36) thus obtained may be isolated and purified in well-known separation and purification method such as concentration, concentration under reduced pressure, reprecipitation, solvent extraction, crystallization and chromatography, or subjected to the next step without isolation and purification.

### (Step 28)

This step is a process for producing a compound (I) by reacting the compound (36) with a compound (37) in the presence of a palladium catalyst, a phosphine ligand and a base.

Palladium catalysts as used in this step include, for example, tris(dibenzylideneacetone)palladium(0), palladium acetate(II), palladium trifluoroacetate(II), palladium chloride(II), 1,1'-bis(diphenylphosphino)ferrocenedichloropalladium(II) and dichlorobis(triphenylphosphine)palladium(II).

An amount of the palladium catalyst used is typically 0.1-1.0 equivalent, preferably 0.1-0.5 equivalent, relative to 1 equivalent of the compound (36).

Phosphine ligands as used in this step include, for example, 2-(di-tert-butylphosphino)biphenyl, 4,5-bis(diphenylphosphino)-9,9-dimethylxanthene, 1,1'-bis(diphenylphosphino)ferrocene and 2,2'-bis(diphenylphosphino)-1,1'-binaphthyl.
An amount of the phosphine ligands used is typically 0.1-2.0 equivalents, preferably 0.1-0.5 equivalent, relative to 1 equivalent of the compound (36).
Bases as used in this step include , for example, potassium phosphate, potassium carbonate, cesium carbonate, tert-butoxypotassium and tert-butoxysodium.

An amount of the base used is typically 1.0-5.0 equivalents, preferably 1.0-3.0 equivalents, relative to 1 equivalent of the compound (36).
An amount of the compound (37) used is typically 1.0-5.0 equivalents, preferably 1.0-2.0 equivalents, relative to 1 equivalent of the compound (36).

The reaction temperature is typically from 0°C to 100°C, preferably from 50°C to 100°C.

The reaction time is typically 10 minutes to 48 hours, preferably 10 minutes to 24 hours.

Unless interfering with the reaction, any solvent may be used in this step, examples of which include ethylene glycol dimethyl ether, 1,4-dioxane, THF, DMF and toluene.
In addition, a compound (28-1) encompassed by the compound (28) and a compound (1-1) encompassed by the compound (1) can be produced, for example, by the following process:

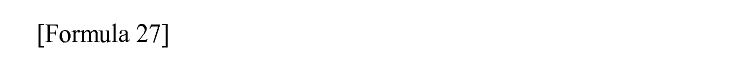

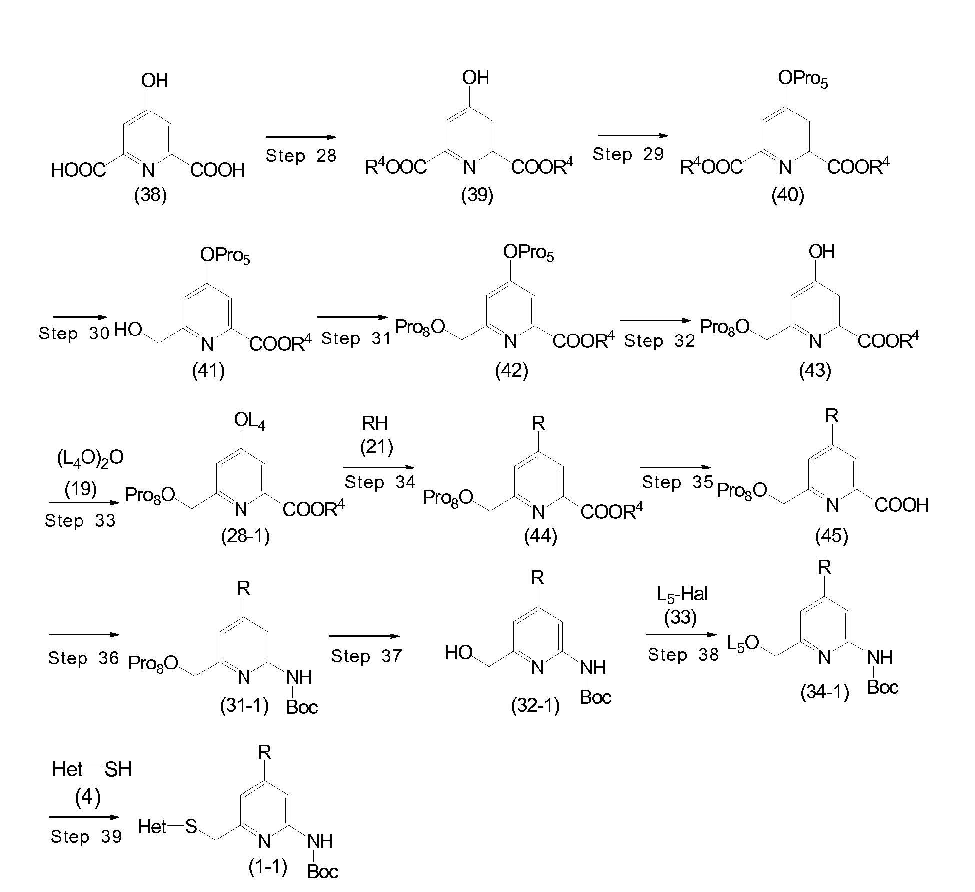

(wherein Boc represents tert-butoxycarbonyl; and the other symbols have the same definitions specified above).

### (Step 28)

This step is a process for producing a compound (39) by reacting 4-hydroxy-2,6-pyridinecarboxylic acid (38) or a hydrate thereof with alcohol R⁴OH in the presence of an acid.

Alcohol compounds R⁴OH as used in this step include methanol and ethanol.

An amount of the alcohol compound R⁴OH used may be a solvent amount, relative to 1 equivalent of the compound (38).

Acids as used in this step include p-toluenesulfonic acid and hydrates thereof.

An amount of the acid used is typically 0.1-2.0 equivalents, preferably 0.1-0.5 equivalent, relative to 1 equivalent of the compound (38).
The reaction temperature is typically from room temperature to 150°C, preferably from 50°C to 100°C.
The reaction time is typically 10 minutes to 48 hours, preferably 10 minutes to 24 hours.
An alcohol compound R⁴OH is used as a solvent used in this step.

The compound (39) thus obtained may be isolated and purified in well-known separation and purification method such as concentration, concentration under reduced pressure, reprecipitation, solvent extraction, crystallization and chromatography, or subjected to the next step without isolation and purification.

### (Step 29)

This step is a process for producing a compound (40) by introducing a protective group Pro₅ into a hydroxy group of the compound (39).

The reaction in this step can be carried out by a method as described in the document (T.W. Green: Protective Groups in Organic Synthesis, Second Edition, John Wiley & Sons (1991)), methods equivalent thereto or combinations of these with usual methods. Pro₅ includes, for example, a benzyl group.

The compound (40) thus obtained may be isolated and purified in well-known separation and purification method such as concentration, concentration under reduced pressure, reprecipitation, solvent extraction, crystallization and chromatography, or subjected to the next step without isolation and purification.

### (Step 30)

This step is a process for producing a compound (41) by reducing one of two ester groups (-COOR⁴) of the compound (40) to a hydroxymethyl group in the presence of calcium chloride and sodium borohydride.

An amount of calcium chloride used is typically 0.5-2.0 equivalents, preferably 0.5-1.0 equivalent, relative to 1 equivalent of the compound (40).

An amount of sodium borohydride used is typically 0.5-2.0 equivalents, preferably 0.5-1.0 equivalent, relative to 1 equivalent of the compound (40).
The reaction temperature is typically from 0°C to 50°C, preferably from 0°C to room temperature.
The reaction time is typically 10 minutes to 24 hours, preferably 10 minutes to 12 hours.
Unless interfering with the reaction, reaction solvents as used in this step include, but are not limited to, for example, methanol, ethanol, chloroform, methylene chloride and THF, among which ethanol is preferred.

The compound (41) thus obtained may be isolated and purified in well-known separation and purification method such as concentration, concentration under reduced pressure, reprecipitation, solvent extraction, crystallization and chromatography, or subjected to the next step without isolation and purification.

### (Step 31)

This step is a process for producing a compound (42) by introducing a protective group Pro₈ into a hydroxy group of the compound (41) in the presence of an acid catalyst.

The reaction in this step can be carried out by a method as described in the document (T.W. Green: Protective Groups in Organic Synthesis, Second Edition, John Wiley & Sons (1991)), methods equivalent thereto or combinations of these with usual methods. Pro₅ includes, for example, tetrahydropyranyl.

When a tetrahydropyranyl group is introduced into a hydroxy group of the compound (41), the compound (42) in which a hydroxy group of the compound (41) is protected by a tetrahydropyranyl group can be obtained by reacting the compound (41) with 3,4-dihydro-2H-pyran in the presence of an acid catalyst such as pyridinium p-toluenesulfonate in a solvent such as chloroform.

The compound (42) thus obtained may be isolated and purified in well-known separation and purification method such as concentration, concentration under reduced pressure, reprecipitation, solvent extraction, crystallization and chromatography, or subjected to the next step without isolation and purification.

### (Step 32)

This step is a process for producing a compound (43) by removing a protective group Pro₅ for hydroxy of the compound (42).

The reaction in this step can be carried out by a method as described in the document (T.W. Green: Protective Groups in Organic Synthesis, Second Edition, John Wiley & Sons (1991)), methods equivalent thereto or combinations of these with usual methods.

When Pro₅ is a benzyl group, the compound (43) can be produced with the compound (42), 10% palladium carbon and cyclohexene in a solvent such as methanol.

The compound (43) thus obtained may be isolated and purified in well-known separation and purification method such as concentration, concentration under reduced pressure, reprecipitation, solvent extraction, crystallization and chromatography, or subjected to the next step without isolation and purification.

### (Step 33)

This step is a process for producing a compound (28-1) by reacting the compound (43) with the compound (19) in the presence of a base.

The reaction in this step can be carried out by the same method as in Step 14 described above, methods equivalent thereto or combinations of these with usual methods.

The compound (28-1) thus obtained may be isolated and purified in well-known separation and purification method such as concentration, concentration under reduced pressure, reprecipitation, solvent extraction, crystallization and chromatography, or subjected to the next step without isolation and purification.

### (Step 34)

This step is a process for producing a compound (44) by reacting the compound (28-1) with the compound (21) in the presence of a base.

The reaction in this step can be carried out by the same method as in Step 20 described above, methods equivalent thereto or combinations of these with usual methods.

The compound (44) thus obtained may be isolated and purified in well-known separation and purification method such as concentration, concentration under reduced pressure, reprecipitation, solvent extraction, crystallization and chromatography, or subjected to the next step without isolation and purification.

### (Step 35)

This step is a process for producing a compound (45) by removing a protective group for carboxyl of the compound (44). The reaction in this step can be carried out by a method as described in the document (T.W. Green: Protective Groups in Organic Synthesis, Second Edition, John Wiley & Sons (1991)), methods equivalent thereto or combinations of these with usual methods. For example, R⁴ is lower alkyl such as ethyl, a compound (45) can be produced by reacting the compound (44) with sodium hydroxide in a methanol solvent at 0°C to room temperature.

The compound (45) thus obtained may be isolated and purified in well-known separation and purification method such as concentration, concentration under reduced pressure, reprecipitation, solvent extraction, crystallization and chromatography, or subjected to the next step without isolation and purification.

### (Step 36)

This step is a process for producing a compound (31-1) by reacting the compound (45), tert-butanol and diphenylphosphoryl azide in the presence of a base.

The reaction in this step can be carried out by the same method as in Step 22 described above, methods equivalent thereto or combinations of these with usual methods.

The compound (31-1) thus obtained may be isolated and purified in well-known separation and purification method such as concentration, concentration under reduced pressure, reprecipitation, solvent extraction, crystallization and chromatography, or subjected to the next step without isolation and purification.

### (Step 37)

This step is a process for producing a compound (32-1) by removing a protective group Pro₈ for hydroxy of the compound (31-1). The reaction in this step can be carried out by a method as described in the document (T.W. Green: Protective Groups in Organic Synthesis, Second Edition, John Wiley & Sons (1991)), methods equivalent thereto or combinations of these with usual methods.

The compound (32-1) thus obtained may be isolated and purified in well-known separation and purification method such as concentration, concentration under reduced pressure, reprecipitation, solvent extraction, crystallization and chromatography, or subjected to the next step without isolation and purification.

### (Step 38)

This step is a process for producing a compound (34-1) by reacting the compound (32-1) with the compound (33) in the presence of a base.

The reaction in this step can be carried out by the same method as in Step 24 described above, methods equivalent thereto or combinations of these with usual methods.

The compound (34-1) thus obtained may be isolated and purified in well-known separation and purification method such as concentration, concentration under reduced pressure, reprecipitation, solvent extraction, crystallization and chromatography, or subjected to the next step without isolation and purification.

### (Step 39)

This step is a process for producing the compound (1-1) by reacting the compound (34-1) with the compound (4) or salt thereof in the presence of a base.

The reaction in this step can be carried out by the same method as in Step 3 described above, methods equivalent thereto or combinations of these with usual methods.

The compound (1-1) thus obtained may be isolated and purified in well-known separation and purification method such as concentration, concentration under reduced pressure, reprecipitation, solvent extraction, crystallization and chromatography, or subjected to the next step without isolation and purification.

In addition, a compound (3-1-1) encompassed by the compound (3-1) and a compound (6-1) encompassed by the compound (6) can be produced, for example, by the following process:

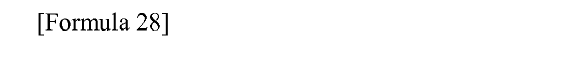

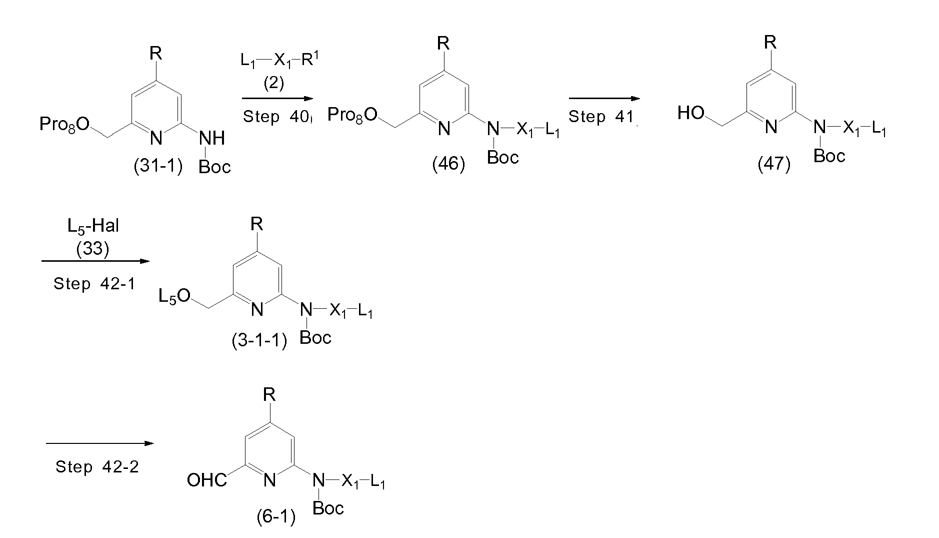

(wherein each symbol has the same definition specified above).

### (Step 40)

This step is a process for producing a compound (46) by reacting the compound (31-1) with the compound (2) in the presence of a base. Instead of the compound (2), a compound represented by a compound (2-1): 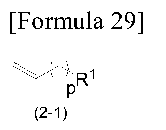 (wherein each symbol has the same definition specified above) may be also used.

The reaction in this step can be carried out by the same method as in Step 1 described above, methods equivalent thereto or combinations of these with usual methods.
The compound (46) thus obtained may be isolated and purified in well-known separation and purification method such as concentration, concentration under reduced pressure, reprecipitation, solvent extraction, crystallization and chromatography, or subjected to the next step without isolation and purification.

### (Step 41)

This step is a process for producing a compound (47) by removing a protective group Pro₈ for hydroxy of the compound (46). The reaction in this step can be carried out by a method as described in the document (T.W. Green: Protective Groups in Organic Synthesis, Second Edition, John Wiley & Sons (1991)), methods equivalent thereto or combinations of these with usual methods.

The compound (47) thus obtained may be isolated and purified in well-known separation and purification method such as concentration, concentration under reduced pressure, reprecipitation, solvent extraction, crystallization and chromatography, or subjected to the next step without isolation and purification.

### (Step 42-1)

This step is a process for producing a compound (3-1-1) by reacting the compound (47) with the compound (33) in the presence of a base.

The process in this step can be carried out by the same method as in Step 24 described above, methods equivalent thereto or combinations of these with usual methods.
The compound (3-1-1) thus obtained may be isolated and purified in well-known separation and purification method such as concentration, concentration under reduced pressure, reprecipitation, solvent extraction, crystallization and chromatography, or subjected to the next step without isolation and purification.

### (Step 42-2)

This step is a process for producing a compound (6-1) by reacting the compound (47) with sulfur trioxide-pyridine complex in the presence of a base. An amount of the base used is typically 1.0-5.0 equivalents, preferably 1.0-3.0 equivalents, relative to 1 equivalent of the compound (47).

Examples of the base include triethylamine, trimethylamine and diisopropylethylamine.

An amount of the sulfur trioxide-pyridine complex used is typically 1.0-5.0 equivalents, preferably 1.0-3.0 equivalents, relative to 1 equivalent of the compound (47).

The reaction temperature is typically from 0°C to 100°C, preferably from 0°C to 50°C.

The reaction time is typically 10 minutes to 48 hours, preferably 10 minutes to 24 hours.

Unless interfering with the reaction, any solvent may be used in this step, examples of which include DMSO, chloroform, methylene chloride, THF and diethyl ether.

The compound (6-1) thus obtained may be isolated and purified in well-known separation and purification method such as concentration, concentration under reduced pressure, reprecipitation, solvent extraction, crystallization and chromatography, or subjected to the next step without isolation and purification.

In addition, a compound (9-1) encompassed by the compound (9) can be produced, for example, by the following process:

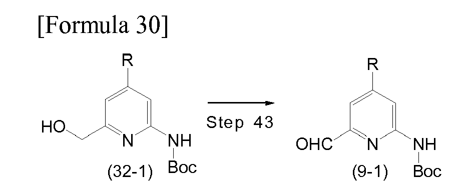

(wherein each symbol has the same definition specified above).

### (Step 43)

This step is a process for producing the compound (9-1) by reacting the compound (32-1) with sulfur trioxide-pyridine complex in the presence of a base.

The reaction in this step can be carried out by the same method as in Step 42-2 described above, methods equivalent thereto or combinations of these with usual methods.

The compound (9-1) thus obtained may be isolated and purified in well-known separation and purification method such as concentration, concentration under reduced pressure, reprecipitation, solvent extraction, crystallization and chromatography, or subjected to the next step without isolation and purification.
In addition, a compound (15-1) encompassed by the compound (15) can be produced, for example, by the following process:

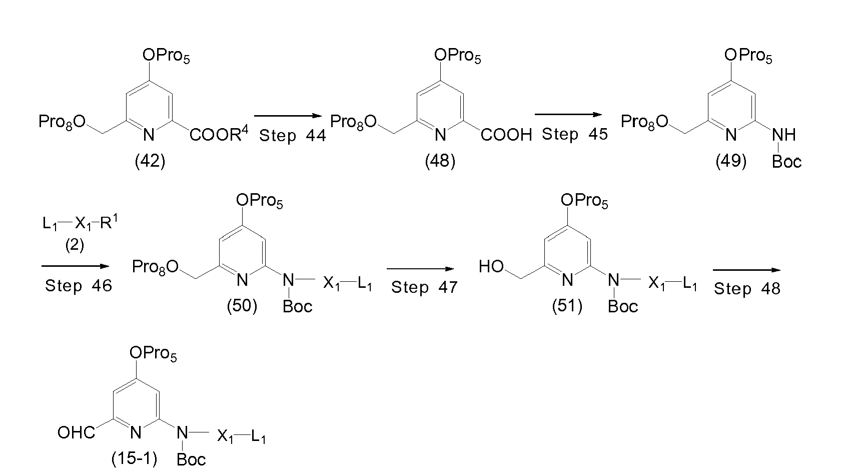

(wherein each symbol has the same definition specified above).

### (Step 44)

This step is a process for producing the compound (48) by removing a protective group for carboxyl of the compound (42).
The reaction in this step can be carried out by the same method as in Step 35 described above, methods equivalent thereto or combinations of these with usual methods.

The compound (48) thus obtained may be isolated and purified in well-known separation and purification method such as concentration, concentration under reduced pressure, reprecipitation, solvent extraction, crystallization and chromatography, or subjected to the next step without isolation and purification.

### (Step 45)

This step is a process for producing a compound (49) by reacting the compound (48), tert-butanol and diphenylphosphoryl azide in the presence of a base.

The reaction in this step can be carried out by the same method as in Step 36 described above, methods equivalent thereto or combinations of these with usual methods.

The compound (49) thus obtained may be isolated and purified in well-known separation and purification method such as concentration, concentration under reduced pressure, reprecipitation, solvent extraction, crystallization and chromatography, or subjected to the next step without isolation and purification.

### (Step 46)

This step is a process for producing a compound (50) by reacting the compound (49) with the compound (2) in the presence of a base. Instead of the compound (2), a compound represented by a compound (2-1):

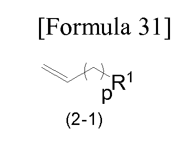

[wherein each symbol has the same definition specified above] may be also used.

The reaction in this step can be carried out by the same method as in Step 1 described above, methods equivalent thereto or combinations of these with usual methods.

The compound (50) thus obtained may be isolated and purified in well-known separation and purification method such as concentration, concentration under reduced pressure, reprecipitation, solvent extraction, crystallization and chromatography, or subjected to the next step without isolation and purification.

### (Step 47)

This step is a process for producing a compound (51) by removing a protective group Pro₈ for hydroxy of the compound (50). The reaction in this step can be carried out by a method as described in the document (T.W. Green: Protective Groups in Organic Synthesis, Second Edition, John Wiley & Sons (1991)), methods equivalent thereto or combinations of these with usual methods.

The compound (51) thus obtained may be isolated and purified in well-known separation and purification method such as concentration, concentration under reduced pressure, reprecipitation, solvent extraction, crystallization and chromatography, or subjected to the next step without isolation and purification.

### (Step 48)

This step is a process for producing a compound (15-1) by reacting the compound (51) with sulfur trioxide-pyridine complex in the presence of a base.

The reaction in this step can be carried out by the same method as in Step 42-2 described above, methods equivalent thereto or combinations of these with usual methods.

The compound (15-1) thus obtained may be isolated and purified in well-known separation and purification method such as concentration, concentration under reduced pressure, reprecipitation, solvent extraction, crystallization and chromatography, or subjected to the next step without isolation and purification.

In addition, a compound (23-1)

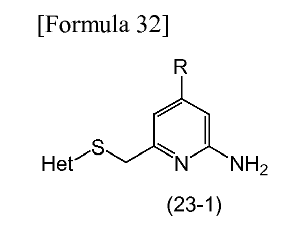

which is encompassed by the compound (23), can be produced by removing a protective group Pro₁ for amino of the compound (1). The protective group Pro₁ for amino can be removed by a method as described in the document (T.W Green: Protective Groups in Organic Synthesis, Second Edition, John Wiley & Sons (1991)), methods equivalent thereto or combinations of these with usual methods.

The compound (23-1) thus obtained may be isolated and purified in well-known separation and purification method such as concentration, concentration under reduced pressure, reprecipitation, solvent extraction, crystallization and chromatography, or subjected to the next step without isolation and purification.
Alkylaminopyridine derivatives provided by the present invention may be present as pharmaceutically acceptable salts, which can be produced according to usual methods using the compound (I) according to the present invention or the compound represented by the formula (I-1), (1-2), (1-3) or (1-4) encompassed by the compound (I).

Specifically, when the compound according to the formula (I), (I-1), (1-2), (1-3) or (1-4) has a basic group derived from, for example, an amino or pyridyl group, in the molecule, the compound can be also converted into a corresponding pharmaceutically acceptable salt by processing the compound with an acid.
Examples of such acid addition salts include hydrohalic acid salts such as hydrochloride, hydrofluorate, hydrobromide and hydroiodide; inorganic acid salts such as nitride, perchlorate, sulfate, phosphate and carbonate; lower alkyl sulfonate salts such as methanesulfonate, trifluoromethanesulfonate and ethanesulfonate; aryl sulfonates such as benzensuplhonate and p-toluenesulfonate; organic salts such as fumarate, succinate, citrate, tartrate, oxalate and maleate; and acid addition salts of organic acids, for example, amino acids, such as glutamate and aspartate. In addition, when the compound according to the present invention has an acidic group, such as carboxyl, in the group, the compound can be also converted into a corresponding pharmaceutically acceptable salt by processing the compound with a base. Examples of such base addition salts include alkali metal salts such as sodium and potassium; alkaline earth metal salts such as calcium and magnesium; ammonium salts; and salts of organic bases such as guanidine, triethylamine and dicyclohexylamine. Furthermore, the compound according to the present invention may be present in the form of a free compound or any hydrate or solvate of a salt thereof.
Furthermore, in the compound according to the present invention, a stereoisomer or a tautomer, such as an optical isomer, a diastereoisomer or a geometrical isomer, is sometimes present depending on the form of a substituent. It will be appreciated that these isomers are encompassed entirely by compounds according to the present invention. Furthermore, it will be appreciated that any mixture of these isomers is also encompassed by compounds according to the present invention.
The utility of compounds according to the present invention as a medicament is specifically proved, for example, in the following pharmacological examples 1 or 2.

### Pharmacological Test Example 1 (NPY Binding Inhibition Test)

A cDNA sequence encoding a human NPY Y1 receptor [Accession No. L07615] was cloned into expression vectors pEF1x (made by Invitrogen Inc.). The obtained expression vectors were transfected to host cells CHO-K1 NFAT β-Lactamase (Aurora) by cationic lipid method [see Proceedings of the National Academy of Sciences of the United States of America, 84: 7413(1987)] to give NPYY1 receptor expression cells.

A membrane sample prepared from the cells which expressed the NPY Y1 receptor was incubated together with a test compound and [¹²⁵I] peptide YY (manufactured by Amersham) (20,000 cpm) in an assay buffer (HBSS buffer (pH 7.4) containing 20 mM HEPES, 0.5% BSA, 1 mM phenylmethylsulfonylfluoride and 0.1% bacitracin) at 25°C for 2 hours, then filtered through a glass filter GF/C, and washed with 20 mM HEPES buffer (pH 7.4), followed by measuring the radioactivity of the cake on the glass filter with γ counter. Nonspecific binding was measured in the presence of 1 µM peptide YY and a 50% Inhibitory Concentration (IC₅₀) of the test compound against specific [¹²⁵I] peptide YY binding was determined [Endocrinology, 131: 2090(1992)]. The results are summarized in Table 1.

**[Table 1]**

| Test Compound | IC50 (nM) |
|---|---|
| Example 1 | 16 |
| Example 5 | 17 |
| Example 9 | 2.2 |
| Example 12 | 27.5 |
| Example 13 | 44 |
| Example 21 | 26 |
| Example 32 | 10 |
| Example 34 | 6.5 |
| Example 38 | 2.8 |
| Example 48 | 16 |

As shown above, compounds of the present invention potently inhibited peptide YY (NPY homologue) binding to NPYY1 receptors.

Based on the results, a compound (I) according to the present invention is useful as an agent for prevention and/or treatment of various diseases associated with NPY, for example, cardiovascular diseases such as hypertension, nephropathy, cardiac diseases and angiospasm; diseases of central nervous system such as bulimia, depression, epilepsy and dementia; metabolic diseases such as obesity, diabetes mellitus and hormone abnormality, or glaucoma, particularly, for example, as an agent for prevention and/or treatment of bulimia, obesity or diabetes mellitus.

In addition, the affinities of compounds according to the present invention and compounds described in Patent Document 7 described above for human ERG (hERG) were examined. This examination method was carried out according to the method described in documents (for example, Japanese Unexamined Patent Application Publication No. 2004-512268).

The activities of the compounds according to the present invention for human ERG (hERG) are summarized as follows:

**[Table 2]**

| Test Compound | Activity for Human ERG (µM) |
|---|---|
| Example 1 | 33 |
| Example 5 | 16 |
| Example 9 | 5.3 |
| Example 12 | 14 |
| Example 13 | 8.2 |
| Example 21 | 14 |
| Example 32 | 11 |
| Example 34 | 7.3 |
| Example 38 | 7.4 |
| Example 48 | 5.9 |

In contrast, the activities of the compounds described in Patent Document 7 described above for human ERG (hERG) are summarized as follows:

**[Table 3]**

| Test Compound | Activity for Human ERG (nM) |
|---|---|
| 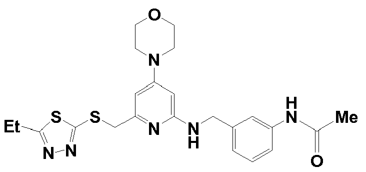 | 570 |
| 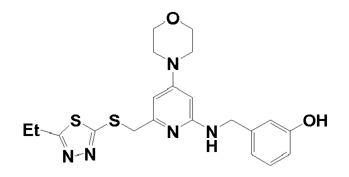 | 160 |
| 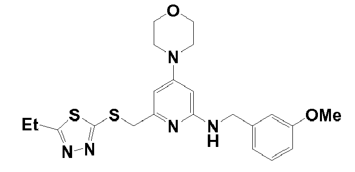 | 28 |

Based on the results, the compounds according to the present invention have extremely low affinities for human ERG (hERG) compared to the compounds described in Patent Document 7 described above, are therefore considered to cause low frequencies of or to hardly cause side effects such as arrhythmia, and are thus extremely useful as medicaments.

Compounds represented by the general formula (I) may be orally or parenterally administered and can be formulated in forms suitable for such administration, resulting in possible provision of the compounds as treatment agents for cardiovascular diseases such as angina pectoris, acute congestive heart failure, myocardial infarction, hypertension, nephropathy, electrolyte abnormality, angiospasm and arteriosclerosis; diseases of central nervous system such as bulimia, depression, anxiety, convulsion, epilepsy, dementia, pain, alcoholism, withdrawal symptoms associated with drug deprivation, circadian rhythm abnormality, schizophrenia, memory disorder, sleep disorder and cognition disorder; metabolic diseases such as obesity, diabetes mellitus, abnormal hormone secretion, hypercholesterolemia, hyperlipidemia, gout and fatty liver; reproductive system diseases such as infertility, premature labor and sexual function disorder; gastrointestinal system diseases; respiratory system diseases; inflammatory diseases; glaucoma; for example, atherosclerosis, hypogonadism, hyperandrogenism, polycystic ovary syndrome, hypertrichosis, gastrointestinal motility disorder, gastroesophageal reflux associated with obesity, obesity hypoventilation syndrome (Pickwickian syndrome), sleep apnea syndrome, inflammation, systemic vasculitis, degenerative arthritis, insulin resistance, bronchoconstriction, alcoholophilia, metabolic syndrome (syndrome X), Alzheimer's disease, cardiac hypertrophy, left ventricular hypertrophy, hypertriglyceridemia and low HDL cholesteremia; cardiovascular diseases such as coronary heart disease (CHD), cerebrovascular disease, stroke, peripheral vascular disease and sudden death; gallbladder disease; cancer (breast cancer, endometrial cancer or colon cancer); breathlessness; hyperuricemia; fertility disorder; lumbago; or anesthetic allergy. When a compound of the present invention is clinically used, a pharmaceutically acceptable additive may be also added, depending on its dosage form, to produce various preparations, followed by administering the preparations. Such additives, for which various additives that are used typically in the field of preparation can be used, include, for example, gelatin, lactose, saccharose, titanium oxide, starch, crystalline cellulose, hydroxypropyl methylcellulose, carboxymethyl cellulose, corn starch, microcrystalline wax, white petrolatum, magnesium aluminometasilicate, anhydrous calcium phosphate, citric acid, trisodium citrate, hydroxypropylcellulose, sorbitol, sorbitan fatty acid ester, polysorbates, sucrose fatty acid esters, polyoxyethylene, hydrogenated castor oil, polyvinyl pyrrolidone, magnesium stearate, light anhydrous silicic acid, talc, vegetable oil, benzyl alcohol, gum arabic, propylene glycol, polyalkylene glycol, cyclodextrin and hydroxypropyl cyclodextrin.

Examples of dosage forms as formulated mixtures with such additives include solid preparations such as tablets, capsules, granules, powder and suppositories; and liquid preparations such as syrups, elixirs and injectables. Such preparations may be formulated according to the techniques well-known in the art of pharmaceutical formulation. Liquid preparations may be in the form of preparations which are dissolved or suspended in water or other appropriate media just before use. In the case of injectable preparations in particular, they may be dissolved or suspended in physiological saline or glucose solution if necessary, optionally together with a buffer and a preservative.
When compounds of the present invention are used clinically, for example, a daily dose for an adult is 0.01-100 mg/kg, preferably 0.03-1 mg/kg in a single dose or in divided doses when administered orally, or 0.001-10 mg/kg, preferably 0.001-0.1 mg/kg, more preferably 0.01-0.1 mg/kg, in a single dose or in divided doses when administered parenterally, though the dose and the frequency of dosage may vary depending upon the sex, age, body weight, the severity of condition of a patient, and the type and range of the desired therapeutic effects.
An ordinarily skilled physician, veterinarian or clinician can readily determine and prescribe the effective amount of the drug required to prevent, suppress or arrest the progress of diseases.
Such preparations may contain a compound of the present invention at a rate of 1.0-100%, preferably 1.0-60%, by weight of the total drug. Such preparations may also contain other therapeutically-effective compounds.

The compounds of the present invention can be used in combination with other agents useful for treating metabolic and/or eating disorders. The individual components of such combinations can be administered separately at different times during the course of therapy or concurrently in divided or single preparations. The present invention is therefore to be understood as embracing all such regimes of simultaneous or alternating treatment and the term "administering" herein is to be interpreted accordingly. It will be understood that the scope of combinations of the compounds of the present invention with other agents useful for treating metabolic and/or eating disorders include in principle any combination with any pharmaceutical composition useful for treating metabolic and/or eating disorders.

Diabetes mellitus is caused by multiple factors and is characterized by elevated levels of plasma glucose (hyperglycemia) in the fasting state. There are two generally recognized forms of diabetes mellitus: type 1 diabetes mellitus, or insulin dependent diabetes mellitus (IDDM) caused by hyposecretion of insulin which is a hormone regulating glucose utilization, and type 2 diabetes mellitus, or non-insulin dependent diabetes mellitus (NIDDM), wherein patients exhibit hyperinsulinemia (plasma insulin levels that are similar or even elevated in comparison with non-diabetic subjects), while at the same time demonstrating hyperglycemia. Type 1 diabetes mellitus is typically treated with exogenous insulin administered via injection. However, type 2 diabetes mellitus often exhibits the phenomena of aggravating insulin resistance, such that the effect of insulin in stimulating glucose and lipid metabolism in the main insulin-sensitive tissues, namely, muscle, liver and adipose tissues, is diminished. In patients with non-insulin dependent diabetes mellitus (NIDDM), the plasma insulin levels, even when they are elevated, are insufficient to overcome the pronounced insulin resistance, resulting in hyperglycemia. Therefore, the treatment with single administration of exogenous insulin becomes difficult.

Insulin resistance is not yet completely understood. Insulin resistance results in insufficient activation of glucose uptake, diminished oxidation of glucose and storage of glycogen in muscle, inadequate repression of lipolysis in adipose tissue and inadequate glucose production and secretion by the liver. The persistent or uncontrolled hyperglycemia that occurs in diabetes mellitus is associated with increased morbidity and mortality. Type 2 diabetes mellitus is at increased risk of developing cardiovascular complications, for example, atherosclerosis, coronary heart disease, stroke, peripheral vascular disease, hypertension, nephropathy, neuropathy and retinopathy.
Non-insulin dependent diabetes is also associated with cardiac hypertrophy, in particular left ventricular hypertrophy (Devereux, R. B., Circulation, 101:2271-2276 (2000)). Cardiac hypertrophy, such as left ventricular hypertrophy, is due to the chronic blood pressure elevation or increased circulating blood volume. Left ventricular hypertrophy (LVH) is characterized by thickening of the left ventricular wall, including increased left ventricular mass, and is defined as a left ventricular mass index exceeding 131 g/m² of the body surface area in men, and 100 g/m² in women [Savage et al., The Framingham Study, Circulation, 75 (1 Pt 2): 26-33 (1987)].

Left ventricular hypertrophy is associated with increased incidence of cardiovascular diseases, such as congestive heart failure, ischemic heart disease, cardiovascular and all-cause mortality, sudden death and stroke. Therefore, regression of left ventricular hypertrophy is associated with a reduction in cardiovascular risk. The incidence of morbid events in patients with progression of left ventricular hypertrophy has been reported to be greater than that in patients with regression of left ventricular hypertrophy.
Current treatments for hypertrophy include non-pharmacological interventions, such as weight reduction, sodium restriction and aerobic physical exercise, can reduce left ventricular mass [Ghali, J. K. et al., American Journal of Geriatric Cardiology, 6:38-49 (1997)].

Many patients who have insulin resistance but have not yet developed type 2 diabetes mellitus are also at a risk of developing metabolic syndrome, also referred to as syndrome X or plurimetabolic syndrome. The period of 5 to 10 years preceding the development of impaired glucose tolerance is associated with a number of hormonal imbalances, which give rise to an enlargement of visceral fat mass, hypertension, insulin resistance and hyperlipidemia [Bjornstop, P., Current Topics in Diabetes Research, eds. Belfore, F., Bergman, R. N., and Molinath, G. M., Front Diabetes, Basel, Karger, 12:182-192 (1993)]. Similarly, metabolic syndrome is characterized by insulin resistance, along with enlargement of visceral fat mass, hyperinsulinemia, hyperglycemia, syndrome X, low HDL and high VLDL. Although the causal relationship between the various components of metabolic syndrome remains to be confirmed, insulin resistance is likely to play an important role [Requen, G. M., et al., N. Eng. J. Med. 334:374-381 (1996); Despres, J-P., et al., N. Engl. J. Med. 334:952-957 (1996); Wajchenberg, B. L., et al., Diabetes/Metabolism Rev. 10:19-29 (1994)]. Patients with metabolic syndrome, whether or not they develop diabetes mellitus, are at increased risk of developing the cardiovascular complications listed above. Associations have been recently reported to be also found between left ventricular hypertrophy and metabolic syndrome [Marcus, R. et al. Circulation, 90:928-936 (1994); Lind, L. et al., J Hypertens. 13:433-38 (1995); Paolisso, G et al., Am J Hypertens., 10:1250-1256 (1997)].

Type 2 diabetes mellitus is treated with a variety of therapeutic agents including PPAR agonists such as glitazones; biguanides; protein tyrosine phosphatase-1B inhibitors; dipeptidyl peptidase IV inhibitors; insulin; insulin mimetics; sulfonylureas; meglitinides; α-glucoside hydrolase inhibitors; and α-amylase inhibitors.
Increasing the plasma level of insulin by administration of sulfonylureas (for example tolbutamide and glipizide) or meglitinides, which stimulate the pancreatic β-cells to secrete more insulin, and by injection of insulin when sulfonylureas or meglitinides become ineffective, can result in insulin concentrations high enough to stimulate insulin-resistant tissues. However, hypoglycemia, dangerously low levels of plasma glucose, can result from, and increasing insulin resistance due to the even higher plasma insulin levels can occur. The biguanides increase insulin sensitivity resulting in some correction of hyperglycemia. Alpha-amylase inhibitors inhibit the enzymatic degradation of starch or glycogen into maltose, have the action of delaying absorption of glucose in the intestine, and also reduce the amounts of bioavailable sugars. Metformin monotherapy is often used for treating the patients with type 2 diabetes mellitus who also develop obesity and/or dyslipidemia. Lack of appropriate response to metformin will be followed by treatment with sulfonylureas, thiazolidinediones, insulin or alpha glucosidase inhibitors. However, the two biguanides, phenformin and metformin, can also induce lactic acidosis and nausea/diarrhea, respectively. Alpha glucosidase inhibitors, such as acarbose, cause intestinal functional disorder.

The glitazones, also known as thiazolidinediones (such as 5-benzylthiazolidine-2,4-diones), are a more recently described class of compounds with potential for a novel mode of action in improving many symptoms of type 2 diabetes mellitus. These agents, which are agonists of the peroxisome proliferator activated receptor (PPAR) gamma subtype, substantially increase insulin sensitivity in muscle, liver and adipose tissue in several animal models of type 2 diabetes mellitus, resulting in partial or complete correction of the elevated plasma glucose levels without inducing hypoglycemia. Newer PPAR agonists that are being developed for treatment of type 2 diabetes mellitus and/or dyslipidemia are agonists of one or more of the PPAR alpha, gamma and delta subtypes.

However, treatment of diabetes mellitus with PPARγ agonists sometimes results in cardiac hypertrophy, or an increase in heart weight. Recent labeling revisions for Avandia (rosiglitazone maleate), a PPARγ agonist, suggest that patients may experience fluid retention and volume-related events, such as edema and congestive heart failure. Cardiac hypertrophy related to PPARγ agonist treatment is likely to be typically treated by withdrawing PPAR treatment.
Treatment of type 2 diabetes mellitus also typically includes weight control with physical exercise and dieting. While physical exercise and reductions in dietary intake of calories will dramatically improve the diabetic condition, compliance with this treatment is very poor because of well-entrenched sedentary lifestyles and excess food consumption, especially of foods containing high amounts of saturated fat. Furthermore, weight reduction by increased exercise is difficult for most patients with diabetes mellitus because the patients may also develop related symptoms.

Abnormal glucose homeostasis is also associated directly or indirectly with obesity, hypertension and lipidosis. Obesity also increases the likelihood of insulin resistance, and the resulting insulin resistance will increase body weight. Therefore, therapeutic control of glucose homeostasis, lipid metabolism, obesity and hypertension are critically important in the clinical management and treatment of diabetes mellitus.
Obesity, which can be defined as a body weight more than 20% above the ideal body weight, is a major health concern in Western societies. It is estimated that one out of three adults in the United States is overweight or obese. Obesity is the result of a positive energy balance, as a consequence of increased ratio of caloric intake to energy consumption. [B. Staels et al., J. Biol. Chem. 270(27), 15958 (1995); F. Lonnquist et al., Nature Medicine 1(9), 950 (1995)]. Although the molecular factors regulating food intake and body weight balance are incompletely understood, several genetic factors have been identified.

Epidemiological studies have shown that increasing degrees of overweight and obesity are important predictors of decreased life expectancy. Obesity causes or exacerbates many health problems, both independently and in association with other diseases. The medical problems associated with obesity, which can be serious and life-threatening, include: type 2 diabetes mellitus; hypertension; hyperinsulinism; insulin resistance; lipidosis; hyperlipidemia; endometrial, breast, prostate, kidney and large intestine cancers; degenerative arthritis; respiratory complications, such as non-obstructive sleep apnea syndrome; gallstone disease; arteriosclerosis; cardiac disease; abnormal heart rhythms; and arrhythmia [Kopelman, P. G., Nature 404, 635-643 (2000)]. Obesity is also associated with metabolic syndrome, circulatory disorder such as cardiac hypertrophy, in particular left ventricular hypertrophy, premature death, a significant increase in mortality from stroke, myocardial infarction, congestive heart failure, coronary heart disease and sudden death.

Abdominal obesity has been linked with coronary artery disease at a high risk, and with three of its major risk factors: high blood pressure, diabetes mellitus that develops in adulthood, and hyperlipidemia. Losing weight dramatically reduces these risks. Abdominal obesity is further closely associated with abnormal glucose tolerance, hyperinsulinemia, hypertriglyceridemia, and other disorders associated with metabolic syndrome (syndrome X), such as decreased levels of high density lipoproteins (HDL) and increased levels of very low density lipoproteins (VLDL) (Montague et al., Diabetes, 2000, 49:883-888).

Obesity and obesity-related diseases, such as diabetes mellitus, are often treated by encouraging patients to lose weight by reducing their food intake or by increasing their exercise level, thereby increasing their energy consumption. A sustained weight loss of 5% to 10% of body weight can lead to improvement of obesity-related diseases such as diabetes mellitus, left ventricular hypertrophy, degenerative arthritis and cardiorespiratory dysfunction.

Weight loss drugs used for the treatment of obesity include orlistat [Davidson, M. H. et al. (1999) JAMA 281:235-42], dexfenfluramine [Guy Grand, B. et al. (1989) Lancet 2:1142-5], sibutramine [Bray, G. A. et al. (1999) Obes. Res. &:189-98] and phentermine [Douglas, A. et al. (1983) Int. J. Obes. 7:591-5]. However, the side effects of such anti-obesity agents may limit their use. Dexfenfluramine was withdrawn from the market because of suspected valvular heart disease; use of orlistat is limited by gastrointestinal side effects; and the use of sibutramine is limited by its cardiovascular side effects which have led to deaths and it was withdrawn from the market in Italy.

The term "diabetes mellitus," as used herein, includes both insulin dependent diabetes mellitus (i.e., IDDM, also known as type 1 diabetes mellitus) and non-insulin dependent diabetes mellitus (i.e., NIDDM, also known as type 2 diabetes mellitus). The compositions of the present invention are useful for treating both type 1 and type 2 diabetes. The compositions are especially effective for treating type 2 diabetes mellitus. The compositions of the present invention are also useful especially for treating and/or preventing gestational diabetes mellitus.

Compounds or combination compositions of the present invention are efficacious for treatment of diabetes mellitus. One outcome of the treatment may be decreasing the glucose level in a subject with elevated glucose levels. Another outcome of the treatment may be decreasing insulin levels in a subject with elevated insulin levels. Another outcome of the treatment is decreasing plasma triglycerides in a subject with elevated plasma triglycerides. Another outcome of the treatment is decreasing LDL cholesterol in a subject with high LDL cholesterol levels. Another outcome of the treatment is increasing HDL cholesterol in a subject with low HDL cholesterol levels. Another outcome of the treatment is increasing insulin sensitivity. Another outcome of the treatment may be enhancing glucose tolerance in a subject with abnormal glucose tolerance. Another outcome of the treatment may be decreasing insulin resistance.

Compounds or combination compositions of the present invention are efficacious for prevention of diabetes mellitus.

The term "hypertension" as used herein includes essential hypertension wherein the cause is not known or where hypertension is due to at least one cause, such as changes in both the heart and blood vessels; and secondary hypertension wherein the cause is known. Causes of secondary hypertension include, but are not limited to, obesity; kidney disease; hormonal disorders; use of certain drugs, such as oral contraceptives, adrenocorticosteroids, cyclosporines, and the like. The term "hypertension" includes high blood pressure, in which both the systolic and diastolic pressure levels are elevated, and isolated systolic hypertension, in which only the systolic pressure is elevated to greater than or equal to 140 mm Hg, while the diastolic pressure is less than 90 mm Hg. One outcome of treatment is decreasing blood pressure in a subject with high blood pressure.

Lipidosis or disorders of lipid metabolism, include various conditions characterized by abnormal concentrations of one or more lipids (for example, cholesterol and triglycerides), and/or apolipoproteins (for example, apolipoproteins A, B, C and E), and/or lipoproteins (for example, macromolecular complexes formed by the lipid and the apolipoprotein that allow lipids to circulate in blood, such as LDL, VLDL and IDL). Hyperlipidemia is associated with abnormally high levels of lipids, LDL and VLDL cholesterol, and/or triglycerides.

The term "metabolic syndrome," also known as syndrome X, is denned in the Third Report of the National Cholesterol Education Program Expert Panel on Detection, Evaluation and Treatment of High Blood Cholesterol in Adults (ATP-III) [E. S. Ford et al., JAMA, vol. 287 (3), Jan. 16, 2002, pp 356-359]. Briefly, a person is defined as having metabolic syndrome if the person has three or more of the following symptoms: abdominal obesity, hypertriglyceridemia, low HDL cholesterol, high blood pressure, and high fasting plasma glucose. The criteria for these are defined in ATP-III.

The term "left ventricular hypertrophy" (LVH) as used herein includes three patterns of left ventricular hypertrophy that have been identified based on left ventricular mass index [LVMI=left ventricular mass (g) divided by body surface area (m²)] and relative wall thickness (RWT=2 X posterior wall thickness/left ventricular end diastolic diameter). The three patterns means: concentric LVH which is typically exemplified by a left ventricular mass index of 144 and a relative wall thickness of 0.52; eccentric LVH which is typically exemplified by a left ventricular mass index of 136 and a relative wall thickness of 0.38; and concentric left ventricular remodeling which is typically exemplified by a LVMI of 93 and a relative wall thickness of 0.38. Normal LVMI is typically 85, and normal RWT is typically about 0.36. Patients with concentric left ventricular (LV) remodeling have a cardiovascular risk intermediate between those with normal left ventricular structure and those with left ventricular hypertrophy.

One outcome of treatment of diabetes mellitus while minimizing cardiac hypertrophy or left ventricular hypertrophy may be a decrease in ventricular mass. Another outcome of treatment of diabetes mellitus while minimizing cardiac hypertrophy or left ventricular hypertrophy may be a decrease in the rate of increase of ventricular mass. Another outcome of treatment of diabetes mellitus while minimizing cardiac hypertrophy or left ventricular hypertrophy may be a decrease in ventricular wall thickness. Another outcome of treatment of diabetes mellitus while minimizing cardiac hypertrophy or left ventricular hypertrophy may be a decrease in the rate of increase in ventricular wall thickness.

The term "obesity" as used herein is a condition in which there is an excess of body fat. The definition of obesity is based on the Body Mass Index (BMI), which is calculated as body weight per height in meters squared (kg/m²). In Europe and the U.S.A., "obesity" refers to a condition whereby a healthy subject has a Body Mass Index (BMI) greater than or equal to 30 kg/m², or a condition whereby a subject with at least one complication has a BMI greater than or equal to 27 kg/m². A "subject at risk of obesity" refers to a healthy subject with a BMI of 25 kg/m² or more but less than 30 kg/m² or a subject with at least one complication with a BMI of 25 kg/m² or more but less than 27 kg/m².

The risks associated with obesity occur at a low Body Mass Index (BMI) in Asians, compared to that in Westerners. In Asian countries, including Japan, "obesity" refers to a condition whereby a subject with at least one obesity-induced or obesity-related complication, that requires weight reduction or that would be improved by weight reduction, has a BMI greater than or equal to 25 kg/m². In Asian countries, a "subject at risk of obesity" is a subject with a BMI of 23 kg/m² or more but less than 25 kg/m².
As used herein, the term "obesity" is meant to encompass all of the above definitions of obesity.

Obesity-induced or obesity-related complications include, but are not limited to, diabetes mellitus, abnormal glucose tolerance, insulin-resistance syndrome, dyslipidemia, hypertension, hyperuricemia, gout, coronary artery disease, myocardial infarction, angina pectoris, sleep apnea syndrome, Pickwickian syndrome, fatty liver, cerebral infarction, cerebral thrombosis, transient ischemic attack, orthopedic disorders, degenerative arthritis, lumbago, emmeniopathy and infertility. In particular, complications include: hypertension, hyperlipidemia, dyslipidemia, abnormal glucose tolerance, cardiovascular diseases, sleep apnea syndrome, diabetes mellitus and other obesity-related conditions.

Treatment of obesity and obesity-related disorders refers to the administration of the compounds or mixture compositions of the present invention to reduce or maintain the body weight of an obese patient. One outcome of treatment may be reducing the body weight of an obese patient relative to that subject's body weight immediately before the administration of the compounds or mixture compositions according to the present invention. Another outcome of treatment may be maintaining body weight previously lost as a result of diet, exercise, or pharmacotherapy. Another outcome of treatment may be decreasing the occurrence of and/or the severity of obesity-related diseases. The treatment may result in a reduction in food and/or calorie intake, including a reduction in total food intake, or a reduction of intake of specific components of the diet such as carbohydrates or fats; and/or the inhibition of nutrient absorption; and/or the inhibition of the reduction of metabolic rate. The treatment may also result in an alteration of metabolic rate, such as an inhibition of the reduction of metabolic rate or an increase in metabolic rate; and/or in minimization of the metabolic resistance that typically results from weight loss.

Prevention of obesity and obesity-related disorders refers to the administration of the compounds or mixture compositions of the present invention to reduce or maintain the body weight of a subj ect at risk of obesity. One outcome of prevention may be reducing the body weight of a subject at risk of obesity relative to subject's body weight immediately before the administration of the compounds or mixture compositions according to the present invention. Another outcome of prevention may be maintaining body weight previously lost as a result of diet, exercise, or pharmacotherapy. Another outcome of prevention may be preventing obesity from occurring if the treatment is administered prior to the onset of obesity in a subject at risk of obesity. Another outcome of prevention may be decreasing the occurrence and/or severity of obesity-related disorders if the treatment is administered prior to the onset of obesity in a subject at risk of obesity. Moreover, if treatment is commenced in already obese subjects, such treatment may prevent the occurrence and progression or reduce severity of obesity-related disorders, such as, but not limited to, arteriosclerosis, type 2 diabetes mellitus, polycystic ovary syndrome, cardiovascular diseases, degenerative arthritis, dermatological disorders, hypertension, insulin resistance, hypercholesterolemia, hypertriglyceridemia and gallstone disease.

The term "atherosclerosis" as used herein includes vascular diseases and conditions that are recognized and understood by physicians practicing in the relevant fields of medicine. Atherosclerosis, coronary heart disease (also known as coronary artery disease or ischemic heart disease), cerebrovascular disease and peripheral vasodilatation diseases are all clinical manifestations of atherosclerosis and are therefore encompassed by the terms "atherosclerosis" and "atherosclerotic disease." The composition of a therapeutically effective amount of an anti-obesity agent in combination with a therapeutically effective amount of an anti-diabetic agent may be administered to prevent or reduce the risk of occurrence or recurrence, of coronary heart disease, cerebrovascular disease or intermittent claudication. Coronary heart disease events are intended to include CHD death, myocardial infarction (such as heart attack) and revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. The term "atherosclerotic disease event" as used herein is intended to encompass coronary heart disease events, cerebrovascular events and intermittent claudication. It is intended that persons who have previously experienced one or more non-fatal atherosclerotic disease events are those for whom the potential for recurrence of such an event exists.

Circadian rhythms affect physiological parameters. Physiological parameters include rest-activity, sleep-wake cycles, body temperature, rhythms in hormone levels, and oscillations in general physiology. When these parameters are out of synchrony with the daily clock, a circadian rhythm imbalance occurs which can affect physiology, performance on a variety of tasks and one's emotional well being. The present invention is useful, for example, in the prevention or treatment of conditions associated with circadian rhythmicity as well as mental and physical disorders associated with travel across time zones and with rotating shift-work schedules.

In another embodiment, the present invention provides a method for the prevention or treatment of a circadian rhythm disorder in a mammal, including syndrome, shift-work sleep disorder, delayed sleep-phase syndrome, advanced sleep-phase syndrome, and non-24-hour sleep-wake disorder.

In another embodiment, the present invention provides a method for shortening the time of re-entrainment (return to normal entrainment of the circadian rhythms; synchronized to the environmental light-dark cycle) in a subject following an irregular shift in the sleep-wake cycle.

In another embodiment, the present invention provides a method for alleviating the effects of jet lag in a traveler. The purpose of this embodiment is to assist the body to adjust physiologically to the changes in sleep and eating patterns when crossing several time zones.

In a preferred embodiment, the present invention provides a method for resetting the internal circadian clock in a patient to match the patient's current activity/sleep cycle. For example, such a method is effective for shift workers changing from a day to a night shift or vice versa.

The present invention provides a method for enhancing or improving sleep quality by increasing sleep efficiency and augmenting sleep maintenance. In addition, the present invention provides a method for preventing and treating sleep disorders and sleep disturbances. The present invention further provides a pharmaceutical composition for enhancing or improving sleep quality and increasing sleep efficiency and sleep stability. The present invention is useful for the treatment of sleep disorders, including Disorders of Initiating and Maintaining Sleep (insomnias) ("DIMS") which can arise from psychophysiological causes, as a consequence of psychiatric disorders(particularly related to anxiety), from drugs and alcohol use and abuse (particularly during drug and alcohol withdrawal stages), childhood onset DIMS, nocturnal myoclonus and restless legs and non specific REM (eye movement) disturbances as seen in ageing.

The following outcomes in a patient which are provided by the present invention may be related to improvement in sleep quality: an increase in value which is calculated from the time that a subject actually sleeps divided by the time that a subject is attempting to sleep; a decrease in sleep latency (the time it takes to fall asleep); a decrease in the number of awakenings during sleep; a decrease in the time spent awake following the initial onset of sleep; an increase in the total amount of sleep; an increase the amount and percentage of REM sleep; an increase in the duration and occurrence of REM sleep; a reduction in the fragmentation of REM sleep; an increase in the amount and percentage of slow-wave (for example, stage 3 or 4) sleep; an increase in the amount and percentage of stage 2 sleep; a decrease in the number of awakenings, especially in the early morning; an increase in daytime alertness; and increased sleep stability. Secondary outcomes which may be provided by the present invention include enhanced cognitive function and increased memory retention. "Method for enhancing the quality of sleeps" refers to a method that results in outcomes in a patient which may be related to enhancement in sleep quality, including, but not limited to, the outcomes correlated to enhancement of sleep quality as defined above.

The present invention is further useful for the prevention and treatment of sleep disorders and sleep disturbances including sleep problems associated with insomnia, hypersomnia, sleep apnea syndrome, narcolepsy, nocturnal myoclonus, REM sleep interruptions, jet-lag, shift workers' sleep disturbances, dysomnias, noctiphobia, night eating syndrome, insomnias associated with depression or with emotional/mood disorders, dysfunctions associated with sleep (parasomnias), as well as sleep walking and enuresis, as well as sleep disorders which accompany aging. Sleep disorders and sleep disturbances are generally characterized by difficulty in initiating or maintaining sleep or in obtaining restful or enough sleep.

In addition, certain drugs may also cause reductions in REM sleep as a side effect and the present invention may be used to correct those types of sleeping disorders as well. The present invention would also be of benefit in the treatment of syndromes such as fibromyalgia which are manifested by non-restorative sleep and muscle pain or sleep apnea which is associated with respiratory disturbances during sleep. It will be clear that the present invention is not limited to just sleep disorders and sleep disturbances, but is applicable to a wide variety of conditions which result from a diminished quality of sleep.

Compounds of the present invention and compositions thereof, or combinations of these and other drugs, are useful for treating and preventing these conditions.

In the present invention, a subject mammal is preferably a human. Although the present invention is applicable for both old and young people, it may find greater application in elderly people. Further, although the invention may be employed to enhance the sleep of healthy people, it may be especially beneficial for enhancing the sleep quality of people suffering from sleep disorders or sleep disturbances.

The compositions according to the present invention may be used in combination with other drugs that may also be useful in the treatment, prevention or control of disorders, such as hypertension, hypertension associated with obesity, hypertension-related disorders, cardiac hypertrophy, left ventricular hypertrophy, and metabolic syndrome, obesity and obesity-related disorders. Such other drugs may be administered, by a route and in an amount commonly used therefore, concurrently or sequentially with a composition according to the present invention. When a composition of the present invention is used concurrently with one or more other drugs, a pharmaceutical composition in unit dosage form containing such other drugs and the composition of the present invention is preferred. However, the combination therapy also includes therapies in which the composition according to the present invention and one or more other drugs are administered on different overlapping dosage schedules.
It is also contemplated that when used in combination with one or more other active ingredients, the composition according to the present invention and the other active ingredients may be used in lower doses than when each is used singly. Accordingly, the pharmaceutical compositions of the present invention include those that contain one or more other active ingredients, in addition to a composition of the present invention.

Examples of other active ingredients that may be administered in combination with a composition according to the present invention, and either administered separately or in the same pharmaceutical composition, include, but are not limited to:
(a) anti-diabetic agents such as (i) PPARγ agonists such as glitazones (for example ciglitazone, darglitazone, englitazone, isaglitazone (MCC-555), pioglitazone, rosiglitazone, troglitazone, BRL49653, CLX-0921 and 5-BTZD), GW-0207, LG-100641 and LY-300512; (ii) biguanides such as buformin, metformin and phenformin; (iii) protein tyrosine phosphatase-1B (PTP-1B) inhibitors; (iv) sulfonylureas such as acetohexamide, chlorpropamide, diabinese, glybenclamide, glipizide, glyburide, glimepiride, gliclazide, glipentide, gliquidone, glisolamide, tolazamide and tolbutamide; (v) meglitinides such as repaglinide and nateglinide; (vi) alpha glucoside hydrolase inhibitors such as acarbose, adiposine, camiglibose, emiglitate, miglitol, voglibose, pradimicin-Q, salbostatin, CKD-711, MDL-25,637, MDL-73,945 and MOR 14; (vii) alpha-amylase inhibitors such as tendamistat, trestatin and Al-3688; (viii) insulin secreatagogues such as linogliride and A-4166; (ix) fatty acid oxidation inhibitors, such as clomoxir and etomoxir; (x) A2 antagonists, such as midaglizole, isaglidole, deriglidole, idazoxan, earoxan and fluparoxan; (xi) insulin or insulin mimetics, such as biota, LP-100, novarapid, insulin detemir, insulin lispro, insulin glargine, insulin zinc suspension (lente and ultralente), Lys-Pro insulin, GLP-1 (73-7) (insulin tropin) and GLP-1 (7-36)-NH₂; (xii) non-thiazolidinediones such as JT-501 and farglitazar (GW-2570/GI-262579); (xiii) PPARα/γ dual agonists such as MK-0767, CLX-0940, GW-1536, GW-1929, GW-2433, KRP-297, L-796449, LR-90 and SB219994; (xiv) other insulin sensitizing drugs; and (xv) VPAC2 receptor agonists;
(b) lipid lowering agents such as (i) bile acid sequestrants such as cholestyramine, colesevelem, colestipol, dialkylaminoalkyl derivatives of a cross-linked dextran, Colestid^{®}, LoCholest^{®} and Questran^{®}; (ii) HMG-CoAreductase inhibitors such as atorvastatin, itavastatin, fluvastatin, lovastatin, pravastatin, rivastatin, rosuvastatin, simvastatin and ZD-4522; (iii) HMG-CoA synthase inhibitors; (iv) cholesterol absorption inhibitors such as stanol esters, beta-sitosterol, sterol glycosides such as tiqueside, and azetidinones such as ezetimibe; (v) acyl coenzyme A-cholesterol acyl transferase (ACAT) inhibitors such as avasimibe, eflucimibe, KY505 and SMP797; (vi) CETP inhibitors such as JTT705, torcetrapib, CP532,632, BAY63-2149, SC591 and SC795; (vii) squalene synthetase inhibitors; (viii) anti-oxidants such as probucol; (ix) PPARα agonists, such as beclofibrate, benzafibrate, ciprofibrate, clofibrate, etofibrate, fenofibrate, gemcabene, gemfibrozil, GW7647, BM170744, LY518674, and other fibric acid derivatives such as Atromid^{®}, Lopid^{®} and Tricor^{®}; (x) FXR receptor modulators such as GW4064 and SR103912; (xi) LXR receptor such as GW3965, T9013137 and XTC0179628; (xii) lipoprotein synthesis inhibitors such as niacin; (xiii) rennin-angiotensin system inhibitors; (xiv) PPARδ partial agonists; (xv) bile acid reabsorption inhibitors, such as BARI1453, SC435, PHA384640, S8921 and AZD7706; (xvi) PPARδ agonists such as GW501516 and GW590735; (xvii) triglyceride synthesis inhibitors; (xviii) microsomal triglyceride transport (MTTP) inhibitors, such as inplitapide, LAB687 and CP346086; (xix) transcription modulators; (xx) squalene epoxidase inhibitors; (xxi) low density lipoprotein (LDL) receptor inducers; (xxii) antiplatelet drugs; (xxiii) 5-LO or FLAP inhibitors; and (xxiv) niacin receptor agonists;
(c) anti-hypertensive agents such as (i) diuretics, such as thiazides, including chlorthalidone, chlorthiazide, dichlorophenamide, hydroflumethiazide, indapamide and hydrochlorothiazide; loop diuretics, such as bumetanide, ethacrynic acid, furosemide and torsemide; potassium sparing agents, such as amiloride and triamterene; and aldosterone antagonists, such as spironolactone and epirenone; (ii) beta-adrenergic blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, propanolol, sotalol, tertatolol, tilisolol and timolol; (iii) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine and verapamil; (iv) angiotensin converting enzyme (ACE) inhibitors such as benazepril, captopril, cilazapril, delapril, enalapril, fosinopril, imidapril, losinopril, moexipril, quinapril, quinaprilat, ramipril, perindopril, perindropril, quanipril, spirapril, tenocapril, trandolapril and zofenopril; (v) neutral endopeptidase inhibitors such as omapatrilat, cadoxatril, ecadotril, fosidotril, sampatrilat, AVE7688 and ER4030; (vi) endothelin antagonists such as tezosentan, A308165 and YM62899; (vii) vasodilators such as hydralazine, clonidine, minoxidil and nicotinyl alcohol; (viii) angiotensin II receptor antagonists such as candesartan, eprosartan, irbesartan, losartan, pratosartan, tasosartan, telmisartan, valsartan, EXP-3137, FI6828K and RNH6270; (ix) α/β adrenergic blockers such as nipradilol, arotinolol and amosulalol; (x) alpha 1 blockers, such as terazosin, urapidil, prazosin, bunazosin, trimazosin, doxazosin, naftopidil, indoramin, WHIP 164 and XEN010; (xi) alpha 2 agonists such as lofexidine, tiamenidine, moxonidine, rilmenidine and guanobenz, and the like; and (xii) aldosterone inhibitors;
(d) anti-obesity agents, such as (i) 5HT (serotonin) transporter inhibitors, such as paroxetine, fluoxetine, fenfluramine, fluvoxamine, sertraline and imipramine; (ii) NE (norepinephrine) transporter inhibitors, such as GW320659, despiramine, talsupram and nomifensine; (iii) CB-1 (cannabinoind-1 receptor) antagonist/inverse agonists, such as rimonabant (Sanofi Synthelabo), SR-147778 (Sanofi Synthelabo), BAY65-2520 (Bayer) and SLV319 (Solvay), and those disclosed in U.S. Pat. Nos. 5,532,237, 4,973,587, 5,013,837, 5,081,122, 5,112, 820, 5,292,736, 5,624,941 and 6,028,084; and WO96/33159, WO98/33765, WO98/43636, WO98/43635, WO01/09120, WO01/96330, WO98/31227, WO98/41519, WO98/37061, WO00/10967, WO00/10968, WO97/29079, WO99/02499, WO01/58869, WO02/076949, WO01/64632, WO01/64633, WO03/006007 and WO03/007887; and EPO Application No. EP-658546; (iv) ghrelin antagonists, such as those disclosed in WO01/87335 and WO02/08250; (v) H3 (histamine H3) antagonist/inverse agonists, such as thioperamide, 3-(1H-imidazol-4-yl)propyl N-(4-pentenyl)carbamate, clobenpropit, iodophenpropit, imoproxifan, GT2394 (Gliatech) and A331440, and those disclosed in WO02/15905; and O-[3-(1H-imidazol-4-yl)propanol]carbamates [Kiec-Kononowicz, K. et al., Pharmazie, 55:349-55 (2000)], piperidine-containing histamine H3-receptor antagonists [Lazewska, D. et al., Pharmazie, 56:927-32(2001)], benzophenone derivatives and related compounds [Sasse, A. et al., Arch. Pharm. (Weinheim) 334:45-52 (2001)], substituted N-phenylcarbamates (Reidemeister, S. et al., Pharmazie, 55:83-6 (2000)], and proxifan derivatives [Sasse, A. et al., J. Med. Chem. 43:3335-43 (2000)]; (vi) melanin-concentrating hormone 1 receptor (MCH1R) antagonists, such as T-226296 (Takeda), SNP-7941 (Synaptic), and those disclosed in WO01/82924, WO01/87834, WO02/051809, WO02/06245, WO02/076929, WO02/076947, WO02/04433, WO02/51809, WO02/083134, WO02/094799, WO03/004027, and Japanese Patent Application No. JP13226269; (vii) MCH2R (melanin concentrating hormone 2R) agonist/antagonists; (viii) NPY1 (neuropeptide Y Y1) antagonists, such as BIBP3226, 2-[1-(5-chloro-3-isopropyloxycarbonylaminophenyl)ethylamino]-6-[2-(5-ethyl-4-methy 1-1,3-thiazol-2-yl)ethyl]-4-morpholinopyridine, BIBO3304, LY-357897, CP-671906 and GI-264879A; and those disclosed in U.S. Pat. No. 6,001,836; and WO96/14307, WO01/23387, WO99/51600, WO01/85690, WO01/85098, WO01/85173 and WO01/89528; (ix) NPY5 (neuropeptide Y Y5) antagonists, such as L-152,804, GW-569180A, GW-594884A, GW-587081X, GW-548118X, FR 235,208, FR-226928, FR240662, FR252384, 1229U91, GI-264879A, CGP71683A, LY-377897, LY366377, PD-160170, SR-120562A, SR-120819A, JCF-104 and H409/22; and those compounds disclosed in U.S. Pat. Nos. 6,140,354, 6,191,160, 6,258,837, 6,313,298, 6,337,332, 6,329,395 and 6,340,683; U.S. Pat. Nos. 6,326,375, 6,329,395, 6,337,332 and 6,335,345; European Patent Nos. EP-01010691 and EP-01044970; and PCT International Patent Publication Nos. WO97/19682, WO97/20820, WO97/20821, WO97/20822, WO97/20823, WO98/27063, WO00/107409, WO00/185714, WO00/185730, WO0O/6488 WO00/68197, WO00/6984 WO01/09120, WO01/14376, WO01/85714, WO01/85730, WO01/07409, WO01/02379, WO01/23388, WO01/23389, WO01/44201, WO01/62737, WO01/62738, WO01/09120, WO02/20488, WO02/22592, WO02/48152, WO02/49648 and WO02/094789; and Norman et al., J. Med. Chem. 43:4288-4312 (2000); (x) leptin, such as recombinant human leptin (PEG-OB, Hoffman La Roche) and recombinant methionyl human leptin (Amgen); (xi) leptin derivatives, such as those disclosed in U.S. Pat. Nos. 5,552,524; 5,552,523; 5,552,522; and 5,521,283; and PCT International Publication Nos. WO96/23513, WO96/23514, WO96/23515, WO96/23516, WO96/23517, WO96/23518, WO96/23519 and WO96/23520; (xii) opioid antagonists, such as nalmefene (Revex^{®}), 3-methoxynaltrexone, naloxone and naltrexone; and those disclosed in WO00/21509; (xiii) orexin antagonists, such as SB-334867-A; and those disclosed in WO01/96302, WO01/68609, WO02/51232, WO02/51838 and WO03/023561; (xiv) BRS3 (bombesin receptor subtype 3) agonists; (xv) CCK-A (cholecystokinin-A) agonists, such as AR-R15849, GI181771, JMV-180, A-71378, A-71623 and SR146131, and those disclosed in U.S. Pat. No. 5,739,106; (xvi) CNTF (ciliary neurotrophic factors), such as GI-181771 (Glaxo-SmithKline); SR146131 (Sanofi Synthelabo); butabindide; and PD 170292 and PD 149164 (Pfizer); (xvii) CNTF derivatives, such as axokine (Regeneron); and WO94/09134, WO98/22128 and WO99/43813; (xviii) GHS (growth hormone secretagogue receptor) agonists, such as NN703, hexarelin, MK-0677, SM-130686, CP-424,391, L-692,429 and L-163,255, and those disclosed in U.S. Pat. No. 6,358,951, U.S. Patent Application Nos. 2002/ 049196 and 2002/022637; and WO01/56592, and WO02/32888; (xix) 5HT2c (serotonin receptor 2c) agonists, such as BVT933, DPCA37215, IK264, PNU22394, WAY161503, R-1065 and YM 348; and those disclosed in U.S. Pat. No. 3,914,250; and WO02/36596, WO02/48124, WO02/10169, WO01/66548, WO02/44152, WO02/51844, WO02/40456 and WO02/40457; (xx) Mc3r (melanocortin 3 receptor) agonists; (xxi) Mc4r (melanocortin 4 receptor) agonists, such as CHIR86036 (Chiron); ME-10142 and ME-10145 (Melacure), and those disclosed in WO99/64002, WO00/74679, WO01/991752, WO01/74844, WO01/70708, WO01/70337, WO01/91752, WO02/059095, WO02/059107, WO02/059108, WO02/059117, WO02/12166, WO02/11715, WO02/12178, WO02/15909, WO02/068387, WO02/068388, WO02/067869, WO03/007949 and WO03/009847; (xxii) monoamine reuptake inhibitors, such as sibutratmine (Meridia^{®}/Reductil^{®}) and a salt thereof, and those compounds disclosed in U.S. Pat. Nos. 4,746,680, 4,806,570 and 5,436,272, and U.S. Patent Publication No. 2002/0006964, and WO01/27068 and WO01/62341; (xxiii) serotonin reuptake inhibitors, such as dexfenfluramine, fluoxetine, and those in U.S. Pat. No. 6,365,633, and WO01/27060 and WO01/162341; (xxiv) GLP-1 (glucagon-like peptide 1) agonists; (xxv) topiramate (Topimax^{®}); (xxvi) phytopharm compound 57 (CP 644,673); (xxvii) ACC2 (acetyl-CoA carboxylase-2) inhibitors; (xxviii) β3 (beta adrenergic receptor 3) agonists, such as AD9677/TAK677 (Dainippon/Takeda), CL-316,243, SB418790, BRL-37344, L-796568, BMS-196085, BRL-35135A, CGP12177A, BTA-243, GW427353, trecadrine, zeneca D7114 and SR59119A, and those disclosed in U.S. Pat. Application Nos. 5,705,515, U.S. Pat. No. 5,451,677, and WO01/74782 and WO02/32897; (xxix) DGAT1 (diacylglycerol acyltransferase 1) inhibitors; (xxx) DGAT2 (diacylglycerol acyltransferase 2) inhibitors; (xxxi) FAS (fatty acid synthase) inhibitors, such as cerulenin and C75; (xxxii) PDE (phosphodiesterase) inhibitors, such as theophylline, pentoxifylline, zaprinast, sildenafil, amrinone, milrinone, cilostamide, rolipram and cilomilast; (xxxiii) thyroid hormone β agonists, such as KB-2611 (KaroBioBMS), and those disclosed in WO02/15845; and Japanese Patent Application No. JP2000256190; (xxxiv) UCP-1 (uncoupling protein 1), 2 or 3 activators, such as phytanic acid, 4-[(E)-2-(5,6,7,8-tetrahydro-5,5,8,8-tetramethyl-2-napthalenyl)-1-propenyl]benzoic acid (TTNPB) and retinoic acid; and those disclosed in WO99/00123; (xxxv) acyl-estrogens, such as oleoyl-estrone, disclosed in del Mar-Grasa, M. et al., Obesity Research, 9:202-9 (2001); (xxxvi) glucocorticoid antagonists; (xxxvii) 11βHSD-1 (11-beta hydroxy steroid dehydrogenase type 1) inhibitors, such as BVT3498, BVT2733, and those compounds disclosed in WO01/90091, WO01/90090 and WO01/90092; (xxxviii) SCD-1 (stearoyl-CoA desaturase-1) inhibitors; (xxxix) dipeptidyl peptidase IV (DP-IV) inhibitors, such as isoleucine thiazolidide, valine pyrrolidide, NVP-DPP728, LAF237, P93/01, TSL225, TMC-2A/2B/2C, FE999011, P9310/K364, VIP0177, SDZ274-444; and the compounds disclosed in WO03/004498, WO03/004496, EP1258476, WO02/083128, WO02/062764, WO03/000250, WO03/002530, WO03/002531, WO03/002553, WO03/002593, WO03/000180 and WO03/000181; (xxxx) lipase inhibitors, such as tetrahydrolipstatin (Orlistat/Xenical^{®}), Triton WR1339, RHC80267, lipstatin, teasaponin, and diethylumbelliferyl phosphate, FL-386, WAY-121898, Bay-N-3176, valilactone, esteracin, ebelactone A, ebelactone B, and RHC80267, and those disclosed in WO01/77094, and U.S. Pat. Nos. 4,598,089, 4,452,813, 5,512,565, 5,391,571, 5,602,151, 4,405,644, 4,189,438 and 4,242,453; (xxxxi) fatty acid transporter inhibitors; (xxxxii) dicarboxylate transporter inhibitors; (xxxxiii) glucose transporter inhibitors; (xxxxiv) phosphate transporter inhibitors; (xxxxv) melanocortin agonists, such as melanotan II or those described in WO99/64002 and WO00/746799; (xxxxvi) melanin concentrating hormone antagonists; (xxxxvii) galanin antagonists; (xxxxviii) CCK agonists; (xxxxix) corticotropin-releasing hormone agonists; and (xxxxx) phosphodiesterase-3B (PDE3B) inhibitors.

The above combinations include combinations of a composition of the present invention not only with one other active compound, but also with two or more other active compounds. There are many examples including combinations of the compositions of the present invention with one, two or more active compounds selected from lipid-lowering agents and anti-hypertensive agents. Combinations of the compositions of the present invention with one, two or more active compounds selected from lipid lowering agents and anti-diabetic agents are useful to treat, control or prevent metabolic syndrome. In particular, compositions including an anti-obesity agent, an anti-hypertensive agent, in addition to an anti-diabetic agent and/or a lipid lowering agent will be useful to synergistically treat, control or prevent metabolic syndrome.

Alkylaminopyridine derivatives according to the present invention, represented by the formula (I), or pharmaceutically acceptable salts, which have a potent antagonistic action to NPY, are useful for treatment and/or prevention of various diseases associated with NPY, for example, cardiovascular diseases such as hypertension, nephropathy, cardiac diseases and angiospasm; cardiovascular diseases such as hypertension, arteriosclerosis, nephropathy, cardiac diseases and angiospasm; diseases of central nervous system such as bulimia, depression, epilepsy, anxiety, alcoholism and dementia; metabolic diseases such as obesity, diabetes mellitus and hormone abnormality, or glaucoma. Since alkylaminopyridine derivatives according to the present invention further have low affinity for hERG compared to aminopyridine derivatives in related art, it is more useful as medicaments.

### Examples

The present invention is further specifically described below referring to Formulation Examples, Examples and Reference Examples, but is not limited thereto.

### Formulation Example 1

The compound (20.0 g) of Example 1, lactose (417 g), crystalline cellulose (80 g) and partially pregelatinized starch (80 g) were mixed using a V-blender. To the mixture was then added magnesium stearate (3.0 g) and the whole was mixed. The mixed powder was tableted in accordance with a conventional method to obtain 3,000 tablets having a diameter of 7.0 mm and a weight of 150 mg per tablet.

### The Content of One Tablet (150 mg)

| | |
|---|---|
| the compound of Example 1 | 5.0 mg |
| lactose | 104.25 mg |
| crystalline cellulose | 20.0 mg |
| partially pregelatinized starch | 20.0 mg |
| magnesium stearate | 0.75 mg |

### Formulation Example 2

In 172.5 grams of purified water were dissolved 10.8 grams of hydroxypropylcellulose 2910 and 2.1 grams of polyethylene glycol 6000. To the solution was dispersed 2.1 grams of titanium dioxide to prepare a coating liquid. Using HICOATER-MINI, 2,500 tablets prepared in Formulation Example 1 were subjected to spray-coating with the coating liquid to provide a film coated tablet with a weight of 155 mg.

### The Content of One Tablet (155 mg)

| | |
|---|---|
| the tablet prepared in Formulation Example 1 | 150 mg |
| hydroxypropylcellulose 2910 | 3.6 mg |
| polyethylene glycol 6000 | 0.7 mg |
| titanium dioxide | 0.7 mg |

In Reference Examples and Examples, thin-layer chromatography employed Silica Gel 60 F₂₅₄ (Merck) as a plate, whereas thin-layer chromatography based on amine employed PLC05 NH (FUJI Silysia) as a plate and a UV detector for a detection method. Wako Gel^{™} C-300 (Wako Pure Chemical Industries) was used for silica gel for column; and a cartridge for FLASH, KP-SIL or KP-NH (Biotage Japan) or Purif-pack SI or Purif-pack NH (Moritex), was used for a charged silica gel column. In addition, NMR spectra were measured using FT NMR "JNM-AL-400" (JEOL); and mass spectra were measured using Quattro II (Micromass).

The meanings of the abbreviations in Examples described below are shown below.
i-Bu: isobutyl
n-Bu: n-butyl
t-Bu: t-butyl
Me: methyl
Et: ethyl
Ph: phenyl
i-Pr: isopropyl
n-Pr: n-propyl
CDCl₃: heavy chloroform
CD₃OD: heavy methanol
DMSO-d₆: heavy dimethylsulfoxide

The meanings of the abbreviations in the nuclear magnetic resonance spectra are shown below.
s: singlet
d: doublet
dd: double doublet
dt: double triplet
t: triplet
m: multiplet
br:broad
brs: broad singlet q: quartet
J: coupling constant
Hz: hertz

### Reference Example 1-1

### 3-bromo-4-oxo-1-piperidine carboxylic acid benzyl eater

In chloroform (1,350 mL) was dissolved 4-oxo-1-piperidinecarboxylic acid benzyl ester (200 g). To the solution was added a solution of bromine (151 g) in chloroform (150 mL) at 0°C over 1 hour, and the mixture was stirred at room temperature for 4 hours. To the reaction solution was added water, and the mixture was extracted with chloroform. The organic layer was washed with a saturated aqueous sodium hydrogencarbonate solution and dried over anhydrous sodium sulfate. Insoluble matters were filtered out, and the filtrate was under reduced pressure to yield the title compound (217 g) as a yellow oil.
mass: 312, 314 (M+1)⁺.

### Reference Example 1-2

### 3-hydroxy-4,4-dimethoxy-1-piperidine carboxylic acid benzyl eater

In methanol (1,800 mL) was dissolved 217 g of 3-bromo-4-oxo-1-piperidine carboxylic acid benzyl ester obtained in Reference Example 1-1. To the solution was added 192 g of potassium carbonate in a state divided into five portions at room temperature over 30 minutes, and the mixture was stirred for 20 hours. The reaction solution was under reduced pressure. To the resultant residue was added water, and the mixture was extracted with ethyl acetate. The organic layer was dried over anhydrous sodium sulfate. Insoluble matters were filtered out, and the filtrate was under reduced pressure. The resultant residue was purified by silica gel column chromatography (hexane/ethyl acetate=88/12) to yield the title compound (59.2 g) as a pale yellow solid.
mass: 296 (M+1)⁺.

### Reference Example 1-3

### 3-(benzyloxy)-4,4-dimethoxy-1-piperidine carboxylic acid benzyl eater

To 10.3 g of 60% sodium hydride was added tetrahydrofuran (315 mL) at 0°C. To the mixture was added a solution of 63.0 g of 3-hydroxy-4,4-dimethoxy-1-piperidine carboxylic acid benzyl ester, obtained in Reference Example 1-2, in tetrahydrofuran (315 mL) over 30 minutes. To the mixture, 30.6 mL of benzyl bromide was then added over 30 minutes. The reaction solution was warmed to room temperature and then heated to reflux for 3 hours. The reaction solution was cooled, and ice was added thereto at 0°C. The mixture was under reduced pressure, and the residue was extracted with ethyl acetate. The organic layer was washed with water and then dried over anhydrous sodium sulfate. Insoluble matters were filtered out, the filtrate was under reduced pressure, and the resultant residue was purified by silica gel column chromatography (hexane/ethyl acetate=92/8) to yield the title compound (46.3 g) as a yellow oil.
mass: 386 (M+1)⁺.

### Reference Example 1-4

### 3-(benzyloxy)-4-oxo-1-piperidine carboxylic acid benzyl eater

In methanol (96.0 mL) was dissolved 48.0 g of 3-(benzyloxy)-4,4-dimethoxy-1-piperidine carboxylic acid benzyl ester obtained in Reference Example 1-3. To the solution was added hydrochloric acid (192 mL), and the mixture was stirred at 40°C for 4 hours. The reaction solution was left standing to cool to room temperature, a saturated saline solution was added thereto, and the mixture was extracted with methylene chloride. The organic layer was washed with a saturated aqueous sodium hydrogencarbonate solution and dried over anhydrous sodium sulfate. Insoluble matters were filtered out, and the filtrate was under reduced pressure to yield the title compound (39.0 g) as a yellow oil.
mass: 340 (M+1)⁺.

### Reference Example 1-5

### 3-(benzyloxy)-4,4-difluoro-1-piperidine carboxylic acid benzyl ester

In methylene chloride (430 mL) was dissolved 43.0 g of 3-(benzyloxy)-4-oxo-1-piperidine carboxylic acid benzyl ester. To the solution was added diethylaminosulfur trifluoride (25.5 mL) at -78°C, and the mixture was stirred at -35°C for 30 minutes. To the reaction solution was added iced water at 0°C, and the mixture was extracted with methylene chloride. The organic layer was washed with a saturated aqueous sodium hydrogencarbonate solution and dried over anhydrous sodium sulfate. Insoluble matters were filtered out, the filtrate was under reduced pressure, and the resultant residue was purified by silica gel column chromatography (hexane/ethyl acetate=96/4) to yield the title compound (15.0 g) as a colorless oil.
mass: 362 (M+1)⁺.

### Reference Example 1-6

### 4,4-difluoro-3-hydroxy-1-piperidine carboxylic acid tert-butyl ester

In tert-butanol (20.0 mL) were suspended di-tert-butyl dicarbonate (12.6 g) and 10% palladium carbon (14.0 g). To the suspension was added a solution of 3-(benzyloxy)-4,4-difluoro-1-piperidine carboxylic acid benzyl ester, obtained in Reference Example 1-5, in tert-butanol (20.0 mL), and the mixture was stirred. To the reaction solution were added tert-butanol (30.0 mL) and methanol (70.0 mL), and the mixture was stirred under hydrogen atmosphere at 60°C and 90 psi for 12 hours. The reaction solution was Celite-filtered, the filtrate was under reduced pressure, and the resultant residue was purified by silica gel column chromatography (methanol/chloroform=2/98) to yield the title compound (4.60 g) as a colorless solid.
mass: 238 (M+1)⁺.

### Reference Example 1-7

### 4,4-difluoro-3-piperidino hydrochloride

To 4,4-difluoro-3-hydroxy-1-piperidine carboxylic acid tert-butyl ester (508 mg) obtained in Reference Example 1-6, 3.00 mL of 10% hydrochloric acid-methanol solution was added, and the mixture was stirred overnight at room temperature. The reaction solution was under reduced pressure to yield the title compound (371 mg) as a white solid.
¹H-NMR(CD₃ OD)δ:2.12-2.25(1H,m),2.42-2.62(1H,m),3.17(1H,dt,J=3.9,12.9Hz),3.27-3.44(3H,m),4.02-4.10(1H,m).

### Reference Example 1-8

### [(2R,5S)-1-benzylpyrrolidin-2,5-dimethanol

In tetrahydrofuran (210 mL) was suspended lithium aluminum hydride (9.94 g). To the suspension was slowly added a solution of (2R,5S)-1-benzyl-2,5-pyrrolidine carboxylic acid diethyl ester (32.0 g) in tetrahydrofuran (50.0 mL), and the mixture was stirred at the same temperature for 3 hours. To the reaction solution was added an excessive amount of sodium sulfate decahydrate, and the mixture was stirred at room temperature for 12 hours. The reaction solution was Celite-filtered, and the filtrate was under reduced pressure to yield the title compound (18.7 g) as a yellow oil.
mass: 222 (M+1)⁺.

### Reference Example 1-9

### 8-benzyl-3-oxa-8-azabiclo[3,2,1]octone hydrochloride

In concentrated sulfuric acid (30.3 mL) and water (10.3 mL), 12.6 g of [(2R,5S)-1-benzylpyrrolidin-2,5-dimethanol obtained in Reference Example 1-8 was dissolved. The solution was heated to reflux at 160°C for 6 hours. The temperature of the reaction solution was returned to room temperature, and the reaction solution was poured into an ice bath. To the liquid was then added a 5N aqueous sodium hydroxide solution under a basic condition, and the mixture was extracted with chloroform. The organic layer was dried over anhydrous sodium sulfate. Insoluble matters were filtered out, and the filtrate was under reduced pressure, followed by adding 4N hydrochloric acid-ethyl acetate to the residue and stirring the mixture at 0°C for 30 minutes. The resultant solid was collected through filtration and washed with ethyl acetate to give the title compound (11.5 g) as a pale white solid.
mass: 204 (M+1)⁺.

### Reference Example 1-10

### 3-oxa-8-azabiclo[3,2,1]octone hydrochloride

In methanol (83.0 mL) was dissolved 8-benzyl-3-oxa-8-azabicyclo[3,2,1]octone hydrochloride (10.0 g) obtained in Reference Example 1-9. To the solution was added 10% palladium carbon (6.24 g), and the mixture was stirred overnight under hydrogen atmosphere at room temperature. The reaction solution was Celite-filtered, and the filtrate was under reduced pressure. The resultant residue was recrystallized from hexane-ethyl acetate to give the title compound (5.63 g) as a white solid.
mass: 114 (M+1)⁺.

### Reference Example 1-11

### [(5-ethyl-4-methyl-1,3-oxazol-2-yl)methyl]phosphonic acid diethyl ester

In concentrated sulfuric acid (20.0 mL) was dissolved N-[(1S)-1-methyl-2-oxobutyl]acetamide (9.78 g), and the solution was stirred at 100°C for 1 hour. The solution was left standing to cool to room temperature, and a 5N aqueous sodium hydroxide solution (about 120 mL) and a saturated aqueous sodium hydrogencarbonate solution were added to the water layer to have a pH of 9, and the mixture was extracted with chloroform. The organic layer was washed with a saturated saline solution and dried over anhydrous sodium sulfate. Insoluble matters were filtered out, and the filtrate was under reduced pressure to give a crude product. To diisopropylamine (18.2 mL) were added a 2.64M n-butyllithium-hexane solution (48.4 mL) and tetrahydrofuran (80.0 mL) at 0°C, and the mixture was stirred for 30 minutes. To the solution was slowly added a solution of the crude product in tetrahydrofuran (20.0 mL) at -78°C for 15 minutes. The mixture was stirred at the same temperature for 30 minutes, diethylchlorophosphate (9.65 mL) was then slowly added to the mixture. The mixture was stirred at the same temperature for 1 hour. Water and a saturated aqueous ammonium chloride solution were added to the reaction solution to be neutral, and the mixture was extracted with ethyl acetate. The organic layer was washed with water and a saturated saline solution and dried over anhydrous sodium sulfate. Insoluble matters were filtered out, and the filtrate was under reduced pressure to give the crude product of the title compound.

### Reference Example 1-12

### [(5-ethyl-4-methyl-1,3-thiazol-2-yl)methyl]phosphonic acid diethyl ester

To diisopropylamine (0.25 mL) were added a 2.64M n-butyllithium-hexane solution (0.66 mL) and tetrahydrofuran (2.00 mL) at 0°C, and the mixture was stirred for 30 minutes. To the solution was slowly added a solution of 5-ethyl-2,4-dimethyl-1,3-thiazole (164 mg) in tetrahydrofuran (2.00 mL) at -78°C for 15 minutes. The mixture was stirred for 30 minutes at the same temperature, followed by slowly adding diethylchlorophosphate (0.17 mL) to the mixture. The mixture was stirred at the same temperature for 1 hour. Water and a saturated aqueous ammonium chloride solution were added to the reaction solution to be neutral, and the mixture was extracted with ethyl acetate. The organic layer was washed with water and a saturated saline solution and dried over anhydrous sodium sulfate. Insoluble matters were filtered out, and the filtrate was under reduced pressure to give the crude product of the title compound.

### Reference Example 1-13

### [(4,5-dimethyl-1,3-oxazol-2-yl)methyl]phosphonic acid diethyl ester

The crude product of the title compound was obtained by the same method as in Reference Example 1-12, methods equivalent thereto or combinations of these with usual methods using 2,4,5-trimethyl-1,3-oxazole instead of 5-ethyl-2,4-dimethyl-1,3-thiazole.

Reference Example 1-14

### 3-(bromoethyl)-1,5-dimethyl-1H-pyrazole

In tetrahydrofuran (10.0 mL) was dissolved (1,5-dimethyl-1H-pyrazol-3-yl)methanol (410 mg). To the solution was added dropwise phosphorus tribromide (2.10 g), and the mixture was stirred at room temperature for 24 hours. The reaction solution was poured on ice, and the mixture was vigorously stirred, then neutralized with sodium carbonate, and extracted with ethyl acetate. The organic layer was dried over magnesium sulfate, insoluble matters were then filtered out, and the filtrate was under reduced pressure. The resultant residue was purified by silica gel column chromatography (hexane/ethyl acetate=1/1) to yield the title compound (514 mg) as a white solid.
mass: 189, 191 (M+1)⁺.

### Reference Example 1-15

### [(1,5-dimethyl-1H-pyrazol-3-yl)methyl]phosphonic acid dimethylester

In acetonitrile (10.0 mL) was dissolved 3-(bromoethyl)-1,5-dimethyl-1H-pyrazole (438 mg) obtained in Reference Example 1-14. To the solution was added dropwise trimethyl phosphite (5.75 g), and the mixture was stirred at 100°C for 8 hours. The reaction solution was left standing to cool to room temperature and then under reduced pressure. To the resultant residue was added a saturated aqueous sodium hydrogencarbonate solution, and the mixture was extracted with chloroform. The organic layer was dried over magnesium sulfate, insoluble matters were then filtered out, and the filtrate was under reduced pressure. The resultant residue was purified by silica gel column chromatography (chloroform/acetone=1/4) to yield the title compound (402 mg).
mass: 219 (M+1)⁺.

### Reference Example 1-16

### 4,5,6,7-tetrahydro-1,3-benzoxazol-2-thiol

In methanol (3.00 mL) was dissolved 2-aminocyclohexane hydrochloride (75.0 mg). To the solution was added triethylamine (84.0 µL), and the mixture was stirred for 30 minutes. To the mixture was added carbon disulfide (180 µL) at 0°C. The mixture was stirred at room temperature for 1.5 hours. The reaction solution was under reduced pressure, 1N hydrochloric acid (1.00 mL) was added thereto, and the mixture was extracted with ethyl acetate. The organic layer was washed with a saturated saline solution and dried over anhydrous sodium sulfate, insoluble matters were then filtered out, and the filtrate was under reduced pressure. The resultant residue was purified by silica gel column chromatography (hexane/ethyl acetate=4/1 to 0/1) to yield the title compound (4.00 mg) as a colorlessness amorphous substance.
mass: 156 (M+1)⁺.

### Reference Example 1-17

### 5-ethyl-4-methyl-1,3-oxazol-2-thiol

The title compound was obtained by the same method as in Reference Example 1-16, methods equivalent thereto or combinations of these with usual methods using 2-amino-3-pentanone hydrochloride instead of 2-aminocyclohexanone hydrochloride.
mass: 144 (M+1)⁺.

### Reference Example 1-18

### N-methylpropanohydrazide

In chloroform (150 mL) was dissolved methylhydrazine (97.0 mL). To the solution was slowly added dropwise a solution of propionic acid chloride (53.0 mL) in chloroform (150 mL) at -78°C, and the mixture was stirred at room temperature for 1.5 hours. The reaction solution was Celite-filtered, and the filtrate was under reduced pressure. The resultant residue was purified by distillation (boiling point: 114-117°C/6 mmHg) to give the title compound (27.0 g) as colorless liquid.
¹H-NMR(CDCl₃)δ:1.10-1.19(3H,m),2.34-2.36(1H,m),2.57-2.63(1H,m),3.18(3H,t,J=10. 9Hz),3.73(1H,brs),4.44(1H,brs).

### Reference Example 1-19

### N-(tert-butyl)-2-methyl-2-propynyl hydrazine carbothioamide

In dimethoxyethane (300 mL) was dissolved N-methylpropanohydrazide (56.0 g) obtained in Reference Example 1-18. To the solution was added t-butylisothiocyanate (84.0 mL), and the mixture was stirred overnight at 100°C. The temperature of the reaction solution was returned to room temperature, the reaction solution was then ice-cooled, and the resultant solid was collected through filtration to give the title compound (81.0 g) as a white solid.
¹H-NMR(CDCl₃ )δ:1.13(3H,t,J=7.4Hz),1.55(9H,s),2.32-2.48(2H,m),3.13(3H,s),6.12(1 H,s), 7.23(1H,s).

### Reference Example 1-20

### 5-ethyl-1-methyl-1H-1,2,4-triazol-3-thiol hydrochloride

In concentrated hydrochloric acid (150 mL) was dissolved N-(tert-butyl)-2-methyl-2-propynyl hydrazine carbothioamide (81.0 g) obtained in Reference Example 1-19, and the mixture was stirred at 100°C for 2 hours. The temperature of the reaction solution was returned to room temperature, the reaction solution was under reduced pressure, and the resultant residue was washed with diethyl ether to give the title compound (55.0 g) as a white solid.
¹H-NMR(CDCl₃ )δ:1.38(3H,t,J=7.4Hz),3.13(2H,t,J=7.4Hz),3.94(3H,s). mass:144(M+1)⁺.

The compounds of Reference Examples 1-21 and 1-22 were obtained by the same methods as in Reference Examples 1-18 to 1-20, methods equivalent thereto or combinations of these with usual methods using the corresponding acid chlorides instead of propionic acid chloride.

### Reference Example 1-21

### 1,5-dimethyl-1H-1,2,4-triazol-3-thiol hydrochloride

The title compound was obtained by the same methods as in Reference Examples 1-18 to 1-20, methods equivalent thereto or combinations of these with usual methods using acetyl chloride instead of propionic acid chloride.
mass: 130 (M+1)⁺.

### Reference Example 1-22

### 5-cyclopropyl-1-methyl-1H-1,2,4-triazol-3-thio hydrochloride

The title compound was obtained by the same methods as in Reference Examples 1-18 to 1-20, methods equivalent thereto or combinations of these with usual methods using cyclopropanecarboxylic acid chloride instead of propionic acid chloride.
mass: 156 (M+1)⁺.

### Reference Example 1-23

### 1,4,5-trimethyl-1H-imidazol-2-thiol

In ethanol (0.75 mL) was dissolved N-methylthiourea (723 mg). To the solution was added acetoin (880 mg), and the mixture was stirred with a microwave reaction apparatus under nitrogen atmosphere at 180°C for 8 minutes and thereafter at 170°C for 1 hour. The temperature of the reaction solution was returned to room temperature, diethyl ether (15 mL) was added to the reaction solution, and the mixture was diluted. The resultant solid was collected through filtration and recrystallized from ethanol to give the title compound (353 mg) as a white solid.
mass: 143 (M+1)⁺.

### Reference Example 1-24

### 1,2-dimethyl-1H-imidazol-4-sulfonyl chloride

To chlorosulfonic acid (20.0 mL) was added 1,2-dimethyl-1H-imidazole (9.60 g) in a state divided into several portions, and the mixture was stirred at 150°C for 3 hours. The reaction solution was left standing to cool to room temperature, thionyl chloride (11.0 mL) was then added thereto, and the mixture was stirred at 100°C for 4 hours. The reaction solution was left standing to cool to room temperature, poured into iced water, and neutralized with sodium carbonate. The resultant solid was collected through filtration, washed with water, then dissolved in chloroform, and dried over anhydrous sodium sulfate. Insoluble matters were filtered out, the filtrate was under reduced pressure, and the resultant residue was recrystallized from chloroform-hexane to give the title compound (5.41 g) as a pale yellow solid.
mass: 195, 197 (M+1)⁺.

### Reference Example 1-25

### 1,2-dimethyl-1H-imidazol-4-thiol hydrochloride

In acetic acid (32.0 mL) was dissolved 1,2-dimethyl-1H-imidazol-4-sulfonyl chloride (1.07 g) obtained in Reference Example 24, and the solution was stirred at 65°C. To the solution was added a solution of tin (II) chloride dihydrate (6.21 g) in concentrated hydrochloric acid (11.0 mL), and the mixture was stirred at the same temperature for 1 hour. The reaction solution was left standing to cool to room temperature, the resultant solid was then collected through filtration, washed with water, and then dried to give the title compound (628 mg) as a yellow solid.
mass: 129 (M+1)⁺.

### Reference Example 1-26

### tributyl(methoxymethyl)stannane

Diisopropylamine (3.20 mL) was dissolved in tetrahydrofuran (30.0 mL), and a 2.64M n-butyllithium-hexane solution (7.50 mL) was added dropwise thereto at -78°C. The reaction solution was warmed to 0°C and stirred for 15 minutes, tri-n-butyltin hydride (4.00 mL) was added dropwise thereto, and the mixture was stirred at the same temperature for 15 minutes. The reaction solution was cooled to -78°C, chloromethyl methyl ether (1.48 mL) was added thereto, and the mixture was stirred at the same temperature for 30 minutes and then stirred overnight at room temperature. The reaction solution was diluted with hexane (70.0 mL), washed with a saturated saline solution, and dried over anhydrous magnesium sulfate. Insoluble matters were filtered, the filtrate was under reduced pressure, and the resultant residue was purified by silica gel column chromatography (hexane/ethyl acetate=1/0 to 10/1) to yield the title compound (2.14 g) as a yellow oil.
mass: 337 (M+1)⁺.

### Reference Example 1-27

### 2-(tert-butoxycarbonylamino)propionaldehyde

In tetrahydrofuran (30.0 mL) was dissolved N-methyl-2-(tert-butoxycarbonylamino)propane hydroxamic acid methyl ester (700 mg). To the solution was added lithium aluminum hydride (172 mg) at -78°C, and the mixture was stirred at the same temperature for 30 minutes, at 0°C for 1 hour and at room temperature for 2 hours. A saturated aqueous ammonium chloride solution (50.0 mL) was added under ice-cooling, the temperature of the mixture was increased to room temperature, and the mixture was extracted with ethyl acetate and dried over anhydrous magnesium sulfate. Insoluble matters were filtered, the filtrate was under reduced pressure, and the resultant residue was purified by silica gel column chromatography (hexane/ethyl acetate=4/1 to 0/1) to yield the title compound (457 mg) as a white solid.
mass: 174 (M+1)⁺.

### Reference Example 1-28

### (2-hydroxy-3-methoxy-1-methylpropyl)carbamic acid tert-butyl ester

In tetrahydrofuran (10.0 mL) was dissolved tributyl(methoxymethyl)stannane (1.00 g) obtained in Reference Example 1-26. To the solution was added dropwise a 2.64M n-butyllithium-hexane solution (1.06 mL) at -78°C, and the mixture was stirred at the same temperature for 30 minutes. To the mixture was added a solution of 2-(tert-butoxycarbonylamino)propionaldehyde (173 mg), obtained in Reference Example 1-27, in tetrahydrofuran (5.00 mL) at -78°C, and the mixture was stirred for 30 minutes. A saturated aqueous ammonium chloride solution was added, the temperature of the mixture was increased to room temperature, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=4/1 to 0/1) to give the title compound (160 mg) as a diastereomeric mixture (cis:trans=1:1), as a colorless amorphous substance.
mass: 242 (M+Na)⁺.

### Reference Example 1-29

### (3-methoxy-1-methyl-2-oxopropyl)carbamic acid tert-butyl ester

In dimethylsulfoxide (10.0 mL) was dissolved (2-hydroxy-3-methoxy-1-methylpropyl)carbamic acid tert-butyl ester (160 mg). To the solution was added triethylamine (0.41 mL), followed by adding sulfur trioxide-pyridine complex (348 mg) and stirring the mixture at room temperature for 2 hours. To the reaction solution was added a saturated aqueous sodium hydrogencarbonate solution, and the mixture was extracted with ethyl acetate, washed with a saturated saline solution, and dried over anhydrous sodium sulfate. Insoluble matters were filtered out, the filtrate was under reduced pressure, and the resultant residue was purified by silica gel column chromatography (hexane/ethyl acetate=9/1 to 3/7) to yield the title compound (128 mg) as a colorless amorphous substance.
mass: 218 (M+1)⁺.

### Reference Example 1-30

### 3-amino-1-methoxy-2-butanone hydrochloride

In a 4N hydrochloric acid-dioxane solution (1.00 mL) was dissolved (3-methoxy-1-methyl-2-oxopropyl)carbamic acid tert-butyl ester (128 mg) obtained in Reference Example 1-29, and the solution was stirred at room temperature for 4 hours. The reaction solution was under reduced pressure. To the resultant residue was added diethyl ether, ultrasonication of the mixture was carried out, and the solvent was removed by concentration under reduced pressure to give a solid. The resultant solid was washed with diethyl ether and dried under reduced pressure to give the title compound (87.6 mg) as a pale orange solid.
mass: 118 (M+1)⁺.

### Reference Example 1-31

### 5-(methoxymethyl)-4-methyl-1,3-oxazol-2-thiol

In methanol (3.00 mL) was dissolved 3-amino-1-methoxy-2-butanone hydrochloride (40.5 mg) obtained in Reference Example 1-30. To the solution was added triethylamine (45.0 µL). To the mixture was then added carbon disulfide (95.0 µL), and the mixture was stirred at 60°C for 3 hours. The temperature of the reaction solution was increased to room temperature, water (10.0 mL) and five drops of 1N hydrochloric acid were added thereto, and the mixture was extracted with ethyl acetate and dried over anhydrous sodium sulfate. Insoluble matters were filtered out, the filtrate was under reduced pressure, and the resultant residue was purified by silica gel thin layer chromatography (hexane/ethyl acetate=1/1) to yield the title compound (5.60 mg) as a pale yellow solid.
mass: 160 (M+1)⁺.

### Reference Example 1-32

### 3,3'-dithiobis(5-methyl-1H-pyrazole)

In tetrahydrofuran (500 mL) was suspended tert-butoxy potassium (22.9 g), and acetone (5.00 mL) was added dropwise to the suspension at room temperature. The mixture was stirred at room temperature for 10 minutes, a solution of carbon disulfide (4.11 mL) in tetrahydrofuran (50.0 mL) was then slowly added dropwise to the mixture at the same temperature, and the mixture was stirred for 2 hours. To the reaction solution was added 4N hydrochloric acid-dioxane solution (51.0 mL) at 0°C, and the mixture was stirred at the same temperature for 30 minutes. Insoluble matters were filtered, and the filtrate was under reduced pressure to give the crude product of 3-oxy-butane dithionic acid. The obtained 3-oxy-butane dithionic acid was dissolved in ethanol (150 mL), and hydrazine monohydrate (3.41 g) was added dropwise to the solution at 0°C. The mixture was stirred for 6 hours by heating under reflux and further stirred at room temperature for 12 hours. The reaction solution was under reduced pressure, a saturated aqueous sodium hydrogencarbonate solution was added to the resultant residue, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=2/1 to 0/1) to yield the title compound (2.03 g) as a dark brown solid.
mass: 227 (M+1)⁺.

### Reference Example 1-33

### 3,3'-dithiobis(1,5-dimethyl-1H-pyrazole)

In dimethylformamide (25.0 mL) was dissolved 3,3'-dithiobis(5-methyl-1H-pyrazole) (1.50 g) obtained in Reference Example 1-32. To the solution were added methyl iodide (1.04 mL) and cesium carbonate (6.48 g), and the mixture was stirred overnight at room temperature. Water was added to the reaction solution, and the mixture was extracted with ethyl acetate, washed with water and a saturated saline solution, and then dried over anhydrous sodium sulfate. Insoluble matters were filtered out, the filtrate was under reduced pressure, and the resultant residue was purified by silica gel column chromatography (hexane/ethyl acetate=2/1 to 0/1) to yield the title compound (492 mg) as a dark brown solid.
mass: 255 (M+1)⁺.

### Reference Example 1-34

### 1,5-dimethyl-1H-pyrazol-3-thiol

In tetrahydrofuran (10.0 mL) was dissolved 3,3'-dithiobis(1,5-dimethyl-1H-pyrazole) (492 mg) obtained in Reference Example 1-33. To the solution was added a 1M aqueous sodium hydrosulfite solution (19.3 mL), and the mixture was stirred at room temperature for 6 hours. The reaction solution was extracted with chloroform, washed with a saturated saline solution, and dried over anhydrous sodium sulfate. Insoluble matters were filtered out, and the filtrate was under reduced pressure to give the crude product (240 mg) of the title compound as a dark brown solid.
mass: 129 (M+1)⁺.

### Reference Example 2-1

### 4-hydroxy-2,6-pyridinedicarboxylic acid diethyl ester

In ethanol (60.0 L) was dissolved 4-hydroxy-2,6-pyridinecarboxylic acid hydrate (3.00 kg). To the solution was added p-toluenesulfonic acid monohydrate (514 g), and the mixture was heated to reflux overnight. The reaction solution was under reduced pressure, a saturated aqueous sodium hydrogencarbonate solution was added to the residue, and the mixture was extracted with chloroform. The organic layer was washed with a saturated saline solution and dried over anhydrous sodium sulfate. Insoluble matters were filtered out, and the filtrate was under reduced pressure to give the title compound (3.48 kg) as a yellow oil.
mass: 240 (M+1)⁺.

### Reference Example 2-2

### 4-(benzyloxy)-2,6-pyridinedicarboxylic acid diethyl ester

In N,N-dimethylformamide (20.0 L) was dissolved 4-hydroxy-2,6-pyridinedicarboxylic acid diethyl ester (3.48 kg) obtained in Reference Example 2-1. To the solution were added potassium carbonate (2.01 kg) and benzyl bromide (1.73 L), and the mixture was stirred at room temperature for 4 hours. Water was added to the reaction solution, and the mixture was extracted with ethyl acetate. The organic layer was washed with a saturated saline solution and dried over anhydrous sodium sulfate. Insoluble matters were filtered out, the filtrate was under reduced pressure, and the resultant residue was recrystallized from heptane-ethyl acetate to give the title compound (3.56 kg) as a white solid.
mass: 330 (M+1)⁺.

### Reference Example 2-3

### 4-(benzyloxy)-6-(hydroxymethyl)-2-pyridinecarboxylic acid ethyl eater

In ethanol (8.90 L) was dissolved 4-(benzyloxy)-2,6-pyridinedicarboxylic acid diethyl ester (1.78 kg) obtained in Reference Example 2-2. To the solution were added calcium chloride (420 g) and sodium borohydride (140 g) at 0°C, and the mixture was stirred at room temperature for 5 hours. To the reaction solution was added 5N hydrochloric acid, and the mixture was under reduced pressure. To the residue was added 1N hydrochloric acid, and the mixture was extracted with chloroform. The organic layer was washed sequentially with water, a saturated aqueous sodium hydrogencarbonate solution and a saturated saline solution, and dried over anhydrous sodium sulfate. Insoluble matters were filtered out, and the filtrate was under reduced pressure. The resultant residue was recrystallized from heptane to give the title compound (1.09 kg) as a white solid.
mass: 288 (M+1)⁺.

### Reference Example 2-4

### 4-(benzyloxy)-6-[(tetrahydro-2H-pyran-2-yloxy)methyl]-2-pyridinecarboxylic acid

### ethyl ester

In chloroform (1,500 mL) was dissolved 4-(benzyloxy)-6-(hydroxymethyl)-2-pyridinecarboxylic acid ethyl ester (300 g). To the solution were added 3,4-dihydro-2H-pyrane (191 mL) and p-toluenesulfonic acid pyridinium (26.2 g), and the mixture was heated to reflux for 3 hours. To the reaction solution was added a saturated aqueous sodium hydrogencarbonate solution, and the mixture was extracted with chloroform. The organic layer was washed with a saturated saline solution and dried over anhydrous sodium sulfate. Insoluble matters were filtered out, and the filtrate was under reduced pressure to give the title compound (388 g) as a yellow oil.
mass: 372 (M+1)⁺.

### Reference Example 2-5

### 4-hydroxy-6-[(tetrahydro-2H-pyran-2-yloxy)methyl]-2-pyridinecarboxylic acid ethyl ester

In methanol (1,900 mL) was dissolved 4-(benzyloxy)-6-[(tetrahydro-2H-pyran-2-yloxy)methyl]-2-pyridinecarboxylic acid ethyl ester (388 g) obtained in Reference Example 2-4. To the solution were added 10% palladium carbon (77.6 g) and cyclohexene (1,060 mL), and the mixture was stirred at 80°C for 1 hour. The reaction solution was Celite-filtered, and the filtrate was under reduced pressure to give the title compound (298 g) as a yellow oil.
mass: 282 (M+1)⁺.

### Reference Example 2-6

### 6-[(tetrahydro-2H-pyran-2-yloxy)methyl]-4-{[(trifluoromethyl)sulfonyl]oxy}-2-pyridin ecarboxylic acid ethyl ester

In chloroform (1,250 mL) were dissolved 4-hydroxy-6-[(tetrahydro-2H-pyran-2-yloxy)methyl]-2-pyridinecarboxylic acid ethyl ester (250 g) obtained in Reference Example 2-5 and triethylamine (149 mL). To the solution was slowly added trifluoromethanesulfonic anhydride (165 mL) at 0°C, and the mixture was stirred at the same temperature for 1 hour. To the reaction solution was added a saturated aqueous sodium hydrogencarbonate solution, and the mixture was extracted with chloroform. The organic layer was washed with a saturated saline solution and dried over anhydrous sodium sulfate. Insoluble matters were filtered out, the filtrate was under reduced pressure, and the resultant residue was purified by NH silica gel column chromatography (chloroform) to yield the title compound (364 g) as a yellow oil.
mass: 414 (M+1)⁺.

### Reference Example 2-7

### 4-(benzyloxy)-6-[(tetrahydro-2H-pyran-2-yloxy)methyl]-2-pyridinecarboxylic acid

In methanol (8.30 L) was dissolved 4-(benzyloxy)-6-[(tetrahydro-2H-pyran-2-yloxy)methyl]-2-pyridinecarboxylic acid ethyl ester (2.07 kg) obtained in Reference Example 2-4. To the solution was added a 2N aqueous sodium hydroxide solution (4.20 L) at 0°C, and the mixture was stirred at room temperature for 1 hour. The reaction solution was under reduced pressure, water was added to the residue, and the mixture was extracted with tert-butyl methyl ether. A water layer was made to have a pH of 4 with 5N hydrochloric acid and extracted with chloroform, and the organic layer was then washed with a saturated saline solution and dried over anhydrous sodium sulfate. Insoluble matters were filtered out, and the filtrate was under reduced pressure to give the title compound (2.04 kg) as a yellow oil.
mass: 342 (M-1)⁻.

### Reference Example 2-8

### {4-(benzyloxy)-6-[(tetrahydro-2H-pyran-2-yloxy)methyl]-2-pyridinyl}carbamic acid tert-butyl ester

In dioxane (8.20 L) were dissolved 4-(benzyloxy)-6-[(tetrahydro-2H-pyran-2-yloxy)methyl]-2-pyridinecarboxylic acid (1.02 kg) obtained in Reference Example 2-7, triethylamine (620 mL) and tert-butanol (2.80 L). To the solution was slowly added diphenylphosphoryl azide (610 mL) at 80°C, and the mixture was stirred at the same temperature for 1 hour. Water was added to the reaction solution, and the mixture was extracted with tert-butylmethylether. The organic layer was washed with water and a saturated saline solution and dried over anhydrous sodium sulfate. Insoluble matters were filtered out, the filtrate was under reduced pressure, and the resultant residue was recrystallized from tert-butylmethylether-heptane to give the title compound (690 g) as a white solid.
mass: 415 (M+1)⁺.

### Reference Example 2-9

### {4-hydroxy-6-[(tetrahydro-2H-pyran-2-yloxy)methyl]-2-pyridinyl}carbamic acid tert-butyl ester

The title compound was obtained by the same method as in Reference Example 2-5, methods equivalent thereto or combinations of these with usual methods using {4-(benzyloxy)-6-[(tetrahydro-2H-pyran-2-yloxy)methyl]-2-pyridinyl}carbamic acid tert-butyl ester instead of 4-(benzyloxy)-6-[(tetrahydro-2H-pyran-2-yloxy)methyl]-2-pyridinecarboxylic acid ethyl ester obtained in Reference Example 2-4.
mass: 325 (M+1)⁺. Reference Example 2-10

### 2-[(tert-butoxycarbonyl)amino]-6-[(tetrahydro-2H-pyran-2-yloxy)methyl]-4-pyridinyl trifluoromethanesulfonate

The title compound was obtained by the same method as in Reference Example 2-6, methods equivalent thereto or combinations of these with usual methods using {4-hydroxy-6-[(tetrahydro-2H-pyran-2-yloxy)methyl]-2-pyridinyl}carbamic acid tert-butyl ester obtained in Reference Example 2-9.
mass: 457 (M+1)⁺.

### Reference Example 2-11

### {4-(4-morpholinyl)-6-[(tetrahydro-2H-pyran-2-yloxy)methyl]-2-pyridinyl}carbamic acid tert-butyl ester

In dimethylsulfoxide (15.0 mL) was dissolved 2-[(tert-butoxycarbonyl)amino]-6-[(tetrahydro-2H-pyran-2-yloxy)methyl]-4-pyridinyl trifluoromethanesulfonate (1.02 g). To the solution was added morpholine (1.17 mL), and the mixture was stirred at 50°C for 21 hours. To the reaction solution was added an aqueous sodium hydrogencarbonate solution, and the mixture was extracted with ethyl acetate. The organic layer was washed with an aqueous sodium hydrogencarbonate solution and a saturated saline solution and dried over anhydrous sodium sulfate. Insoluble matters were filtered, the filtrate was under reduced pressure, and the resultant residue was purified by silica gel column chromatography (hexane/ethyl acetate=1/0 to 1/1) to yield the title compound (879 mg) as a colorless solid.
mass: 394 (M+1)⁺.
The compounds of Reference Examples 2-12 and 2-13 were obtained by the same method as in Reference Example 2-11, methods equivalent thereto or combinations of these with usual methods using the corresponding amines instead of morpholine.

### Reference Example 2-12

### [6-[(tetrahydro-2H-pyran-2-yloxy)methyl]-4-(4-thiomorpholinyl)-2-pyridinyl]carbamic acid tert-butyl ester

The title compound was obtained by the same method as in Reference Example 2-11, methods equivalent thereto or combinations of these with usual methods using thiomorpholine instead of morpholine.
mass: 410 (M+1)⁺.

### Reference Example 2-13

### {4-(1-azepanyl)-6-[(tetrahydro-2H-pyran-2-yloxy)methyl]-2-pyridinyl}carbamic acid tert-butyl ester

The title compound was obtained by the same method as in Reference Example 2-11, methods equivalent thereto or combinations of these with usual methods using homopiperidine instead of morpholine.
mass: 406 (M+1)⁺.

### Reference Example 2-14

### 4-(4,4-difluoro-1-piperidinyl)-6-[(tetrahydro-2H-pyran-2-yloxy)methyl]-2-pyridinecarb oxylic acid ethyl ester

In N,N-dimethylformamide (900 mL) was dissolved 4,4-difluoropiperidine hydrochloride (111 g). To the solution were added N,N-diisopropylethylamine (335 mL) and molecular sieve 4A (powder) (1,300 g), and the mixture was stirred at room temperature for 1 hour. To the reaction solution was added a solution of 6-[(tetrahydro-2H-pyran-2-yloxy)methyl]-4-{[(trifluoromethyl)sulfonyl]oxy}-2-pyridin ecarboxylic acid ethyl ester (264 g), obtained in Reference Example 2-6, in N,N-dimethylformamide (400 mL), and the mixture was stirred overnight at 100°C. The reaction solution was Celite-filtered, and the filtrate was diluted with ethyl acetate. This solution was washed with water and a saturated saline solution and dried over anhydrous sodium sulfate. Insoluble matters were filtered out, the filtrate was under reduced pressure, and the resultant residue was purified by NH silica gel column chromatography (hexane/ethyl acetate=1/1) to yield the title compound (241 g) as a yellow oil.
mass: 385 (M+1)⁺.

### Reference Example 2-15

### 4-(4,4-difluoro-1-piperidinyl)-6-[(tetrahydro-2H-pyran-2-yloxy)methyl]-2-pyridinecarb oxylic acid

In methanol (940 mL) was dissolved 4-(4,4-difluoro-1-piperidinyl)-6-[(tetrahydro-2H-pyran-2-yloxy)methyl]-2-pyridinecarb oxylic acid ethyl ester (241 g) obtained as the compound 2-14. To the solution was added a 2N aqueous sodium hydroxide solution (470 mL) at 0°C, and the mixture was stirred at room temperature for 1 hour. The reaction solution was under reduced pressure, water was added to the resultant residue, and the mixture was extracted with diethyl ether. The water layer was made to have a pH of 4 with 5N hydrochloric acid and extracted with chloroform. The organic layer was washed with a saturated saline solution and dried over anhydrous sodium sulfate. Insoluble matters were filtered out, and the filtrate was under reduced pressure to give the title compound (199 g) as a yellow oil.
mass: 357 (M+1)⁺.

### Reference Example 2-16

### {4-(4,4-difluoro-1-piperidinyl)-6-[(tetrahydro-2H-pyran-2-yloxy)methyl]-2-pyridinyl}c arbamic acid tert-butyl ester

In dioxane (1,600 mL) were dissolved 4-(4,4-difluoro-1-piperidinyl)-6-[(tetrahydro-2H-pyran-2-yloxy)methyl]-2-pyridinecarb oxylic acid (199 g) obtained in Reference Example 2-15, triethylamine (117 mL) and tert-butanol (534 mL). To the solution was slowly added diphenylphosphoryl azide (133 mL) at 80°C, and the mixture was stirred at the same temperature for 2 hours. Water was added to the reaction solution, the mixture was extracted with ethyl acetate, and the organic layer was then washed with water and a saturated saline solution and dried over anhydrous sodium sulfate. Insoluble matters were filtered out, the filtrate was under reduced pressure, and the resultant residue was purified by NH silica gel column chromatography (hexane/ethyl acetate=1/1) to yield the title compound (220 g) as a yellow oil.
mass: 428 (M+1)⁺.

### Reference Example 2-17

### {4-(3-oxa-8-azabicyclo[3,2,1]octo-8-yl)-6-[(tetrahydro-2H-pyran-2-yloxy)methyl]-2-py ridinyl}carbamic acid tert-butyl ester

The title compound was obtained by the same methods as in Reference Examples 2-14 to 2-16, methods equivalent thereto or combinations of these with usual methods using 3-oxa-8-azabicyclo[3.2.1]octone hydrochloride instead of 4,4-difluoropiperidine hydrochloride.
mass: 420 (M+1)⁺.

### Reference Example 2-18

### [6-(hydroxymethyl)-4-(3-oxa-8-azabicyclo[3,2,1]octo-8-yl)-2-pyridinyl]carbamic acid

### tert-butyl ester

In ethanol (30.0 mL) was dissolved {4-(3-oxa-8-azabicyclo[3,2,1]octo-8-yl)-6-[(tetrahydro-2H-pyran-2-yloxy)methyl]-2-py ridinyl}carbamic acid tert-butyl ester (2.00 g) obtained in Reference Example 2-17. To the solution was added p-toluenesulfonic acid monohydrate (998 mg), and the mixture was stirred at 50°C for 2 hours. The reaction solution was under reduced pressure, a saturated aqueous sodium hydrogencarbonate solution was added to the resultant residue, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=9/1 to 1/1) to yield the title compound (1.60 g) as a colorless solid.
mass: 336 (M+1)⁺.

### Reference Example 2-19

### [6-(tert-butoxycarbonyl)amino]-4-(3-oxa-8-azabicyclo[3,2,1]octo-8-yl)-2-pyridinyl]met hyl methanesulfonate

In chloroform (20.0 mL) was dissolved [6-(hydroxymethyl)-4-(3-oxa-8-azabicyclo[3,2,1]octo-8-yl)-2-pyridinyl]carbamic acid tert-butyl ester (1.60 g) obtained in Reference Example 2-18. To the solution were added triethylamine (1.33 mL) and methanesulfonyl chloride (0.45 mL) at 0°C, and the mixture was stirred at the same temperature for 1 hour. To the reaction solution was added a saturated sodium hydrogencarbonate, and the mixture was extracted with chloroform. The organic layer was washed with a saturated saline solution and dried over anhydrous sodium sulfate. Insoluble matters were filtered out, and the filtrate was under reduced pressure to give the title compound (1.97 g) as a colorless oil.
¹ H-NMR(CDCl₃ )δ:1.51(9H,s),1.96-2.17(4H,m),3.05(3H,s),3.56(2H,d,J=10.9Hz),3.80( 2H,d,J=10.9Hz),4.16-4.22(2H,m),5.05(2H,s),6.40(1H,d,J=1.6Hz),7.04(1H,brs),7.23(1H, s).

### Reference Example 2-20

### [6-{[(4,5-dimethyl-1,3-thiazol-2-yl)thio]methyl}-4-(3-oxa-8-azabicyclo[3,2,1]octo-8-yl) -2-pyridinyl]carbamic acid tert-butyl ester

In N,N-dimethylformamide (5.00 mL) was dissolved [6-(tert-butoxycarbonyl)amino]-4-(3-oxa-8-azabicyclo[3,2,1]octo-8-yl)-2-pyridinyl]met hyl methanesulfonate (158 mg) obtained in Reference Example 2-19. To the solution were added potassium carbonate (158 mg) and 4,5-dimethyl-2-mercapto-1,3-thiazole (61.0 mg), and the mixture was stirred at room temperature for 2 hours. Water was added to the reaction solution, the mixture was extracted with ethyl acetate, and the organic layer was then washed with water and a saturated saline solution and dried over anhydrous sodium sulfate. Insoluble matters were filtered out, the filtrate was under reduced pressure, and the resultant residue was purified by silica gel column chromatography (hexane/ethyl acetate=1/0 to 1/1) to yield the title compound (150 mg) as a white solid.
¹ H-NMR(CDCl₃ )δ:1.49(9H,s),1.93-2.14(4H,m),2.28(6H,s),3.51(2H,d,J=10.9Hz),3.76( 2H,d,J=10.9Hz),4.07-4.16(2H,m),4.20(2H,s),6.35(1H,d,J=1.6Hz),7.10(1H,brs),7.15(1H, d,J=1.6Hz).
mass:463(M+1)⁺.

The compounds of Reference Examples 2-21 and 2-22 were obtained by the same method as in Reference Example 2-20, methods equivalent thereto or combinations of these with usual methods using
[6-(tert-butoxycarbonyl)amino]-4-(3-oxa-8-azabicyclo[3,2,1]octo-8-yl)-2-pyridinyl]met hyl methanesulfonate obtained in Reference Example 2-19 and the corresponding thiols instead of 4,5-dimethyl-2-mercaptothiazole.

### Reference Example 2-21

### [6-{[(4,5-dimethyl-1,3-oxazol-2-yl)thio]methyl}-4-(3-oxa-8-azabicyclo[3,2,1]octo-8-yl) -2-pyridinyl]carbamic acid tert-butyl ester

The title compound was obtained by the same method as in Reference Example 2-20, methods equivalent thereto or combinations of these with usual methods using 4,5-dimethyl-1,3-oxazol-2-thiol instead of 4,5-dimethyl-2-mercapto-1,3-thiazole.
mass: 447 (M+1)⁺.

### Reference Example 2-22

### {4-(3-oxa-8-azabicyclo[3,2,1]octo-8-yl)-6-[(4,5,6,7-tetrahydro-1,3-benzoxazol-2-ylthio) methyl]-2-pyridinyl}carbamic acid tert-butyl ester

The title compound was obtained by the same method as in Reference Example 2-20, methods equivalent thereto or combinations of these with usual methods using 2-mercapto-4,5,6,7-tetrahydro-1,3-benzoxazole instead of 4,5-dimethyl-2-mercapto-1,3-thiazole.
mass: 473 (M+1)⁺.

The compounds of Reference Example 2-23 to Reference Example 2-28 were obtained by the same methods as in Reference Example 2-18 to Reference Example 2-20, methods equivalent thereto or combinations of these with usual methods using {4-(4-morpholinyl)-6-[(tetrahydro-2H-pyran-2-yloxy)methyl]-2-pyridinyl} carbamic acid tert-butyl ester obtained in Reference Example 2-11 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using the corresponding thiols instead of 4,5-dimethyl-2-mercapto-1,3-thiazole.

### Reference Example 2-23

### [6-{[(4,5-dimethyl-1,3-thiazol-2-yl)thio]methyl}-4-(4-morpholinyl)-2-pyridinyl]carbam ic acid tert- butyl ester

The title compound was obtained by the same methods as in Reference Example 2-18 to Reference Example 2-20, methods equivalent thereto or combinations of these with usual methods using 4,5-dimethyl-2-mercapto-1,3-thiazole.
mass: 437 (M+1)⁺.

### Reference Example 2-24

### [6-{[(5-ethyl-4-methyl-1,3-oxazol-2-yl)thio]methyl}-4-(4-morpholinyl)-2-pyridinyl]car bamic acid tert-butyl ester

The title compound was obtained by the same methods as in Reference Example 2-18 to Reference Example 2-20, methods equivalent thereto or combinations of these with usual methods using 5-ethyl-4-methyl-1,3-oxazol-2-thiol obtained in Reference Example 1-17.
mass: 435 (M+1)⁺.

### Reference Example 2-25

### 4-(4-morpholinyl)-6-{[(1,4,5-trimethyl-1H-imidazol-2-yl)thio]methyl}-2-pyridinyl)carb amic acid tert-butyl ester

The title compound was obtained by the same methods as in Reference Example 2-18 to Reference Example 2-20, methods equivalent thereto or combinations of these with usual methods using 1,4,5-trimethyl-1H-imidazol-2-thiol obtained in Reference Example 1-23.
mass: 434 (M+1)⁺.

### Reference Example 2-26

### [6-{[(4,5-dimethyl-1,3-oxazol-2-yl)thio]methyl}-4-(4-morpholinyl)-2-pyridinyl]carbam ic acid tert-butyl ester

The title compound was obtained by the same methods as in Reference Example 2-18 to Reference Example 2-20, methods equivalent thereto or combinations of these with usual methods using 4,5-dimethyl-1,3-oxazol-2-thiol.
mass: 421 (M+1)⁺.

### Reference Example 2-27

### [6-{[(1,2-dimethyl-1H-imidazol-4-yl)thio]methyl}-4-(4-morpholinyl)-2-pyridinyl]carba mic acid tert-butyl ester

The title compound was obtained by the same methods as in Reference Example 2-18 to Reference Example 2-20, methods equivalent thereto or combinations of these with usual methods using 1,2-dimethyl-1H-imidazol-4-thiol hydrochloride obtained in Reference Example 1-25.
mass: 420 (M+1)⁺.

### Reference Example 2-28

### [6-({[5-(methoxymethyl)-4-methyl-1,3-oxazol-2-yl]thio}methyl)-4-(4-morpholinyl)-2-p yridinyl]carbamic acid tert-butyl ester

The title compound was obtained by the same methods as in Reference Example 2-18 to Reference Example 2-20, methods equivalent thereto or combinations of these with usual methods using 5-(methoxymethyl)-4-methyl-1,3-oxazol-2-thiol obtained in Reference Example 1-31.
mass: 451 (M+1)⁺.

The compounds of Reference Example 2-29 and Reference Example 2-30 were obtained by the same methods as in Reference Example 2-18 to Reference Example 2-20, methods equivalent thereto or combinations of these with usual methods using [6-[(tetrahydro-2H-pyran-2-yloxy)methyl]-4-(4-thiomorpholinyl)-2-pyridinyl]carbamic acid tert-butyl ester obtained in Reference Example 2-12 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using the corresponding thiols instead of 4,5-dimethyl-2-mercapto-1,3-thiazole.

### Reference Example 2-29

### (4-(4-thiomorpholinyl)-6-{[(1,4,5-trimethyl-1H-imidazol-2-yl)thio]methyl}-2-pyridinyl )carbamic acid tert-butyl ester

The title compound was obtained by the same methods as in Reference Example 2-18 to Reference Example 2-20, methods equivalent thereto or combinations of these with usual methods using 1,4,5-trimethyl-1H-imidazol-2-thiol obtained in Reference Example 1-23 instead of 4,5-dimethyl-2-mercapto-1,3-thiazole.
mass: 450 (M+1)⁺.

### Reference Example 2-30

### [6-{[(4,5-dimethyl-1,3-oxazol-2-yl)thio]methyl}-4-(4-thiomorpholinyl)-2-pyridinyl]car bamic acid tert-butyl ester

The title compound was obtained by the same methods as in Reference Example 2-18 to Reference Example 2-20, methods equivalent thereto or combinations of these with usual methods using 4,5-dimethyl-2-mercapto-1,3-oxazole instead of 4,5-dimethyl-2-mercapto-1,3-thiazole.
mass: 437 (M+1)⁺.

### Reference Example 2-31

### (4-(4,4-difluoro-1-piperidinyl)-6-{[(4,5-dimethyl-1,3-oxazol-2-yl)thio]methyl}-2-pyridi nyl)carbamic acid tert-butyl ester

The title compound was obtained by the same methods as in Reference Example 2-18 to Reference Example 2-20, methods equivalent thereto or combinations of these with usual methods using {4-(4,4-difluoro-1-piperidinyl)-6-[(tetrahydro-2H-pyran-2-yloxy)methyl]-2-pyridinyl}c arbamic acid tert-butyl ester obtained in Reference Example 2-16 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using 4,5-dimethyl-2-mercapto-1,3-oxazole instead of 4,5-dimethyl-2-mercapto-1,3-thiazole.
mass: 455 (M+1)⁺.

### Reference Example 2-32

### {4-(4,4-difluoro-1-piperidinyl)-6-[(tetrahydro-2H-pyran-2-yloxy)methyl]-2-pyridinyl}( 2,2,2-trifluoroethyl)carbamic acid tert-butyl ester

In N,N-dimethylformamide (1,100 mL) was dissolved {4-(4,4-difluoro-1-piperidinyl)-6-[(tetrahydro-2H-pyran-2-yloxy)methyl]-2-pyridinyl}c arbamic acid tert-butyl ester (220 g) obtained in Reference Example 2-16. To the solution was added 60% sodium hydride (41.2 g) at 0°C, and the mixture was stirred at the same temperature for 30 minutes. To the reaction solution was slowly added 2,2,2-trifluoroethyl trifluoromethanesulfonate (185 mL), and the mixture was stirred at room temperature for 1 hour. Water was added to the reaction solution, the mixture was extracted with ethyl acetate, and the organic layer was washed with water and a saturated saline solution and dried over anhydrous sodium sulfate. Insoluble matters were filtered out, the filtrate was under reduced pressure, and the resultant residue was purified by NH silica gel column chromatography (hexane/acetic acid ethyl=4/1) and then recrystallized from hexane to give the title compound (173 g) as a white solid mass: 510 (M+1)⁺.

The compounds of Reference Example 2-33 to Reference Example 2-36 were obtained by the same method as in Reference Example 2-32, methods equivalent thereto or combinations of these with usual methods using the corresponding 2-aminopyridine derivatives instead of {4-(4,4-difluoro-1-piperidinyl)-6-[(tetrahydro-2H-pyran-2-yloxy)methyl]-2-pyridinyl}c arbamic acid tert-butyl ester.

### Reference Example 2-33

### {4-(3-oxa-8-azabicyclo[3,2,1]octo-8-yl)-6-[(tetrahydro-2H-pyran-2-yloxy)methyl]-2-py ridinyl}(2,2,2-trifluoroethyl)carbamic acid tert-butyl ester

The title compound was obtained by the same method as in Reference Example 2-32, methods equivalent thereto or combinations of these with usual methods using {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17.
mass: 502 (M+1)⁺.

### Reference Example 2-34

### {4-(4-morpholinyl)-6-[(tetrahydro-2H-pyran-2-yloxy)methyl]-2-pyridinyl}-(2,2,2-triflu oroethyl)carbamic acid tert-butyl ester

The title compound was obtained by the same method as in Reference Example 2-32, methods equivalent thereto or combinations of these with usual methods using {4-(4-morpholinyl)-6-[(tetrahydro-2H-pyran-2-yloxy)methyl]-2-pyridinyl} carbamic acid tert-butyl ester obtained in Reference Example 2-11.
mass: 476 (M+1)⁺.

### Reference Example 2-35

### {4-(1-azepanyl)-6-[(tetrahydro-2H-pyran-2-yloxy)methyl]-2-pyridinyl}(2,2,2-trifluoroet hyl)carbamic acid tert-butyl ester

The title compound was obtained by the same method as in Reference Example 2-32, methods equivalent thereto or combinations of these with usual methods using {4-(1-azepanyl)-6-[(tetrahydro-2H-pyran-2-yloxy)methyl]-2-pyridinyl}carbamic acid tert-butyl ester obtained in Reference Example 2-13.
mass: 488 (M+1)⁺.

### Reference Example 2-36

### [6-[(tetrahydro-2H-pyran-2-yloxy)methyl]-4-(4-thiomorpholinyl)-2-pyridinyl](2,2,2-trif luoroethyl)carbamic acid tert-butyl ester

The title compound was obtained by the same method as in Reference Example 2-32, methods equivalent thereto or combinations of these with usual methods using [6-[(tetrahydro-2H-pyran-2-yloxy)methyl]-4-(4-thiomorpholinyl)-2-pyridinyl]carbamic acid tert-butyl ester obtained in Reference Example 2-12.
mass: 492 (M+1)⁺.

### Reference Example 2-37

### (cyanomethyl){4-(4,4-difluoro-1-piperidinyl)-6-[(tetrahydro-2H-pyran-2-yloxy)methyl] -2-pyridinyl}carbamic acid tert-butyl ester

The title compound was obtained by the same method as in Reference Example 2-32, methods equivalent thereto or combinations of these with usual methods using {4-(4,4-difluoro-1-piperidinyl)-6-[(tetrahydro-2H-pyran-2-yloxy)methyl]-2-pyridinyl}c arbamic acid tert-butyl ester obtained in Reference Example 2-16 and bromoacetonitrile instead of trifluoromethanesulfonic acid 2,2,2-trifluoroethyl ester.
mass: 467 (M+1)⁺.

### Reference Example 2-38

### (cyanomethyl)[6-{[(4,5-dimethyl-1,3-thiazol-2-yl)thio]methyl}-4-(3-oxa-8-azabicyclo[3 ,2,1]octo-8-yl)-2-pyridinyl]carbamic acid tert-butyl ester

The title compound was obtained by the same method as in Reference Example 2-32, methods equivalent thereto or combinations of these with usual methods using [6- {[(4,5-dimethyl-1,3-thiazol-2-yl)thio]methyl}-4-(3-oxa-8-azabicyclo[3,2,1]octo-8-yl) -2-pyridinyl]carbamic acid tert-butyl ester obtained in Reference Example 2-20 and bromoacetonitrile instead of trifluoromethanesulfonic acid 2,2,2-trifluoroethyl ester.
mass: 502 (M+1)⁺.

### Reference Example 2-39

### {4-(1,1-dioxide-4-thiomorpholinyl)-6-[(tetrahydro-2H-pyran-2-yloxy)methyl]-2-pyridin yl}(2,2,2-trifluoroethyl)carbamic acid tert-butyl ester

In acetone (168 mL) and a 20% aqueous acetic acid solution (84.0 mL) was dissolved [6-[(tetrahydro-2H-pyran-2-yloxy)methyl]-4-(4-thiomorpholinyl)-2-pyridinyl](2,2,2-trif luoroethyl)carbamic acid tert-butyl ester (8.37 g). To the solution was added potassium permanganate (6.78 g) at 0°C, and the mixture was stirred at room temperature for 18 hours. An aqueous sodium sulfite solution was added at 0°C, and the mixture was stirred at the same temperature for 5 minutes. The reaction solution was Celite-filtered, and the filtrate was under reduced pressure. The resultant residue was dissolved in chloroform, and the solution was washed with an aqueous sodium hydrogencarbonate solution and dried over anhydrous sodium sulfate. Insoluble matters were filtered out, the filtrate was under reduced pressure, and the resultant residue was purified by silica gel column chromatography (chloroform/ethyl acetate=1/0 to 7/3) to yield the title compound (7.63 g) as a colorless oil.
mass: 524 (M+1)⁺.

The compounds of Reference Example 2-40 to Reference Example 2-45 were obtained by the same methods as in Reference Example 2-18 to Reference Example 2-20, methods equivalent thereto or combinations of these with usual methods using {4-(4,4-difluoro-1-piperidinyl)-6-[(tetrahydro-2H-pyran-2-yloxy)methyl]-2-pyridinyl}( 2,2,2-trifluoroethyl)carbamic acid tert-butyl ester obtained in Reference Example 2-32 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using the corresponding thiols instead of 4,5-dimethyl-2-mercapto-1,3-thiazole.

### Reference Example 2-40

### (4-(4,4-difluoro-1-piperidinyl)-6-{[(5-methyl-1H-l12,4-triazol-3-yl)thio]methyl}-2-pyri dinyl)(2,2,2-trifluoroethyl)carbamic acid tert-butyl ester

The title compound was obtained by the same methods as in Reference Example 2-18 to Reference Example 2-20, methods equivalent thereto or combinations of these with usual methods using 5-methyl-1H-1,2,4-triazol-3-thiol.
mass: 523 (M+1)⁺.

### Reference Example 2-41

### (4-(4,4-difluoro-1-piperidinyl)-6-{[(1,5-dimethyl-1H-1,2,4-triazol-3-yl)thio]methyl}-2-pyridinyl)(2,2,2-trifluoroethyl)carbamic acid tert-butyl ester

The title compound was obtained by the same methods as in Reference Example 2-18 to Reference Example 2-20, methods equivalent thereto or combinations of these with usual methods using 1,5-dimethyl-1H-1,2,4-triazol-3-thiol hydrochloride obtained in Reference Example 1-21.
mass: 537 (M+1)⁺.

### Reference Example 2-42

### (4-(4,4-(difluoro-1-piperidinyl)-6-{[(5-ethyl-1-methyl-1H-1,2,4-triazo1-3-yl]thiolmethyl }-2-pyridinyl)(2,2,2-trifluoroethyl)carbamic acid tert-butyl ester

The title compound was obtained by the same methods as in Reference Example 2-18 to Reference Example 2-20, methods equivalent thereto or combinations of these with usual methods using 5-ethyl-1-methyl-1H-1,2,4-triazol-3-thiol hydrochloride obtained in Reference Example 1-20.
mass: 551 (M+1)⁺.

### Reference Example 2-43

### (5-cyclopropyl-1-methyl-1H-1,2,4-triazol-3-yl)thio]methyl}-4-(4,4-difluoro-1-piperidin yl)-2-pyridinyl](2,2,2-trifluoroethyl)carbamic acid tert-butyl ester

The title compound was obtained by the same methods as in Reference Example 2-18 to Reference Example 2-20, methods equivalent thereto or combinations of these with usual methods using 5-cyclopropyl-1-methyl-1H-1,2,4-triazol-3-thiol hydrochloride obtained in Reference Example 1-22.
mass: 563 (M+1)⁺.

### Reference Example 2-44

### (4-(4,4-difluoro-1-piperidinyl)-6-{[(1,2-dimethyl-1H-imidazol-4-yl)thio]methyl}-2-pyri dinyl)(2,2,2-trifluoroethyl)carbamic acid tert-butyl ester

The title compound was obtained by the same methods as in Reference Example 2-18 to Reference Example 2-20, methods equivalent thereto or combinations of these with usual methods using 1,2-dimethyl-1H-imidazol-4-thiol hydrochloride obtained in Reference Example 1-25.
mass: 536 (M+1)⁺.

### Reference Example 2-45

### (4-(4,4-difluoro-1-piperidinyl)-6-{[(1,5-dimethyl-1H-pyrazol-3-yl)thio]methyl}-2-pyrid inyl)(2,2,2-trifluoroethyl)carbamic acid tert-butyl ester

The title compound was obtained by the same methods as in Reference Example 2-18 to Reference Example 2-20, methods equivalent thereto or combinations of these with usual methods using 1,5-dimethyl-1H-pyrazol-3-thiol obtained in Reference Example 1-34.
mass: 536 (M+1)⁺.

The compounds of Reference Example 2-46 to Reference Example 2-48 were obtained by the same methods as in Reference Example 2-18 to Reference Example 2-20, methods equivalent thereto or combinations of these with usual methods using {4-(3-oxa-8-azabicyclo[3,2,1]octo-8-yl)-6-[(tetrahydro-2H-pyran-2-yloxy)methyl]-2-py ridinyl}(2,2,2-trifluoroethyl)carbamic acid tert-butyl ester obtained in Reference Example 2-33 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using the corresponding thiols instead of 4,5-dimethyl-2-mercapto-1,3-thiazole.

### Reference Example 2-46

### [6-{[(4,5-dimethyl-1,3-thiazol-2-yl)thio]methyl}-4-(3-oxa-8-azabicyclo[3,2,1]octo-8-yl) -2-pyridinyl](2,2,2-trifluoroethyl)carbamic acid tert-butyl ester

The title compound was obtained by the same methods as in Reference Example 2-18 to Reference Example 2-20, methods equivalent thereto or combinations of these with usual methods using 4,5-dimethyl-2-mercapto-1,3-thiazole.
mass: 545 (M+1)⁺.

### Reference Example 2-47

### [6-{[(4,5-dimethyl-1,3-oxazol-2-yl)thio]methyl}-4-(3-oxa-8-azabicyclo[3,2,1]octo-8-yl) -2-pyridinyl](2,2,2-trifluoroethyl)carbamic acid tert-butyl ester

The title compound was obtained by the same methods as in Reference Example 2-18 to Reference Example 2-20, methods equivalent thereto or combinations of these with usual methods using 4,5-dimethyl-1,3-oxazol-2-thiol.
mass: 529 (M+1)⁺.

### Reference Example 2-48

### [6-{[(1,5-dimethyl-1H-pyrazol-3-yl)thio]methyl}-4-(3-oxa-azabicyclo[3,2,1]octo-8-yl)-2-pyridinyl](2,2,2-trifluoroethyl)carbamic acid tert-butyl ester

The title compound was obtained by the same methods as in Reference Example 2-18 to Reference Example 2-20, methods equivalent thereto or combinations of these with usual methods using 1,5-dimethyl-1H-pyrazol-3-thiol obtained in Reference Example 1-34.
mass: 528 (M+1)⁺.

### Reference Example 2-49

### (4-(1-azepanyl)-6-{[(1,2-dimethyl-1H-imidazo1-4-yl)thio]methyl}-2-pyridinyl)(2,2,2-tri fluoroethyl)carbamic acid tert-butyl ester

The title compound was obtained by the same methods as in Reference Example 2-18 to Reference Example 2-20, methods equivalent thereto or combinations of these with usual methods using {4-(1-azepanyl)-6-[(tetrahydro-2H-pyran-2-yloxy)methyl]-2-pyridinyl}(2,2,2-trifluoroet hyl)carbamic acid tert-butyl ester obtained in Reference Example 2-35 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using 1,2-dimethyl-1H-imidazol-4-thiol hydrochloride obtained in Reference Example 1-25 instead of 4,5-dimethyl-2-mercapto-1,3-thiazole.
mass: 514 (M+1)⁺.

The compounds of Reference Example 2-50 and Reference Example 2-51 were obtained by the same methods as in Reference Example 2-18 to Reference Example 2-20, methods equivalent thereto or combinations of these with usual methods using [6-[(tetrahydro-2H-pyran-2-yloxy)methyl]-4-(4-thiomorpholinyl)-2-pyridinyl](2,2,2-trif luoroethyl)carbamic acid tert-butyl ester obtained in Reference Example 2-36 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using the corresponding thiols instead of 4,5-dimethyl-2-mercapto-1,3-thiazole.

### Reference Example 2-50

### [6-{[(5-methyl-1H-1,2,4-triazol-3-yl)thio]methyl}-4-(4-thiomorpholinyl)-2-pyridinyl](2 ,2,2-trifluoroethyl)carbamic acid tert-butyl ester

The title compound was obtained by the same methods as in Reference Example 2-18 to Reference Example 2-20, methods equivalent thereto or combinations of these with usual methods using 5-methyl-2H-1,2,4-triazol-3-thiol.
mass: 505 (M+1)⁺.

### Reference Example 2-51

### [6-{[(2-methyl-1H-imidazol-4-yl)thio]methyl}-4-(4-thiomorpholinyl)-2-pyridinyl](2,2,2 -trifluoroethyl)carbamic acid tert-butyl ester

The title compound was obtained by the same methods as in Reference Example 2-18 to Reference Example 2-20, methods equivalent thereto or combinations of these with usual methods using 2-methyl-1H-imidazol-4-thiol.
mass: 504 (M+1)⁺.

The compounds of Reference Example 2-52 and Reference Example 2-53 were obtained by the same methods as in Reference Example 2-18 to Reference Example 2-20, methods equivalent thereto or combinations of these with usual methods using {4-(1,1-dioxide-4-thiomorpholinyl)-6-[(tetrahydro-2H-pyran-2-yloxy)methyl]-2-pyridin yl}(2,2,2-trifluoroethyl)carbamic acid tert-butyl ester obtained in Reference Example 2-39 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17 and using the corresponding thiols instead of 4,5-dimethyl-2-mercaptothiazole.

### Reference Example 2-52

### [6-{[(4,5-dimethyl-1,3-oxazol-2-yl)thio]methyl}-4-(1,1-dioxide-4-thiomorpholinyl)-2-p iperidinyl](2,2,2-trifluoroethyl)carbamic acid tert-butyl ester

The title compound was obtained by the same methods as in Reference Example 2-18 to Reference Example 2-20, methods equivalent thereto or combinations of these with usual methods using 4,5-dimethyl-1,3-oxazol-2-thiol.
mass: 551 (M+1)⁺.

### Reference Example 2-53

### {4-(1,1-dioxide-4-thiomorpholinyl)-6-[(4,5,6,7-tetrahydro-1,3-benzoxazol-2-ylthio)met hyl]-2-pyridinyl}(2,2,2-trifluoroethyl)carbamic acid tert-butyl ester

The title compound was obtained by the same methods as in Reference Example 2-18 to Reference Example 2-20, methods equivalent thereto or combinations of these with usual methods using 4,5,6,7-tetrahydro-1,3-benzoxazol-2-thiol obtained in Reference Example 1-16.
mass: 577 (M+1)⁺.

### Reference Example 2-54

### {(tert-butoxycarbonyl)[4-(4,4-difluoro-1-piperidinyl)-6-(hydroxymethyl)-2-pyridinyl]a mino}acetic acid ethyl ester

In ethanol (2.00 mL) was dissolved (cyanomethyl){4-(4,4-difluoro-1-piperidinyl)-6-[(tetrahydro-2H-pyran-2-yloxy)methyl] -2-pyridinyl}carbamic acid tert-butyl ester (149 mg) obtained in Reference Example 2-37. To the solution was added p-toluenesulfonic acid monohydrate (73.0 mg), and the mixture was stirred at 40°C for 2 hours. To the reaction solution was added a saturated aqueous sodium hydrogencarbonate solution, and the mixture was extracted with ethyl acetate. The organic layer was washed with a saturated saline solution and then dried over anhydrous magnesium sulfate. Insoluble matters were filtered out, the filtrate was under reduced pressure, and the resultant residue was purified by silica gel column chromatography (hexane/ethyl acetate=98/2 to 3/7) to yield the title compound (95.0 mg) as a yellow oil.
¹ H-NMR(CDCl₃ )δ:1.29(3H,t,J=7.0Hz),1.53(9H,s),2.02-2.09(4H,m),3.53-3.54(4H,m),4. 21(2H,q,J=7.0Hz),4.56(2H,s),4.65(2H,s),6.40(1H,s),7.28(1H,s).

### Reference Example 2-55

### [(tert-butoxycarbonyl)(4-(4,4-difluoro-1-piperidinyl)-6-{[(1,2-dimethyl-1H-imidazol-4-yl)thio]methyl}-2-pyridinyl)amino]acetic acid ethyl eater

The title compound was obtained by the same methods as in Reference Example 2-19 and Reference Example 2-20, methods equivalent thereto or combinations of these with usual methods using {(tert-butoxycarbonyl)[4-(4,4-difluoro-1-piperidinyl)-6-(hydroxymethyl)-2-pyridinyl]a mino}acetic acid ethyl ester obtained in Reference Example 2-54 and using 1,2-dimethyl-1H-imidazol-4-thiol hydrochloride obtained in Reference Example 1-25 instead of 4,5-dimethyl-2-mercapto-1,3-thiazole.
mass: 540 (M+1)⁺.

### Reference Example 2-56

### 6-{[(4,5-dimethyl-1,3-oxazol-2-yl)thio]methyl}-4-(4-morpholinyl)-2-pyridinamine

Trifluoroacetic acid (20.0 mL) was added to [6-{[(4,5-dimethyl-1,3-oxazol-2-yl)thio]methyl}-4-(4-morpholinyl)-2-pyridinyl]carbam ic acid tert-butyl ester (1.00 g) obtained in Reference Example 2-26 at 0°C, and the mixture was stirred at room temperature for 2 hours. The reaction solution was under reduced pressure, a saturated aqueous sodium hydrogencarbonate solution was added to the resultant residue, and the mixture was extracted with ethyl acetate. The organic layer was washed with a saturated saline solution and dried over anhydrous sodium sulfate. Insoluble matters were filtered out, and the filtrate was under reduced pressure. The resultant residue was purified by NH silica gel column chromatography (methanol/chloroform=2/98) to give the title compound (735 mg) as a brown oil.
mass: 321 (M+1)⁺.

### Reference Example 2-57

### 4-(2-chloro-6-{[(4,5-dimethyl-1,3-oxazol-2-yl)thio]methyl}-4-pyridinyl)morpholine

In a 5N aqueous hydrochloric acid solution (4.50 mL) was suspended 6-{[(4,5-dimethyl-1,3-oxazol-2-yl)thio]methyl}-4-(4-morpholinyl)-2-pyridinamine (735 mg) obtained in Reference Example 2-56 at -5°C. To the suspension was added a solution of sodium nitrite (317 mg) in water (4.50 mL) while being maintained at -5°C, and the mixture was stirred at the same temperature for 1 hour. Potassium iodide (34.6 g) was dissolved in a 5N aqueous hydrochloric acid solution (1.50 mL), and the solution was added to the reaction solution while being maintained at -5°C. The temperature of the mixture was increased to room temperature, and the water layer was made to have a pH of 9 with an aqueous sodium hydroxide solution and a saturated aqueous sodium hydrogencarbonate solution and extracted with ethyl acetate. The organic layer was washed with water and an aqueous sodium disulfite solution and dried over anhydrous sodium sulfate. Insoluble matters were filtered out, the filtrate was under reduced pressure, and the resultant residue was purified by silica gel column chromatography (hexane/ethyl acetate=3/2) to yield the title compound (198 mg) as a pale yellow oil.
¹ H-NMR(CDCl₃ )δ:2.05(3H,s),2.19(3H,s),3.28(4H,t,J=5.0Hz),3.81(4H,t,J=5.0Hz),4.30( 2H,s),6.55(1H,d,J=2.0Hz),6.84(1H,d,J=2.0Hz).

### Reference Example 2-58

### 3-({[6-{[(4,5-dimethyl-1,3-oxazol-2-yl)thio]methyl}-4-(4-morpholinyl)-2-pyridinyl]ami no}methxl)-1-piperidine carboxylic acid tert-butyl ester

In tetrahydrofuran (3.00 mL) was dissolved 6-{[(4,5-dimethyl-1,3-oxazol-2-yl)thio]methyl}-4-(4-morpholinyl)-2-pyridinamine (31.0 mg) obtained in Reference Example 2-56. To the solution were sequentially added N-tert-butoxycarbonyl-3-formylpiperidine (25.0 mg), acetic acid (6.00 µL) and sodium triacetoxyborohydride (41.0 mg), and the mixture was stirred overnight at room temperature. A saturated aqueous sodium hydrogencarbonate solution was added to the reaction solution, the mixture was extracted with ethyl acetate, and the organic layer was washed with a saturated saline solution and dried over anhydrous sodium sulfate. Insoluble matters were filtered out, the filtrate was under reduced pressure, and the resultant residue was purified by preparative thin layer chromatography (ethyl acetate) to yield the title compound (29.0 mg) as a colorless oil.
mass: 518 (M+1)⁺.

### Reference Example 2-59

### 6-{[(4,5-dimethyl-1,3-oxazol-2-yl)thio]methyl}-4-(4-morpholinyl)-N-(3-piperidinylmet hyl)-2-pyridinamine dihydrochloride

A 10% hydrochloric acid-methanol solution (1.00 mL) was added to 3-({[6-{[(4,5-dimethyl-1,3-oxazol-2-yl)thio]methyl}-4-(4-morpholinyl)-2-pyridinyl]ami no}methyl)-1-piperidine carboxylic acid tert-butyl ester (29.0 mg) obtained in Reference Example 2-58, and the mixture was stirred overnight at room temperature. The reaction solution was under reduced pressure to give the title compound (28.0 mg) as a yellow oil.
¹ H-NMR(CD₃OD)δ:1.21-2.25(11H,m),2.77-3.00(2H,m),3.29-3.79(12H,m),4.34(2H,s), 5.98(1H,d,J=2.3Hz),6.61(1H,d,J=2.3Hz).

### Reference Example 2-60

### {4-(1,1-dioxide-4-thiomorpholinyl)-6-[(2,2,2-trifluoroethyl)amino]-2-pyridinyl}methan ol

The title compound was obtained by the same method as in Reference Example 2-18, methods equivalent thereto or combinations of these with usual methods using {4-(1,1-dioxide-4-thiomorpholinyl)-6-[(tetrahydro-2H-pyran-2-yloxy)methyl]-2-pyridin yl}(2,2,2-trifluoroethyl)carbamic acid tert-butyl ester obtained in Reference Example 2-39 instead of {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17.
mass: 340 (M+1)⁺.

### Reference Example 2-61

### 6-(chloromethyl)-4-(1,1-dioxide-4-thiomorpholinyl)-N-(2,2,2-trifluoroethyl)-2-pyridina mine

In chloroform (4.00 mL) and N,N-dimethylformamide (2.00 mL) was dissolved {4-(1,1-dioxide-4-thiomorpholinyl)-6-[(2,2,2-trifluoroethyl)amino]-2-pyridinyl}methan ol (100 mg) obtained in Reference Example 2-60. To the solution was added thionyl chloride (75.0 µL), and the mixture was heated to reflux for 30 minutes. The reaction solution was under reduced pressure, an aqueous sodium hydrogencarbonate solution was added to the resultant residue, and the mixture was extracted with ethyl acetate. The organic layer was washed with an aqueous sodium hydrogencarbonate solution and a saturated saline solution and dried over anhydrous sodium sulfate. Insoluble matters were filtered out, and the filtrate was under reduced pressure to give the title compound (106 mg) as a colorless oil.
mass: 358, 360 (M+1)⁺.

### Reference Example 2-62

### [6-(hydroxymethyl)-4-(4-thiomorpholinyl)-2-pyridinyl](2,2,2-trifluoroethyl)carbamic acid tert-butyl ester

In ethanol (30.0 mL) was dissolved [6-[(tetrahydro-2H-pyran-2-yloxy)methyl]-4-(4-thiomorpholinyl)-2-pyridinyl](2,2,2-trif luoroethyl)carbamic acid tert-butyl ester (3.60 g) obtained in Reference Example 2-36. To the solutions was added p-toluenesulfonic acid monohydrate (1.53 g), and the mixture was stirred at 50°C for 2 hours. The reaction solution was under reduced pressure, a saturated aqueous sodium hydrogencarbonate solution was added to the resultant residue, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=9/1 to 1/1) to yield the title compound (2.63 g) as a colorless solid.
mass: 408 (M+1)⁺.

### Reference Example 2-63

### [6-formyl-4-(4-thiomorpholinyl)-2-pyridinyl](2,2,2-trifluoroethyl)carbamic acid tert-butyl ester

In dimethylsulfoxide (30.0 mL) and chloroform (6.00 mL) was dissolved [6-(hydroxymethyl)-4-(4-thiomorpholinyl)-2-pyridinyl](2,2,2-trifluoroethyl)carbamic acid tert-butyl ester (2.63 g) obtained in Reference Example 2-62. To the solution were added triethylamine (9.00 mL) and sulfur trioxide-pyridine complex (2.63 g) at 0°C, and the mixture was stirred at room temperature for 1.5 hours. Water was added to the reaction solution, and the mixture was extracted with ethyl acetate. The organic layer was washed with water and a saturated saline solution and dried over anhydrous sodium sulfate. Insoluble matters were filtered out, the filtrate was under reduced pressure, and the resultant residue was purified by silica gel column chromatography (hexane/ethyl acetate=1/0 to 2/1) to yield the title compound (2.60 g) as a white solid.
¹ H-NMR(CDCl₃ )δ:1.53(9H,s),2.68-2.72(4H,m),3.83-3.87(4H,m),4.84(2H,q,J=8.2Hz),7 .14(1H,d,J=2.3Hz),7.21-7.24(1H,m),9.85(1H,s)

### Reference Example 2-64

### [6-[2-(4,5-dimethyl-1,3-oxazol-2-yl)-1-hydroxyethyl]-4-(4-thiomorpholinyl)-2-pyridiny 1](2,2,2-trifluoroethyl)carbamic acid tert-butyl ester

Diisopropylamine (0.34 mL) was dissolved in tetrahydrofuran (2.00 mL). To the solution was slowly added a 2.70M n-butyllithium-hexane solution (0.87 mL) at -78°C, and the mixture was stirred at the same temperature for 15 minutes. To the reaction solution was added 2,4,5-trimethyl-1,3-oxazole (206 mg) at -78°C, and the mixture was stirred at the same temperature for 30 minutes. To the reaction solution was added a solution of [6-formyl-4-(4-thiomorpholinyl)-2-pyridinyl](2,2,2-trifluoroethyl)carbamic acid tert-butyl ester (480 mg), obtained in Reference Example 2-63, in tetrahydrofuran (2.00 mL) at -78°C, and the mixture was stirred at -78 to 0°C for 1.5 hours. To the reaction solution was added a saturated aqueous ammonium chloride solution, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=9/1 to 1/1) to yield the title compound (228 mg) as a colorless oil.
mass: 517 (M+1)⁺.

The compounds of Reference Example 2-65 to Reference Example 2-69 were obtained by the same methods as in Reference Example 2-62 to Reference Example 2-64, methods equivalent thereto or combinations of these with usual methods using the corresponding 2-aminopyridine derivatives instead of [6-[(tetrahydro-2H-pyran-2-yloxy)methyl]-4-(4-thiomorpholinyl)-2-pyridinyl](2,2,2-trif luoroethyl)carbamic acid tert-butyl ester obtained in Reference Example 2-36 and using the corresponding oxazoles instead of 2,4,5-trimethyl-1,3-oxazole.

### Reference Example 2-65

### {4-(4,4-difluoro-1-piperidinyl)-6-[2-(4,5-dimethyl-1,3-oxazol-2-yl)-1-hydroxyethyl]-2-pyridinyl}(2,2,2-trifluoroethyl)carbamic acid tert-butyl ester

The title compound was obtained by the same methods as in Reference Example 2-62 to Reference Example 2-64, methods equivalent thereto or combinations of these with usual methods using {4-(4,4-difluoro-1-piperidinyl)-6-[(tetrahydro-2H-pyran-2-yloxy)methyl]-2-pyridinyl}( 2,2,2-trifluoroethyl)carbamic acid tert-butyl ester obtained in Reference Example 2-32 and 2,4,5-trimethyl-1,3-oxazole.
mass: 535 (M+1)⁺.

### Reference Example 2-66

### {4-(4,4-difluoro-1-piperidinyl)-6-[2-(5-ethyl-1-methyl-1,3-oxazol-2-yl)-1-hydroxyethyl ]-2-pyridinyl}(2,2,2-trifluoroethyl)carbamic acid tert-butyl ester

The title compound was obtained by the same methods as in Reference Example 2-62 to Reference Example 2-64, methods equivalent thereto or combinations of these with usual methods using {4-(4,4-difluoro-1-piperidinyl)-6-[(tetrahydro-2H-pyran-2-yloxy)methyl]-2-pyridinyl}( 2,2,2-trifluoroethyl)carbamic acid tert-butyl ester obtained in Reference Example 2-32 and 2,4-dimethyl-5-ethyl-1,3-oxazole.
mass: 549 (M+1)⁺.

### Reference Example 2-67

### [6-[2-(5-ethyl-4-methyl-1,3-oxazol-2-yl)-1-hydroxyethyl]-4-(3-oxa-azabicyclo[3,2,1]oct o-8-yl)-2-pyridinyl](2,2,2-trifluoroethyl)carbamic acid tert-butyl ester

The title compound was obtained by the same methods as in Reference Example 2-62 to Reference Example 2-64, methods equivalent thereto or combinations of these with usual methods using {4-(3-oxa-8-azabicyclo[3,2,1]octo-8-yl)-6-[(tetrahydro-2H-pyran-2-yloxy)methyl]-2-py ridinyl}(2,2,2-trifluoroethyl)carbamic acid tert-butyl ester obtained in Reference Example 2-33 and 2,4-dimethyl-5-ethyl-1,3-oxazole.
mass: 541 (M+1)⁺.

### Reference Example 2-68

### [6-[2-(4,5-dimethyl-1,3-thiazo1-2-yl)-1-hydroxyethyl]-4-(3-oxa-8-azabicyclo[3,2,1]octo-8-yl)-2-pyridinyl](2,2,2-trifluoroethyl)carbamic acid tert-butyl ester

The title compound was obtained by the same methods as in Reference Example 2-62 to Reference Example 2-64, methods equivalent thereto or combinations of these with usual methods using {4-(3-oxa-8-azabicyclo[3,2,1]octo-8-yl)-6-[(tetrahydro-2H-pyran-2-yloxy)methyl]-2-py ridinyl}(2,2,2-trifluoroethyl)carbamic acid tert-butyl ester obtained in Reference Example 2-33 and 2,4,5-trimethyl-1,3-thiazole.
mass: 543 (M+1)⁺.

### Reference Example 2-69

### [6-[2-(5-ethyl-4-methyl-1,3-oxazol-2-yl)-1-hydroxyethyl]-4-(4-morpholinyl)-2-pyridiny 1](2,2,2-trifluoroethyl)carbamic acid tert-butyl ester

The title compound was obtained by the same methods as in Reference Example 2-62 to Reference Example 2-64, methods equivalent thereto or combinations of these with usual methods using {4-(4-morpholinyl)-6-[(tetrahydro-2H-pyran-2-yloxy)methyl]-2-pyridinyl}(2,2,2-trifluo roethyl)carbamic acid tert-butyl ester obtained in Reference Example 2-34 and 2,4-dimethyl-5-ethyl-1,3-oxazole.
mass: 515 (M+1)⁺.

The compounds of Reference Example 2-70 to Reference Example 2-72 were obtained by the same methods as in Reference Example 2-62 and 2-63, methods equivalent thereto or combinations of these with usual methods using the corresponding 2-aminopyridine derivatives instead of [6-[(tetrahydro-2H-pyran-2-yloxy)methyl]-4-(4-thiomorpholinyl)-2-pyridinyl](2,2,2-trif luoroethyl)carbamic acid tert-butyl ester obtained in Reference Example 2-36.

### Reference Example 2-70

### [4-(4,4-difluoro-1-piperidinyl)-6-formyl-2-pyridinyl]carbamic acid tert-butyl ester

The title compound was obtained by the same methods as in Reference Example 2-62 and Reference Example 2-63, methods equivalent thereto or combinations of these with usual methods using {4-(4,4-difluoro-1-piperidinyl)-6-[(tetrahydro-2H-pyran-2-yloxy)methyl]-2-pyridinyl}c arbamic acid tert-butyl ester obtained in Reference Example 2-16.
mass: 342 (M+1)⁺.

### Reference Example 2-71

### [6-formyl-4-(3-oxa-8-azabicyclo[3,2,1]octo-8-yl)-2-pyridinyl]carbamic acid tert-butyl ester

The title compound was obtained by the same methods as in Reference Example 2-62 and Reference Example 2-63, methods equivalent thereto or combinations of these with usual methods using {4-(3-oxa-8-azabicyclo[3,2,1]octo-8-yl)-6-[(tetrahydro-2H-pyran-2-yloxy)methyl]-2-py ridinyl}carbamic acid tert-butyl ester obtained in Reference Example 2-17.
mass: 334 (M+1)⁺.

### Reference Example 2-72

### [6-formyl-4-(4-morpholinyl)-2-pyridinyl]carbamic acid tert-butyl ester

The title compound was obtained by the same methods as in Reference Example 2-62 and Reference Example 2-63, methods equivalent thereto or combinations of these with usual methods using {4-(4-morpholinyl)-6-[(tetrahydro-2H-pyran-2-yloxy)methyl]-2-pyridinyl}carbamic acid tert-butyl ester obtained in Reference Example 2-11.
mass: 308 (M+1)⁺.

### Reference Example 2-73

### [6-[(E)-2-(5-ethyl-4-methyl-1,3-oxazol-2-yl)vinyl]-4-(4-morpholinyl)-2-pyridinyl]carba mic acid tert-butyl ester

In tetrahydrofuran (200 mL) were dissolved [6-formyl-4-(4-morpholinyl)-2-pyridinyl]carbamic acid tert-butyl ester (7.80 g) obtained in Reference Example 2-72 and [(5-ethyl-4-methyl-1,3-oxazol-2-yl)methyl]phosphonic acid diethyl ester (7.96 g) obtained in Reference Example 1-11. To the solution was added 60% sodium hydride (2.03 g) at 0°C, and the mixture was stirred at room temperature for 3 hours. Water was added to the reaction solution, the mixture was extracted with ethyl acetate, and the organic layer was then washed with water and a saturated saline solution and dried over anhydrous sodium sulfate. Insoluble matters were filtered out, the filtrate was under reduced pressure, and the resultant residue was purified by silica gel column chromatography (hexane/ethyl acetate=4/1 to 7/3) to yield the title compound (7.45 g) as an orange oil.
mass: 415 (M+1)⁺.

### Reference Example 2-74

### [6-[2-(5-ethyl-4-methyl-1,3-oxazol-2-yl)ethyl]-4-(4-morpholinyl)-2-pyridinyl]carbamic acid tert-butyl ester

In methanol (50.0 mL) was dissolved [6-[(E)-2-(5-ethyl-4-methyl-1,3-oxazol-2-yl)vinyl]-4-(4-morpholinyl)-2-pyridinyl]carba mic acid tert-butyl ester (1.60 g) obtained in Reference Example 2-73. To the solution was added 10% palladium carbon (500 mg), and the mixture was stirred under hydrogen atmosphere at room temperature for 60 hours. The reaction solution was Celite-filtered, the filtrate was under reduced pressure, and the resultant residue was purified by silica gel column chromatography (hexane/ethyl acetate=3/2) to yield the title compound (1.19 g) as a colorless solid.
mass: 417 (M+1)⁺.

### Reference Example 2-75

### [6-[2-(5-ethyl-4-methyl-1,3-oxazol-2-yl)ethyl]-4-(4-morpholinyl)-2-pyridinyl][(methylt hio)methyl]carbamic acid tert-butyl ester

The title compound was obtained by the same method as in Reference Example 2-32, methods equivalent thereto or combinations of these with usual methods using [6-[2-(5-ethyl-4-methyl-1,3-oxazol-2-yl)ethyl]-4-(4-morpholinyl)-2-pyridinyl]carbamic acid tert-butyl ester obtained in Reference Example 2-74 and
methyl(chloromethyl)sulfide. ¹ H-NMR(CDCl₃ )δ:1.17(3H,t,J=7.5Hz),1.52(9H,s),2.05(3H,s),2.14(3H,s),2.56(2H,q,J= 7.5Hz),3.04-3.11(4H,m),3.24-3.29(4H,m),3.79-3.84(4H,m),5.16(2H,s),6.38(1H,d,J=2.0 Hz),6.97(1H,d,J=2.0Hz).

The compounds of Reference Example 2-76 to Reference Example 2-78 were obtained by the same methods as in Reference Example 2-73 and 2-74, methods equivalent thereto or combinations of these with usual methods using the corresponding aldehydes instead of [6-formyl-4-(4-morpholinyl)-2-pyridinyl]carbamic acid tert-butyl ester obtained in Reference Example 2-72 and using the corresponding alkyl phosphnates instead of [(5-ethyl-4-methyl-1,3-oxazol-2-yl)methyl]phosphonic acid diethyl ester obtained in Reference Example 1-11.

### Reference Example 2-76

### {4-(4,4-difluoro-1-piperidinyl)-6-[2-(1,5-dimethyl-1H-pyrazol-3-yl)ethyl]-2-pyridinyl}c arbamic acid tert-butyl ester

The title compound was obtained by the same methods as in Reference Example 2-73 and Reference Example 2-74, methods equivalent thereto or combinations of these with usual methods using [4-(4,4-difluoro-1-piperidinyl)-6-formyl-2-pyridinyl]carbamic acid tert-butyl ester obtained in Reference Example 2-70 and [(1,5-dimethyl-1H-pyrazol-3-yl)methyl]phosphonic acid dimethylester obtained in Reference Example 1-15.
mass: 436 (M+1)⁺.

### Reference Example 2-77

### {4-(4,4-difluoro-1-piperidinyl)-6-[2-(4,5-dimethyl-1,3-oxazol-2-yl)ethyl]-2-pyridinyl}c arbamic acid tert-butyl ester

The title compound was obtained by the same methods as in Reference Example 2-73 and Reference Example 2-74, methods equivalent thereto or combinations of these with usual methods using [4-(4,4-difluoro-1-piperidinyl)-6-formyl-2-pyridinyl]carbamic acid tert-butyl ester obtained in Reference Example 2-70 and [(4,5-dimethyl-1,3-oxazol-2-yl)methyl]phosphonic acid diethyl ester obtained in Reference Example 1-13.
mass: 437 (M+1)⁺.

### Reference Example 2-78

### [6-[2-(5-ethyl-4-methyl-1,3-thiazol-2-yl)ethyl]-4-(3-oxa-8-azabicyclo[3,2,1]octo-8-yl)-2 -pyridinyl]carbamic acid tert-butyl ester

The title compound was obtained by the same methods as in Reference Example 2-73 and Reference Example 2-74, methods equivalent thereto or combinations of these with usual methods using [6-formyl-4-(3-oxa-8-azabicyclo[3,2,1]octo-8-yl)-2-pyridinyl]carbamic acid tert-butyl ester obtained in Reference Example 2-71 and [(5-ethyl-4-methyl-1,3-thiazol-2-yl)methyl]phosphonic acid diethyl ester obtained in Reference Example 1-12.
mass: 459 (M+1)⁺.

### Reference Example 2-79

### {4-(benzyloxy)-6-[(tetrahydro-2H-pyran-2-yloxy)methyl]-2-pyridinyl}(2-methoxyethyl )carbamic acid tert-butyl ester

The title compound was obtained by the same method as in Reference Example 2-32, methods equivalent thereto or combinations of these with usual methods using {4-(benzyloxy)-6-[(tetrahydro-2H-pyran-2-yloxy)methyl]-2-pyridinyl}carbamic acid tert-butyl ester obtained in Reference Example 2-8 and using p-toluenesulfonic acid 2-methoxyethyl ester instead of 2,2,2-trifluoroethyl trifluoromethanesulfonate.
mass: 473 (M+1)⁺.

### Reference Example 2-80

### [4-(benzyloxy)-6-formyl-2-pylidinyl](2-methoxyethyl)carbamic acid tert-butyl ester

The title compound was obtained by the same methods as in Reference Example 2-62 and Reference Example 2-63, methods equivalent thereto or combinations of these with usual methods using {4-(benzyloxy)-6-[(tetrahydro-2H-pyran-2-yloxy)methyl]-2-pyridinyl}(2-methoxyethyl )carbamic acid tert-butyl ester obtained in Reference Example 2-79 instead of [6-[(tetrahydro-2H-pyran-2-yloxy)methyl]-4-(4-thiomorpholinyl)-2-pyridinyl](2,2,2-trif luoroethyl)carbamic acid tert-butyl ester Reference Example 2-36.
mass: 387 (M+1)⁺.

### Reference Example 2-81

### {4-(benzyloxy)-6-[2-(5-ethyl-4-methyl-1,3-oxazol-2-yl)ethyl]-2-pyridinyl}(2-methoxye thyl)carbamic acid tert-butyl ester

The title compound was obtained by the same methods as in Reference Example 2-73 and Reference Example 2-74, methods equivalent thereto or combinations of these with usual methods using [4-(benzyloxy)-6-formyl-2-pyridinyl](2-methoxyethyl)carbamic acid tert-butyl ester obtained in Reference Example 2-80 instead of [6-formyl-4-(4-morpholinyl)-2-pyridinyl]carbamic acid tert-butyl ester obtained in Reference Example 2-72.
mass: 496 (M+1)⁺.

### Reference Example 2-82

### 2-[(tert-butoxycarbonyl)(2-methoxyethyl)amino]-6-[2-(5-ethyl-4-meLhyl-1,3-oxazol-2-y l)ethyl]-4-pyridinyl trifluoromethanesulfonate

The title compound was obtained by the same methods as in Reference Example 2-5 and Reference Example 2-6, methods equivalent thereto or combinations of these with usual methods using {4-(benzyloxy)-6-[2-(5-ethyl-4-methyl-1,3-oxazol-2-yl)ethyl]-2-pyridinyl}(2-methoxye thyl)carbamic acid tert-butyl ester obtained in Reference Example 2-81 instead of 4-(benzyloxy)-6-[(tetrahydro-2H-pyran-2-yloxy)methyl]-2-pyridinecarboxylic acid ethyl ester obtained in Reference Example 2-4.
mass: 538 (M+1)⁺.

### Reference Example 2-83

### [6-[2-(5-ethyl-4-methyl-1,3-oxazol-2-yl)ethyl]-4-(3-methyl-4-mopholinyl)-2-pyridinyl] (2-methoxyethyl)carbamic acid tert-butyl ester

In ethylene glycol dimethyl ether (2.00 mL) was dissolved 2-[(tert-butoxycarbonyl)(2-methoxyethyl)amino]-6-[2-(5-ethyl-4-methyl-1,3-oxazol-2-y l)ethyl]-4-pyridinyl trifluoromethanesulfonate (9.10 mg) obtained in Reference Example 2-82. To the solution were added 3-methylmorpholine (8.60 mg), tris(dibenzylideneacetone)dipalladium(0) (2.20 mg), 2-(di-tert-butylphosphino)biphenyl (2.50 mg) and tripotassium phosphate (5.40 mg) under nitrogen atmosphere, and the mixture was stirred overnight at 80°C. The temperature of the reaction solution was returned to room temperature, a saturated aqueous sodium hydrogencarbonate solution was added, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by preparative thin layer chromatography (methanol/chloroform= 1/15) to give the title compound.
mass: 489 (M+1)⁺.

The compounds of Reference Example 2-84 to Reference Example 2-86 were obtained by the same method as in Reference Example 2-83, methods equivalent thereto or combinations of these with usual methods by replacing 3-methylmorpholine by the corresponding amines.

### Reference Example 2-84

### [6-[2-(5-ethyl-4-methyl-1,3-oxazol-2-yl)ethyl]-4-(2-methyl-4-morpholinyl)-2-pyridinyl] (2-methoxyethyl)carbamic acid tert-butyl ester

The title compound was obtained by the same method as in Reference Example 2-83, methods equivalent thereto or combinations of these with usual methods using 2-methylmorpholine.
mass: 489 (M+1)⁺.

### Reference Example 2-85

### {4-(8-azabicyclo[3,2,1]octo-8-yl)-6-[2-(5-ethyl-4-methyl-1,3-oxazol-2-yl)ethyl]-2-pyrid inyl}(2-methoxyethyl)carbamic acid tert-butyl ester

The title compound was obtained by the same method as in Reference Example 2-83, methods equivalent thereto or combinations of these with usual methods using 8-azabicyclo[3,2,1]octone hydrochloride.
mass: 499 (M+1)⁺.

### Reference Example 2-86

### {4-(4,4-difluoro-3-hydroxy-1-piperidinyl)-6-[2-(5-ethyl-4-methyl-1,3-oxazol-2-yl)ethyl ]-2-pyridinyl}(2-methoxyethyl)carbamic acid tert-butyl ester

The title compound was obtained by the same method as in Reference Example 2-83, methods equivalent thereto or combinations of these with usual methods using 4,4-difluoro-3-piperidinol hydrochloride obtained in Reference Example 1-7.
mass: 525 (M+1)⁺.

### Reference Example 2-87

### {4-(4,4-difluoro-3-methoxy-1-piperidinyl)-6-[2-(5-ethyl-4-methyl-1,3-oxazol-2-yl)ethyl ]-2-pyridinyl}(2-methoxyethyl)carbamic acid tert-butyl ester

In tetrahydrofuran (2.00 mL) was dissolved {4-(4,4-difluoro-3-hydroxy-1-piperidinyl)-6-[2-(5-ethyl-4-methyl-1,3-oxazol-2-yl)ethyl ]-2-pyridinyl}(2-methoxyethyl)carbamic acid tert-butyl ester (53.0 mg) obtained in Reference Example 2-86. To the solution was added 60% sodium hydride (8.10 mg) at 0°C, and the mixture was stirred at the same temperature for 15 minutes. Methyl iodide (19.0 µL) was added to the reaction solution, and the mixture was stirred overnight at room temperature. Water was added to the reaction solution, the mixture was extracted with ethyl acetate, and the organic layer was then washed with water and a saturated saline solution and dried over anhydrous sodium sulfate. Insoluble matters were filtered out, and the filtrate was under reduced pressure to give the title compound (54.0 mg) as a yellow oil.
mass: 539 (M+1)⁺.

### Example 1

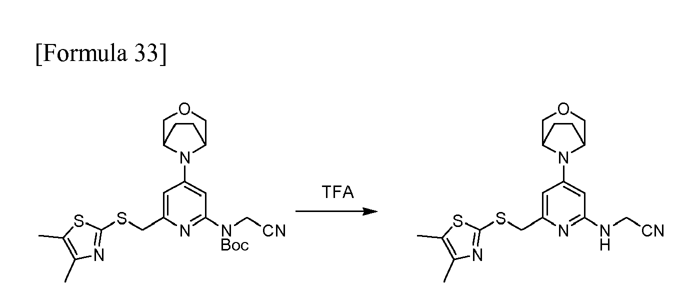

### {[6-{[(4,5-dimethyl-1,3-thiazol-2-yl)thio]methyl}-4-(3-oxa-8-azabicyclo[3,2,1]octo-8-y l)-2 pyridinyl]amino}acetonitrile

Trifluoroacetic acid (0.50 mL) was added to (cyanomethyl)[6-{[(4,5-dimethyl-1,3-thiazol-2-yl)thio]methyl}-4-(3-oxa-8-azabicyclo[3 ,2,1]octo-8-yl)-2-pyridinyl]carbamic acid tert-butyl ester (101 mg) obtained in Reference Example 2-38, and the mixture was stirred at room temperature for 30 minutes. The reaction solution was under reduced pressure, a saturated aqueous sodium hydrogencarbonate solution was added to the residue, and the mixture was extracted with chloroform. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=9/1 t0 0/1) to yield the title compound (51.0 mg) as a colorless amorphous substance. ¹ H-NMR(CDCl₃ )δ:1.97-2.13(4H,m),2.28(6H,s),3.49(2H,d,J=10.9Hz),3.76(2H,d,J=10.9 Hz),4.00-4.04(2H,m),4.23(2H,s),4.30(2H,d,J=6.3Hz),4.47-4.54(1H,m),5.61(1H,d,J=2.0 Hz),6.25(1H,d,J=2.0Hz).
mass:402(M+1)⁺.

The compounds of Example 2 to Example 18 were obtained by the same method as in Example 1, methods equivalent thereto or combinations of these with usual methods using the corresponding Boc protected compounds instead of (cyanomethyl)[6-{[(4,5-dimethyl-1,3-thiazol-2-yl)thio]methyl}-4-(3-oxa-8-azabicyclo[3 ,2,1]octo-8-yl)-2-pyridinyl]carbamic acid tert-butyl ester obtained in Reference Example 2-38.

### Example 2

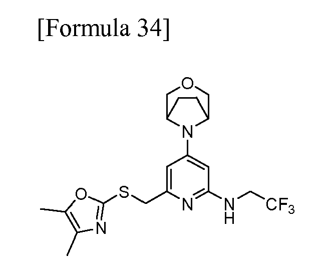

### 6-{[(4,5-dimethyl-1,3-oxazol-2-yl)thio]methyl}-4-(3-oxa-8-azabicyclo[3,2,1]octo-8-yl)-N-(2,2,2-trifluoroethyl)-2-pyridinamine

The title compound was obtained by the same method as in Example 1, methods equivalent thereto or combinations of these with usual methods using [6-{[(4,5-dimethyl-1,3-oxazol-2-yl)thio]methyl}-4-(3-oxa-8-azabicyclo[3,2,1]octo-8-yl) -2-pyridinyl](2,2,2-trifluoroethyl)carbamic acid tert-butyl ester obtained in Reference Example 2-47.
¹ H-NMR(CDCl₃ )δ:1.99-2.03(2H,m),2.05(3H,d,J=1.0Hz),2.07-2.12(2H,m),2.20(3H,d,J =1.0Hz),3.49(2H,d,J=11.2Hz),3.77(2H,d,J=11.2Hz),3.97-4.05(4H,m),4.22(2H,s),5.58(1 H,d,J=2.0Hz),6.22(1H,d,J=2.0Hz).
mass:429(M+1)⁺.

### Example 3

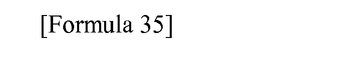

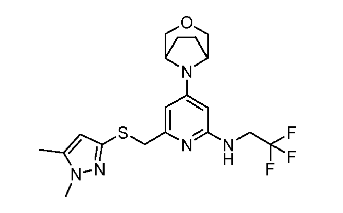

### 6-{[(1,5-dimethyl-1H-pyrazol-3-yl)thio]methyl}-4-(3-oxa-8-azabicyclo[3,2,1]octo-8-yl) -N-(2,2,2-trifluoroethyl)-2-pyridinamine

The title compound was obtained by the same method as in Example 1, methods equivalent thereto or combinations of these with usual methods using [6-{[(1,5-dimethyl-1H-pyrazol-3-yl)thio]methyl}-4-(3-oxa-azabicyclo[3,2,1]octo-8-yl)-2-pyridinyl](2,2,2-trifluoroethyl)carbamic acid tert-butyl ester obtained in Reference Example 2-48. ¹ H-NMR(CDCl₃ )δ:1.99-2.09(4H,m),2.21(3H,s),3.48(2H,d,J=10.9Hz),3.73(3H,s),3.77( 2H,d,J=10.9Hz),3.98(2H,s),4.00-4.06(4H,m),4.50(1H,brs),5.58(1H,s),5.99(1H,s),6.21(1 H,s).
mass:428(M+1)⁺.

### Example 4

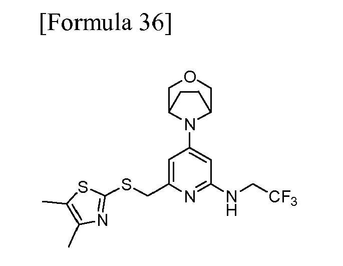

### 6-{[(4,5-dimethyl-1,3-thiazol-2-yl)thio]methyl}-4-(3-oxa-8-azabicyclo[3,2,]octo-8-xl)-N-(2,2,2-trifluoroethyl)-2-pyridinamine

The title compound was obtained by the same method as in Example 1, methods equivalent thereto or combinations of these with usual methods using [6-{[(4,5-dimethyl-1,3-thiazol-2-yl)thio]methyl}-4-(3-oxa-8-azabicyclo[3,2,1]octo-8-yl) -2-pyridinyl](2,2,2-trifluoroethyl)carbamic acid tert-butyl ester obtained in Reference Example 2-46.
¹ H-NMR(CDCl₃ )δ:1.98-2.04(2H,m),2.06-2.12(2H,m),2.28(6H,s),3.49(2H,d,J=11.2Hz), 3.76(2H,d,J=11.2Hz),4.06-3.98(4H,m),4.20(2H,s),4.62(1H,brs),5.58(1H,d,J=2.0Hz),6.2 0(1H,d,J=2.0Hz).
mass:445(M+1)⁺.

### Example 5

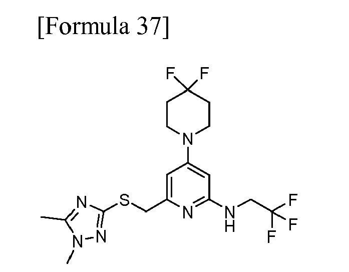

### 4-(4,4-difluoro-1-piperidinyl)-6-{[(1,5-dimethyl-1H-1,2,4-triazol-3-yl)thio]methyl}-N-( 2,2,2-trifluoroethyl)-2-pyridinamine

The title compound was obtained by the same method as in Example 1, methods equivalent thereto or combinations of these with usual methods using (4-(4,4-difluoro-1-piperidinyl)-6-{[(1,5-dimethyl-1H-1,2,4-triazol-3-yl)thio]methyl}-2-pyridinyl)(2,2,2-trifluoroethyl)carbamic acid tert-butyl ester obtained in Reference Example 2-41.
¹ H-NMR(CDCl₃ )δ:1.95-2.05(4H,m),2.42(3H,s),3.44-3.46(4H,m),3.76(3H,s),4.01-4.10( 2H,m),4.23(2H,s),4.47(1H,brs),5.71(1H,d,J=2.0Hz),6.42(1H,d,J=2.0Hz). mass:437(M+1)⁺.

### Example 6

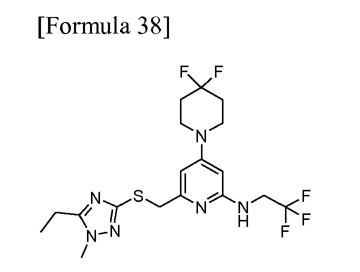

### 4-(4,4-difluoro-1-piperidinyl)-6-{[(5-ethyl-1-methyl-1H-1,2,4-triazol-3-yl)thio]methyl} -N-(2,2,2-trifluoroethyl)-2-pyridinamine

The title compound was obtained by the same method as in Example 1, methods equivalent thereto or combinations of these with usual methods using (4-(4,4-difluoro-1-piperidinyl)-6-{[(5-ethyl-1-methyl-1H-1,2,4-triazol-3-yl)thio]methyl }-2-pyridinyl)(2,2,2-trifluoroethyl)carbamic acid tert-butyl ester obtained in Reference Example 2-42. ¹ H-NMR(CDCl₃ )δ:1.33(3H,t,J=7.6Hz),1.97-1.99(4H,m),2.72(2H,q,J=7.6Hz),3.42-3.45 (4H,m),3.75(3H,s),4.03-4.05(2H,m),4.23(2H,s),4.47(1H,brs),5.70(1H,d,J=2.0Hz),6.43(1 H,d,J=2.0Hz).
mass:451(M+1)⁺.

### Example 7

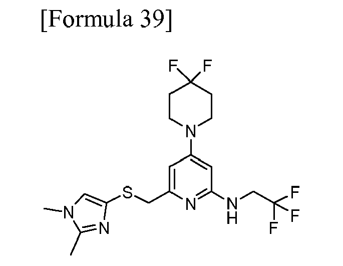

### 4-(4,4-difluoro-1-piperidinyl)-6-{[(1,2-dimethyl-1H-imidazo1-4-yl)thiol]methyl}-N-(2,2 ,2-trifluoroethyl)-2-pyridinamine

The title compound was obtained by the same method as in Example 1, methods equivalent thereto or combinations of these with usual methods using (4-(4,4-difluoro-1-piperidinyl)-6-{[(1,2-dimethyl-1H-imidazo1-4-yl)thio]methyl}-2-pyri dinyl)(2,2,2-trifluoroethyl)carbamic acid tert-butyl ester obtained in Reference Example 2-44.
¹ H-NMR(CDCl₃ )δ:1.93-2.00(4H,m),2.35(3H,s),3.41-3.44(4H,m),3.48(3H,s),3.91(2H,s ),3.99-4.04(2H,m),4.56(1H,brs),5.70(1H,d,J=2.0Hz),6.26(1H,d,J=2.0Hz),6.74(1H,s). mass:436(M+1)⁺.

### Example 8

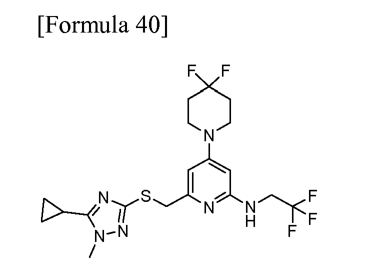

### 6-{[(5-cyclopropyl-1-methyl-1H-1,2,4-triazol-3-yl)thio]methyl}-4-(4,4-difluoro-1-piper idinyl)-N-(2,2,2-trifluoroethyl)-2-pyridinamine

The title compound was obtained by the same method as in Example 1, methods equivalent thereto or combinations of these with usual methods using [6-{(5-cyclopropyl-1-methyl-1H-1,2,4-triazol-3-yl)thio]methyl}-4-(4,4-difluoro-1-piper idinyl)-2-pyridinyl](2,2,2-trifluoroethyl)carbamic acid tert-butyl ester obtained in Reference Example 2-43.
¹ H-NMR(CDCl₃ )δ:1.03-1.10(4H,m),1.76-1.82(1H,m),1.94-2.05(4H,m),3.42-3.45(4H, m),3.83(3H,s),4.02-4.08(2H,m),4.19(2H,s),4.57(1H,brs),5.70(1H,s),6.40(1H,s). mass:463(M+1)⁺.

### Example 9

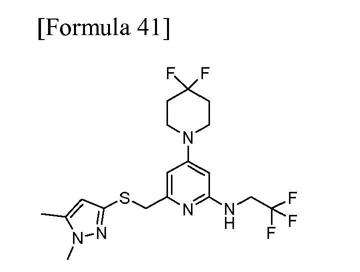

### 4-(4,4-difluoro-1-piperidinyl)-6-{[(1,5-dimethyl-1H-pyrazol-3-yl)thio]methyl}-N-(2,2, -trifluoroethyl)-2-pyridinamine

The title compound was obtained by the same method as in Example 1, methods equivalent thereto or combinations of these with usual methods using (4-(4,4-difluoro-1-piperidinyl)-6-{[(1,5-dimethyl-1H-pyrazol-3-yl)thio]methyl}-2-pyrid inyl)(2,2,2-trifluoroethyl)carbamic acid tert-butyl ester obtained in Reference Example 2-45.
¹ H-NMR(CDCl₃ )δ:1.95-2.02(4H,m),2.21(3H,s),3.42-3.45(4H,m),3.73(3H,s),3.99(2H,s ),4.03-4.09(2H,m),4.49(1H,brs),5.70(1H,d,J=2.0Hz),5.99(1H,s),6.36(1H,d,J=2.0Hz). mass:436(M+1)⁺.

### Example 10

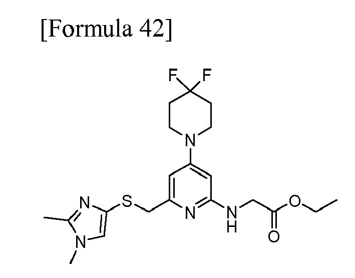

### [(4-(4,4-difluoro-1-piperidinyl)-6-{[(1,2-dimethyl-1H-imidazo1-4-yl)thio]methyl}-2-pyr idinyl)amino]acetic acid ethyl ester

The title compound was obtained by the same method as in Example 1, methods equivalent thereto or combinations of these with usual methods using [(tert-butoxycarbonyl)(4-(4,4-difluoro-1-piperidinyl)-6-{[(1,2-dimethyl-1H-imidazol-4-yl)thio]methyl}-2-pyridinyl)amino]acetic acid ethyl ester obtained in Reference Example 2-55. ¹ H-NMR(CDCl₃ )δ:1.25-1.31(3H,m),1.91-2.01(4H,m),2.35(3H,s),3.40-3.43(4H,m),3.47 (3H,s),3.90(2H,s),4.10(2H,s),4.22(2H,q,J=7.2Hz),4.97(1H,brs),5.65(1H,d,J=2.0Hz),6.2 1(1H,d,J=2.0Hz),6.74(1H,s).
mass:440(M+1)⁺.

### Example 11

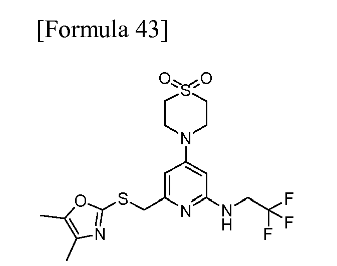

### 6-{[(4,5-dimethyl-1,3-oxazol-2-yl)thio]methyl}-4-(1,1-dioxide-4-thiomorpholinyl)-N-(2 ,2,2-trifluoroethyl)-2-pyridinamine

The title compound was obtained by the same method as in Example 1, methods equivalent thereto or combinations of these with usual methods using [6- {[(4,5-dimethyl-1,3-oxazol-2-yl)thio]methyl}-4-(1,1-dioxide-4-thiomorpholinyl)-2-p iperidinyl](2,2,2-trifluoroethyl)carbamic acid tert-butyl ester obtained in Reference Example 2-52.
1H-NMR(CDCl₃ )δ:2.04-2.06(3H,m),2.14-2.17(3H,m),2.98-3.03(4H,m),3.85-3.93(4H, m),4.00-4.10(2H,m),4.23(2H,s),4.66(1H,brs),5.72(1H,d,J=2.0Hz),6.37(1H,d,J=2.0Hz). mass:451(M+1)⁺.

### Example 12

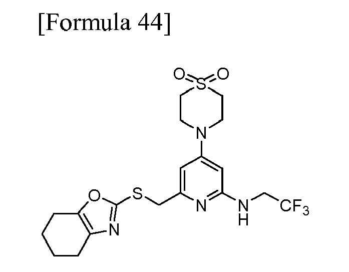

### 4-(1,1-dioxide-4-thiomorpholinyl)-6-[(4,5,6,7-tetrahydro-1,3-benzoxazol-2-ylthio)met yl]-N-(2,2,2-trifluoroethyl)-2-pyridinamine

The title compound was obtained by the same method as in Example 1, methods equivalent thereto or combinations of these with usual methods using {4-(1,1-dioxide-4-thiomorpholinyl)-6-[(4,5,6,7-tetrahydro-1,3-benzoxazol-2-ylthio)met hyl]-2-pyridinyl}(2,2,2-trifluoroethyl)carbamic acid tert-butyl ester obtained in Reference Example 2-53.
¹ H-NMR(CDCl₃ )δ:1.76-1.85(4H,m),2.47-2.50(2H,m),2.56-2.59(2H,m),2.99(4H,t,J=5.0 Hz),3.89(4H,t,J=5.0Hz),4.02-4.11(2H,m),4.25(2H,s),4.78(1H,t,J=6.5Hz),5.74(1H,d,J=2. 0Hz),6.37(1H,d,J=2.0Hz).
mass:477(M+1)⁺.

### Example 13

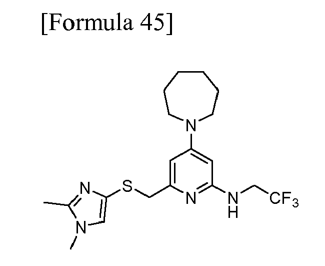

### 4-(1-azepanyl)-6-{[(1,2-dimethyl-1H-imidazol-4-yl)thio]methyl}-N-(2,2,2-trifluoroethy l)-2-pyridinamine

The title compound was obtained by the same method as in Example 1, methods equivalent thereto or combinations of these with usual methods using (4-(1-azepanyl)-6-{[(1,2-dimethyl-1H-imidazol-4-yl)thio]methyl}-2-pyridinyl)(2,2,2-tri fluoroethyl)carbamic acid tert-butyl ester obtained in Reference Example 2-49. ¹ H-NMR(CDCl₃ )δ:1.50(4H,brs),1.69(4H,brs),2.34(3H,s),3.36(4H,t,J=6.0Hz),3.46(3H,s ),3.88(2H,s),3.95-4.04(2H,m),4.54(1H,brs),5.50(1H,d,J=2.0Hz),6.00(1H,d,J=2.0Hz),6.7 3(1H,s).
mass:414(M+1)⁺.

### Example 14

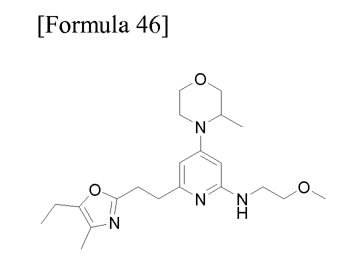

### 6-[2-(5-ethyl-4-methyl-1,3-oxazol-2-yl)ethyl]-N-(2-methoxyethyl)-4-(3-methyl-4-morp holinyl)-2-pyridinamine

The title compound was obtained by the same method as in Example 1, methods equivalent thereto or combinations of these with usual methods using [6-[2-(5-ethyl-4-methyl-1,3-oxazol-2-yl)ethyl]-4-(3-methyl-4-morpholinyl)-2-pyridinyl] (2-methoxyethyl)carbamic acid tert-butyl ester obtained in Reference Example 2-83.
¹ H-NMR(CDCl₃ )δ:1.16(3H,t,J=7.6Hz),1.25(3H,d,J=13.4Hz),2.04(3H,s),2.55(2H,q,J=7 .6Hz),2.98-3.31(6H,m),3.38(3H,s),3.42(2H,q,J=5.0Hz),3.57-3.63(3H,m),3.72-3.85(3H, m),3.98-4.01(1H,m),5.59(1H,brs),5.99(1H,d,J=2.0Hz).
mass:389(M+1)⁺.

### Example 15

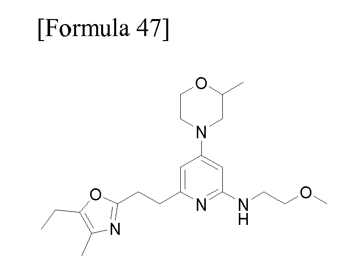

### 6-[2-(5-ethyl-4-methyl-1,3-oxazol-2-yl)ethyl]-N-(2-methoxyethyl)-4-(2-methyl-4-morp holinyl)-2-pyridinamine

The title compound was obtained by the same method as in Example 1, methods equivalent thereto or combinations of these with usual methods using [6-[2-(5-ethyl-4-methyl-1,3-oxazol-2-yl)ethyl]-4-(2-methyl-4-morpholinyl)-2-pyridinyl] (2-methoxyethyl)carbamic acid tert-butyl ester obtained in Reference Example 2-84.
¹ H-NMR(CDCl₃ )δ:1.16(3H,t,J=7.6Hz),1.26(3H,d,J=6.1Hz),2.04(3H,s),2.55(2H,q,J=7. 6Hz),2.60-2.67(1H,m),2.96-3.11(5H,m),3.388(3H,s),3.40-3.43(2H,m),3.52-3.70(6H,m),4 .00(1H,dd,J=2.6,11.6Hz),5.65(1H,brs),6.02(1H,d,J=2.0Hz).
mass:389(M+1)⁺.

### Example 16

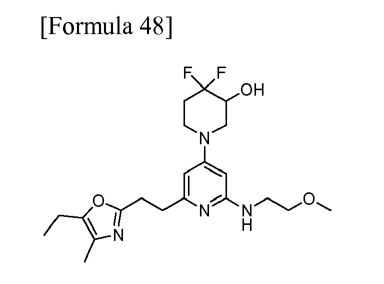

### 1-{2-[2-(5-ethyl-4-methyl-1,3-oxazol-2-yl)ethyl]-6-[(2-methoxyethyl)amino]-4-pyridin yl-4,4-difluoro-piperidinol

The title compound was obtained by the same method as in Example 1, methods equivalent thereto or combinations of these with usual methods using {4-(4,4-difluoro-3-hydroxy-1-piperidinyl)-6-[2-(5-ethyl-4-methyl-1,3-oxazol-2-yl)ethyl ]-2-pyridinyl}(2-methoxyethyl)carbamic acid tert-butyl ester obtained in Reference Example 2-86.
¹ H-NMR(DMSO-d₆ )δ:1.09(3H,t,J=7.4Hz),1.75-2.22(5H,m),2.53(2H,q,J=7.4Hz),2.73-2 .80(2H,m),2.88-2.98(2H,m),2.99-3.74(12H,m),5.75(1H,d,J=2.0Hz),5.97-6.02(1H,m),6.0 8(1H,d,J=2.0Hz),8.22(1H,s).
mass:425(M+1)⁺.

### Example 17

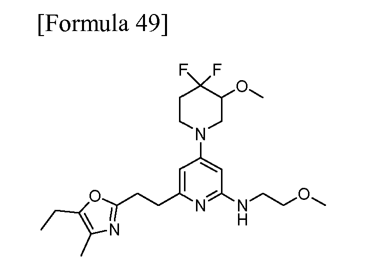

### 4-(4,4-difluoro-3-methoxy-1-piperidinyl)-6-[2-(5-ethyl-4-methyl-1,3-oxazol-2-yl)ethyl] -N-(2-methoxyethyl)-2-pyridinamine

The title compound was obtained by the same method as in Example 1, methods equivalent thereto or combinations of these with usual methods using {4-(4,4-difluoro-3-methoxy-1-piperidinyl)-6-[2-(5-ethyl-4-methyl-1,3-oxazol-2-yl)ethyl ]-2-pyridinyl}(2-methoxyethyl)carbamic acid tert-butyl ester obtained in Reference Example 2-87.
¹ H-NMR(DMSO-d₆ )δ:1.09(3H,t,J=7.4Hz),1.83-2.18(5H,m),2.55(2H,q,J=7.4Hz),2.83-3 .01(4H,m),3.10-3.71(15H,m),5.90(1H,s),6.36(1H,s),8.13(1H,s). mass:439(M+1)⁺.

### Example 18

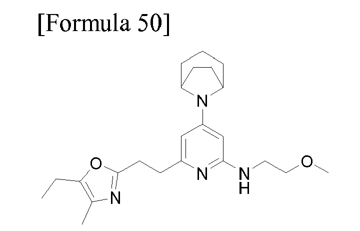

### 4-(8-azabicyclo[3,2,1]octo-8-yl)-6-[2-(5-ethyl-4-methyl-1,3-oxazol-2-yl)ethyl]-N-(2-me thoxyethyl)-2-pyridinamine

The title compound was obtained by the same method as in Example 1, methods equivalent thereto or combinations of these with usual methods using {4-(8-azabicyclo[3,2,1]octo-8-yl)-6-[2-(5-ethyl-4-methyl-1,3-oxazol-2-yl)ethyl]-2-pyrid inyl}(2-methoxyethyl)carbamic acid tert-butyl ester obtained in Reference Example 2-85. ¹ H-NMR(CDCl₃ )δ:1.15-1.19(4H,m),1.79-1.84(5H,m),2.05(5H,m),2.52-2.56(4H,m),2.9 3-2.95(2H,m),3.04-3.07(2H,m),3.38(3H,s),3.41-3.42(2H,m),3.57-3.59(2H,m),4.12(2H,s ),5.51(1H,s),5.93(1H,s),7.01(1H,brs).
mass:399(M+1)⁺.

### Example 19

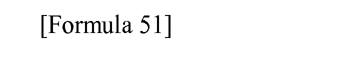

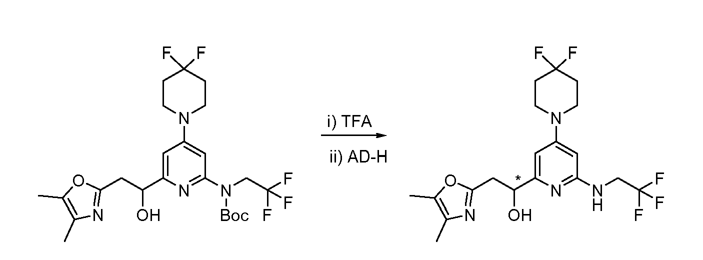

### 1-{4-(4,4-difluoro-1-piperidinyl)-6-[(2,2,2-trifluoroethyl)amino]-2-pyridinyl}-2-(4,5-di methyl-1,3-oxazol-2-yl)ethanol

Trifluoroacetic acid (2.00 mL) was added to {4-(4,4-difluoro-1-piperidinyl)-6-[2-(4,5-dimethyl-1,3-oxazol-2-yl)-1-hydroxyethyl]-2-pyridinyl}(2,2,2-trifluoroethyl)carbamic acid tert-butyl ester (142 mg) obtained in Reference Example 2-65, and the mixture was stirred at room temperature for 1.5 hours. The reaction solution was under reduced pressure, a saturated aqueous sodium hydrogencarbonate solution was added to the resultant residue, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=88/12 to 0/1) to give a racemic mixture (100 mg) of the title compound. The optical resolution of the racemic mixture was carried out with an optically active column (CHIRALPAK AD-H column manufactured by Daicel Chemical Industries, Ltd.; 0.1 % diethylamine-hexane/isopropanol=3/1) to give the title compound (50.5 mg) as a white solid from an earlier fraction. ¹ H-NMR(CDCl₃ )δ:1.95-2.05(7H,m),2.20(3H,s),3.00(1H,dd,J=8.5,15.4Hz),3.13(1H,dd, J=4.1,15.4Hz),3.43-3.49(4H,m),3.98-4.14(2H,m),4.45-4.56(2H,m),4.92(1H,s),5.73(1H, d,J=2.0Hz),6.30(1H,d,J=2.0Hz).
mass:435(M+1)⁺.

The compounds of Example 20 to Example 24 were obtained by the same method as in Example 19, methods equivalent thereto or combinations of these with usual methods using the corresponding Boc protected compounds instead of {4-(4,4-difluoro-1-piperidinyl)-6-[2-(4,5-dimethyl-1,3-oxazol-2-yl)-1-hydroxyethyl]-2-pyridinyl}(2,2,2-trifluoroethyl)carbamic acid tert-butyl ester obtained in Reference Example 2-65.

### Example 20

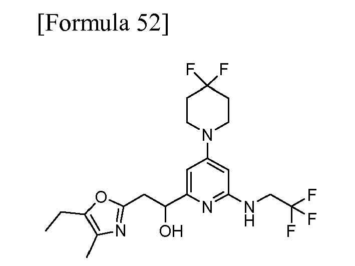

### 1-{4-(4,4-difluoro-1-piperidinyl)-6-[(2,2,2-trifluoroethyl)amino]-2-pyridinyl}-2-(5-ethy 1-4-methyl-1,3-oxazol-2-yl)ethanol

By the same method as in Example 19, methods equivalent thereto or combinations of these with usual methods using {4-(4,4-difluoro-1-piperidinyl)-6-[2-(5-ethyl-1-methyl-1,3-oxazol-2-yl)1-hydroxyethyl] -2-pyridinyl}(2,2,2-trifluoroethyl)carbamic acid tert-butyl ester obtained in Reference Example 2-66, the title compound was obtained from an earlier fraction after further optical resolution with an optically active column (CHIRALPAK AD-H column manufactured by Daicel Chemical Industries, Ltd.; 0.1 % diethylamine-hexane/isopropanol=3/1).
¹ H-NMR(CDC1₃ )δ:1.18(3H,t,J=7.6Hz),1.94-2.07(7H,m),2.57(2H,q,J=7.6Hz),3.02(1H, dd,J=8.6,15.2Hz),3.14(1H,dd,J=4.5,15.4Hz),3.43-3.48(4H,m),3.98-4.15(2H,m),4.46-4.5 8(2H,m),4.90-4.97(1H,m),5.73(1H,d,J=2.0Hz),6.29(1H,d,J=2.0Hz). mass:449(M+1)⁺.

### Example 21

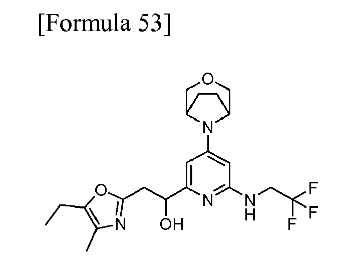

### 2-(5-ethyl-4-methyl-1,3-oxazol-2-yl)-1-{4-(3-oxa-8-azabicyclo[3,2,1]octo-8-yl)-6-[(2,2, 2-trifluoroethyl)amino]-2-pyridinyl}ethanol

By the same method as in Example 19, methods equivalent thereto or combinations of these with usual methods using [6-[2-(5-ethyl-4-methyl-1,3-oxazol-2-yl)-1-hydroxyethyl]-4-(3-oxa-azabicyclo[3,2,1]oct o-8-yl)-2-pyridinyl](2,2,2-trifluoroethyl)carbamic acid tert-butyl ester obtained in Reference Example 2-67, the title compound was obtained from an earlier fraction after further optical resolution with an optically active column (CHIRALPAK AD-H column manufactured by Daicel Chemical Industries, Ltd.; 0.1 % diethylamine-hexane/ethanol=3/1).
¹ H-NMR(CDC1₃ )δ:1.18(3H,t,J=7.6Hz),1.97-2.13(7H,m),2.57(2H,q,J=7.5Hz),3.02(1H, dd,J=8.5,15.4Hz),3.13(1H,dd,J=4.6,15.4Hz),3.47-3.52(2H,m),3.78(2H,t,J=9.8Hz),3.95-4.13(4H,m),4.44-4.63(2H,m),4.89-4.95(1H,m),5.60(1H,d,J=2.0Hz),6.13(1H,d,J=1.5Hz). mass:441(M+1)⁺.

### Example 22

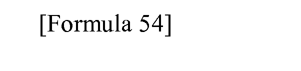

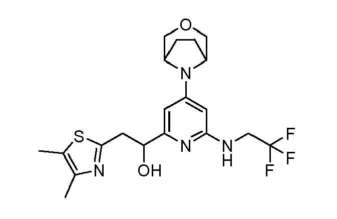

### 2-(4,5-dimethyl-1,3-thiazol-2-yl)-1-{4-(3-oxa-8-azabic[3,2,1]octo-8-yl)-6-[(2,2,2-tr ifluoroethyl)amino]-2-pyridinyl}ethanol

By the same method as in Example 19, methods equivalent thereto or combinations of these with usual methods using [6-[2-(4,5-dimethyl-1,3-thiazol-2-yl)-1-hydroxyethyl]-4-(3-oxa-8-azabicyclo[3,2,1]octo-8-yl)-2-pyridinyl](2,2,2-trifluoroethyl)carbamic acid tert-butyl ester obtained in Reference Example 2-68, the title compound was obtained from an earlier fraction after further optical resolution with an optically active column (CHIRALPAK AD-H column manufactured by Daicel Chemical Industries, Ltd.; 0.1 % diethylamine-hexane/isopropanol=3/2).
¹ H-NMR(CDCl₃ )δ:1.97-2.12(4H,m),2.30(6H,s),3.17(1H,dd,J=8.2,14.9Hz),3.33-3.40(1 H,m),3.49(2H,d,J=10.9Hz),3.77(2H,dd,J=6.3,10.6Hz),4.00-4.10(4H,m),4.48-4.57(1H,m ),4.79-4.88(2H,m),5.60(1H,s),6.17(1H,s).
mass:443(M+1)⁺.

### Example 23

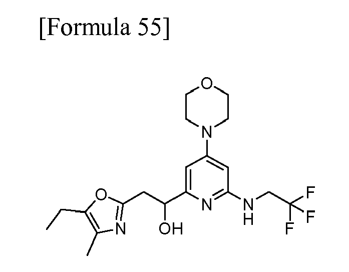

### 2-(5-ethyl-4-methyl-1,3-oxazol-2-yl)-1-{4-(4-morpholinxl)-6-[(2,2,2-trifluoroethyl)ami no]-2-pyridinyl}ethanol

By the same method as in Example 19, methods equivalent thereto or combinations of these with usual methods using [6-[2-(5-ethyl-4-methyl-1,3-oxazol-2-yl)-1-hydroxyethyl]-4-(4-morphohnyl)-2-pyridiny l](2,2,2-trifluoroethyl)carbamic acid tert-butyl ester Reference Example 2-69, the title compound was obtained from an earlier fraction after further optical resolution with an optically active column (CHIRALPAK AD-H column manufactured by Daicel Chemical Industries, Ltd.; 0.1% diethylamine-hexane/ethanol=4/1).
¹ H-NMR(CDCl₃ )δ:1.18(3H,t,J=7.6Hz),2.06(3H,s),2.57(2H,q,J=7.6Hz),3.00(1H,dd,J=8 .8,15.1Hz),3.14(1H,dd,J=4.1,15.4Hz),3.20-3.24(4H,m),3.77-3.82(4H,m),3.99-4.13(2H, m),4.47-4.59(2H,m),4.94(1H,dd,J=4.1,8.5Hz),5.70(1H,d,J=2.0Hz),6.27(1H,d,J=2.0Hz).
mass:415(M+1)⁺.

### Example 24

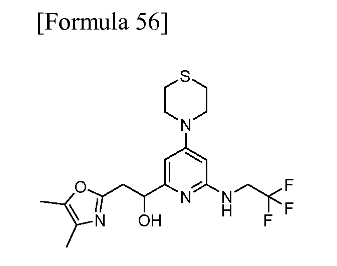

### 2-(4,5-dimethyl-1,3-oxazol-2-yl)-1-{4-(4-thiomorpholinyl)-6-[(2,2,2-trifluoroethyl)ami no]-2-pyridinyl}ethanol

By the same method as in Example 19, methods equivalent thereto or combinations of these with usual methods using [6-[2-(4,5-dimethyl-1,3-oxazol-2-yl)-1-hydroxyethyl]-4-(4-thiomorpholinyl)-2-pyridiny l](2,2,2-trifluoroethyl)carbamic acid tert-butyl ester obtained in Reference Example 2-64, the title compound was obtained from an earlier fraction after further optical resolution with an optically active column (CHIRALPAK AD-H column manufactured by Daicel Chemical Industries, Ltd.; 0.1% diethylamine-hexane/isopropanol=2/1). ¹ H-NMR(CDCl₃ )δ:2.05(3H,s),2.20(3H,s),2.60-2.64(4H,m),2.96-3.15(2H,m),3.69-3.73( 4H,m),3.97-4.16(2H,m),4.45-4.51(2H,m),4.89-4.94(1H,m),5.66(1H,d,J=2.0Hz),6.21(1H ,d,J=2.0Hz).
mass:417(M+1)⁺.

### Example 25

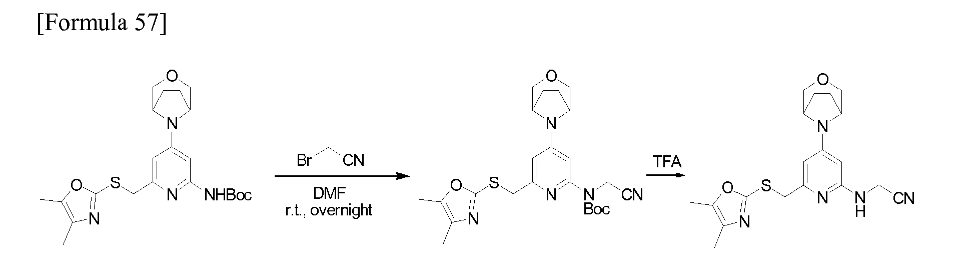

### {[6-{[(4,5-dimethyl-1,3-oxazol-2-yl)thio]methyl}-4-(3-oxa-8-azabicyclo[3,2,1]octo-8-y 1)-2-pyridinyl]amino}acetonitrile

In N,N-dimethylformamide (2.00 mL) was dissolved [6-{[(4,5-dimethyl-1,3-oxazol-2-yl)thio]methyl}-4-(3-oxa-8-azabicyclo[3,2,1]octo-8-yl) -2-pyridinyl]carbamic acid tert-butyl ester (153 mg) obtained in Reference Example 2-21. To the solution was added 60% sodium hydride (27.4 mg) at 0°C, and the mixture was stirred at the same temperature for 30 minutes. To the reaction solution was added bromoacetonitrile (0.12 mL), and the mixture was stirred overnight at room temperature. Water was added to the reaction solution, the mixture was extracted with ethyl acetate, and the organic layer was then washed with water and a saturated saline solution and dried over anhydrous sodium sulfate. Insoluble matters were filtered out, and the filtrate was under reduced pressure. The resultant residue was purified by NH silica gel column chromatography (hexane/ethyl acetate=1/0 to 2/1) to give a crude product. To the resultant crude product (112 mg) was added trifluoroacetic acid (1.00 mL), and the mixture was stirred at room temperature for 30 minutes. The reaction solution was under reduced pressure, a saturated aqueous sodium hydrogencarbonate solution was added to the resultant residue, and the mixture was extracted with chloroform. The organic layer was washed with a saturated saline solution and dried over anhydrous sodium sulfate. Insoluble matters were filtered out, the filtrate was under reduced pressure, and the resultant residue was purified by silica gel column chromatography (hexane/ethyl acetate=9/1 to 0/1) to yield the title compound (53.0 mg) as a colorless amorphous substance.
¹ H-NMR(CDC1₃ )δ:1.97-2.14(7H,m),2.21(3H,s),3.49(2H,d,J=10.6Hz),3.77(2H,d,J=10.6 Hz),4.00-4.05(2H,m),4.16(2H,s),4.28(2H,d,J=6.6Hz),4.52-4.58(1H,m),5.61(1H,d,J=2.0 Hz),6.27(1H,d,J=2.0Hz).
mass:386(M+1)⁺.

The compounds of Example 26 to Example 34 were obtained by the same method as in Example 25, methods equivalent thereto or combinations of these with usual methods using the corresponding Boc protected compounds instead of [6-{[(4,5-dimethyl-1,3-oxazol-2-yl)thio]methyl}-4-(3-oxa-8-azabicyclo[3,2,1]octo-8-yl) -2-pyridinyl]carbamic acid tert-butyl ester obtained in Reference Example 2-21.

### Example 26

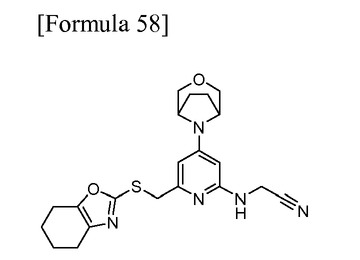

### ({4-(3-oxa-8-azabicyclo[3,2,1]octo-8-yl)-6-[(4,5,6,7-tetrahydro-1,3-benzoxazol-2-ylthio )methyl]-2-pyridinyl}amino)acetonitrile

The title compound was obtained by the same method as in Example 25, methods equivalent thereto or combinations of these with usual methods using {4-(3-oxa-8-azabicyclo[3,2,]octo-8-yl)-6-[(4,5,6,7-tetrahydro-1,3-benzoxazol-2-ylthio) methyl]-2-pyridinyl}carbamic acid tert-butyl ester obtained in Reference Example 2-22.
¹ H-NMR(CDC1₃ )δ:1.73-2.18(8H,m),2.44-2.65(4H,m),3.49(2H,d,J=10.9Hz),3.77(2H,d, J=10.9Hz),4.00-4.05(2H,m),4.27(2H,s),4.28(2H,d,J=6.3Hz),4.53-4.60(1H,m),5.61(1H,d ,J=2.0Hz),6.27(1H,d,J=2.0Hz).
mass:412(M+1)⁺.

### Example 27

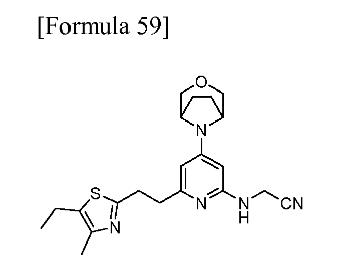

### {[6-[2-(5-ethyl-4-methyl-1,3-thiazol-2-yl)ethyl]-4-(3-oxa-8-azabicyclo[3,2,1]octo-8-yl)-2-pyridinyl]amino}acetonitrile

The title compound was obtained by the same method as in Example 25, methods equivalent thereto or combinations of these with usual methods using [6-[2-(5-ethyl-4-methyl-1,3-thiazol-2-yl)ethyl]-4-(3-oxa-8-azabicyclo[3,2,11]octo-8-yl)-2 -pyridinyl]carbamic acid tert-butyl ester obtained in Reference Example 2-78.
¹ H-NMR(CDC1₃ )δ:1.21(3H,t,J=7.3Hz),1.99-2.14(4H,m),2.30(3H,s),2.69(2H,q,J=7.3Hz ),2.99(2H,t,J=7.8Hz),3.33(2H,t,J=7.8Hz),3.49(2H,d,J=11.2Hz),3.78(2H,d,J=11.2Hz),4.0 2(2H,brs),4.32(2H,d,J=6.3Hz),4.43-4.47(1H,m),5.59(1H,d,J=2.0Hz),6.04(1H,d,J=2.0Hz ).
mass:398(M+1)⁺.

### Example 28

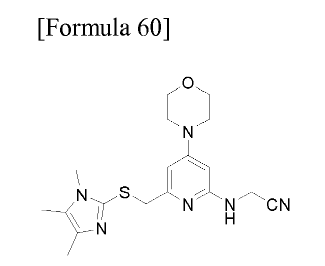

### [(4-(4-morpholinyl)-6-{[(1,4,5-trimethyl-1H-imidazol-2-yl)thio]methyl}-2-pyridinyl)a mino]acetonitrile

The title compound was obtained by the same method as in Example 25, methods equivalent thereto or combinations of these with usual methods using 4-(4-morpholinyl)-6-{[(1,4,5-trimethyl-1H-imidazol-2-yl)thio]methyl}-2-pyridinyl)carb amic acid tert-butyl ester obtained in Reference Example 2-25.
¹ H-NMR(CDCl₃)δ:2.07(3H,s),2.17(3H,s),3.14(4H,t,J=5.1Hz),3.26(3H,s),3.77(4H,t,J=5 .1Hz),3.98(2H,s),4.29(2H,d,J=6.3Hz),4.68(1H,brs),5.69(1H,d,J=2.0Hz),6.02(1H,d,J=2.0 Hz).
mass:373(M+1)⁺.

### Example 29

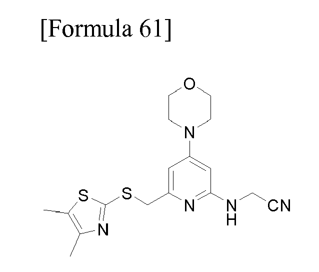

### {[6-{[(4,5-dimethyl-1,3-thiazol-2-yl)thio]methyl}-4-(4-morpholinyl)-2-pyridinyl]amino }acetonitrile

The title compound was obtained by the same method as in Example 25, methods equivalent thereto or combinations of these with usual methods using [6- {[(4,5-dimethyl-1,3-thiazol-2-yl)thio]methyl}-4-(4-morpholinyl)-2-pyridinyl]carbam ic acid tert-butyl ester obtained in Reference Example 2-23.
¹ H-NMR(CD₃ OD)δ:2.25(3H,s),2.30(3H,s),3.20(4H,t,J=5.0Hz),3.76(4H,t,J=5.0Hz),4.1 9(2H,s),4.26(2H,s),5.86(1H,d,J=2.0Hz),6.35(1H,d,J=2.0Hz).
mass:376(M+1)⁺.

### Example 30

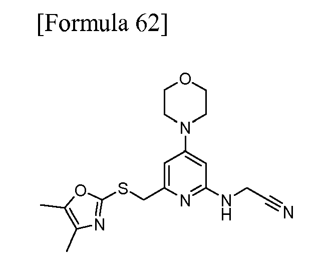

### {[6-{[(4,5-dimethyl-1,3-oxazol-2-yl)thio]methyl}-4-(4-morpholinyl)-2-pyridinyl]amino }acetonitrile

The title compound was obtained by the same method as in Example 25, methods equivalent thereto or combinations of these with usual methods using [6-{[(4,5-dimethyl-1,3-oxazol-2-yl)thio]methyl}-4-(4-morpholinyl)-2-pyridinyl]carbam ic acid tert-butyl ester obtained in Reference Example 2-26.
¹ H-NMR(CDCl₃)δ:2.05(3H,d,J=0.8Hz),2.21(3H,d,J=0.8Hz),3.19-3.24(4H,m),3.77-3.8 3(4H,m),4.27(2H,s),4.29(2H,d,J=6.3Hz),4.56-4.65(1H,m),5.71(1H,d,J=2.0Hz),6.39(1H, d,J=2.0Hz).
mass:360(M+1)⁺.

### Example 31

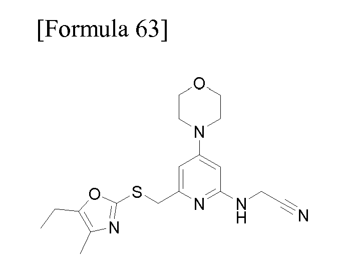

### {[6-{[(5-ethyl-4-methyl-1,3-oxazol-2-yl)thio]methyl}-4-(4-morpholinyl)-2-pyridinyl]a mino}acetonitrile

The title compound was obtained by the same method as in Example 25, methods equivalent thereto or combinations of these with usual methods using [6-{[(5-ethyl-4-methyl-1,3-oxazol-2-yl)thio]methyl}-4-(4-morpholinyl)-2-pyridinyl]car bamic acid tert-butyl ester obtained in Reference Example 2-24.
¹ H-NMR(CDCl₃)δ:1.18(3H,t,J=7.4Hz),2.07(3H,s),2.57(2H,q,J=7.4Hz),3.19-3.23(4H,m ),3.77-3.82(4H,m),4.28(2H,s),4.29(2H,d,J=6.3Hz),4.58-4.59(1H,m),5.71(1H,d,J=2.0Hz) ,6.39(1H,d,J=2.0Hz).
mass:374(M+1)⁺.

### Example 32

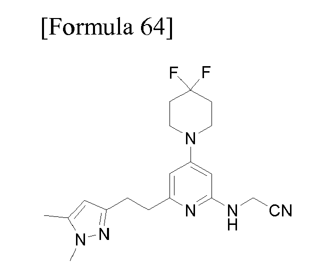

### ({4-(4,4-difluoro-1-piperidinyl)-6-[2-(1,5-dimethyl-1H-pyrazol-3-yl)ethyl]-2-pyridinyl} amino)acetonitrile

The title compound was obtained by the same method as in Example 25, methods equivalent thereto or combinations of these with usual methods using {4-(4,4-difluoro-1-piperidinyl)-6-[2-(1,5-dimethyl-1H-pyrazol-3-yl)ethyl]-2-pyridinyl}c arbamic acid tert-butyl ester obtained in Reference Example 2-76.
¹ H-NMR(CDCl₃)δ:1.93-2.06(4H,m),2.22(3H,s),2.84-3.00(4H,m),3.41-3.48(4H,m),3.72 (3H,s),4.31-4.37(2H,m),4.44-4.53(1H,m),5.70-5.73(1H,m),5.82(1H,s),6.18-6.21(1H,m).
mass:375(M+1)⁺.

### Example 33

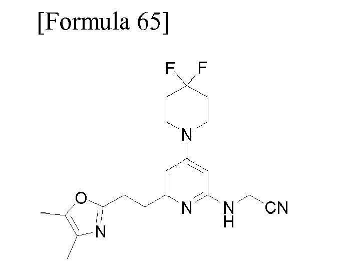

### ({4-(4,4-difluoro-1-piperidinyl)-6-[2-(4,5-dimethyl-1,3-oxazol-2-yl)ethyl]-2-pyridinyl}a mino)acetonitrile

The title compound was obtained by the same method as in Example 25, methods equivalent thereto or combinations of these with usual methods using {4-(4,4-difluoro-1-piperidinyl)-6-[2-(4,5-dimethyl-1,3-oxazol-2-yl)ethyl]-2-pyridinyl}c arbamic acid tert-butyl ester obtained in Reference Example 2-77.
¹ H-NMR(CDCl₃)δ:1.94-2.09(7H,m),2.19(3H,s),2.96-3.13(4H,m),3.39-3.51(4H,m),4.28 -4.33(2H,m),4.45-4.52(1H,m),5.71(1H,d,J=2.0Hz),6.18(1H,d,J=2.0Hz).
mass:376(M+1)⁺.

### Example 34

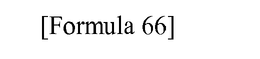

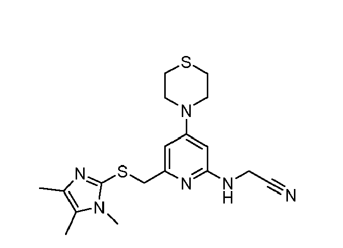

### [(4-(4-thiomorpholinyl)-6-{[(1,4,5-trimethyl-1H-imidazol-2-yl)thio]methyl}-2-pyridiny 1)amino]acetonitrile

The title compound was obtained by the same method as in Example 25, methods equivalent thereto or combinations of these with usual methods using (4-(4-thiomorpholinyl)-6-{[(1,4,5-trimethyl-1H-imidazol-2-yl)thio]methyl}-2-pyridinyl )carbamic acid tert-butyl ester obtained in Reference Example 2-29.
1H-NMR(CDCl₃)δ:2.07(3H,s),2.16(3H,s),2.54-2.57(4H,m),3.25(3H,s),3.60-3.65(4H,m) ,3.93(2H,s),4.30(2H,d,J=6.5Hz),4.66(1H,t,J=6.5Hz),5.65(1H,d,J=2.0Hz),5.91(1H,d,J=2. 0Hz)
mass:389(M+1)⁺.

The compounds of Example 35 to Example 38 were obtained by the same method as in Example 25, methods equivalent thereto or combinations of these with usual methods using the corresponding Boc protected compounds instead of [6- {[(4,5-dimethyl-1,3-oxazol-2-yl)thio]methyl}-4-(3-oxa-8-azabicyclo[3,2,1]octo-8-yl) -2-pyridinyl]carbamic acid tert-butyl ester obtained in Reference Example 2-21 and using alkyl bromide instead of bromoacetonitrile.

### Example 35

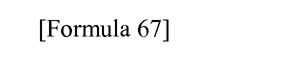

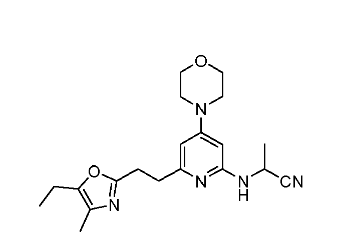

### 2-{[6-[2-(5-ethyl-4-methyl-1,3-oxazol-2-yl)ethyl]-4-(4-morpholinyl)-2-piperidinyl]ami no}propanenitrile

The title compound was obtained by the same method as in Example 25, methods equivalent thereto or combinations of these with usual methods using [6-[2-(5-ethyl-4-methyl-1,3-oxazol-2-yl)ethyl]-4-(4-morpholinyl)-2-pyridinyl]carbamic acid tert-butyl ester obtained in Reference Example 2-74 and 2-bromopropionitrile.
¹ H-NMR(CDCl₃)δ:1.18(3H,t,J=7.6Hz),1.63(3H,d,J=6.8Hz),2.06(3H,s),2.56(2H,q,J=7. 6Hz),2.98-3.14(4H,m),3.20(4H,t,J=5.0Hz),3.80(4H,t,J=5.0Hz),4.33(1H,d,J=7.8Hz),4.89 -4.97(1H,m),5.67(1H,d,J=2.2Hz),6.15(1H,d,J=2.2Hz).
mass:370(M+1)⁺.

### Example 36

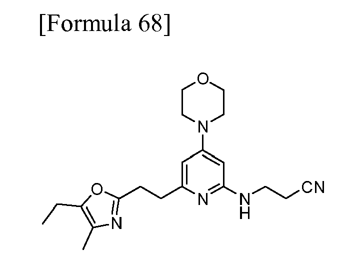

### 3-{[6-[2-(5-ethyl-4-methyl-1,3-oxazol-2-yl)ethyl]-4-(4-morpholinyl)-2-pyridinyl]amino }propanenitrile

The title compound was obtained by the same method as in Example 25, methods equivalent thereto or combinations of these with usual methods using [6-[2-(5-ethyl-4-methyl-1,3-oxazol-2-yl)ethyl]-4-(4-morpholinyl)-2-pyridinyl]carbamic acid tert-butyl ester obtained in Reference Example 2-74 and 3-bromopropionitrile.
¹ H-NMR(CDCl₃)δ:1.17(3H,t,J=7.5Hz),2.05(3H,s),2.56(2H,q,J=7.5Hz),2.72(2H,t,J=6.3 ),2.96-3.09(4H,m),3.21(4H,t,J=4.9Hz),3.64(2H,q,J=6.3Hz),3.80(4H,t,J=4.9Hz),5.63(1H ,d,J=2.2Hz),6.09(1H,d,J=2.2Hz).
mass:370(M+1)⁺.

### Example 37

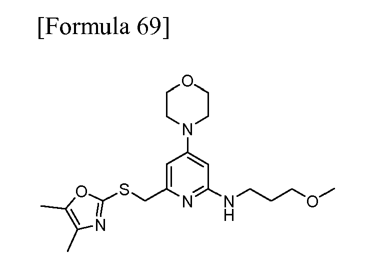

### 6-{[(4,5-dimethyl-1,3-oxazol-2-yl)thio]methyl}-N-(3-methoxypropyl)-4-(4-morpholinyl )-2-pyridinamine

The title compound was obtained by the same method as in Example 25, methods equivalent thereto or combinations of these with usual methods using [6-{[(4,5-dimethyl-1,3-oxazol-2-yl)thio]methyl}-4-(4-morpholinyl)-2-pyridinyl]carbam ic acid tert-butyl ester obtained in Reference Example 2-26 and 3-methoxy-1-bromopropane.
¹ H-NMR(CDCl₃)δ:1.83-1.90(2H,m),2.05(3H,s),2.19(3H,s),3.19-3.24(4H,m),3.29(2H,q ,J=5.9Hz),3.34(3H,s),3.49(3H,t,J=5.9Hz),3.78-3.82(4H,m),4.23(2H,s),4.62-4.68(1H,m), 5.61(1H,d,J=2.0Hz),6.25(1H,d,J=2.0Hz).
mass:393(M+1)⁺.

### Example 38

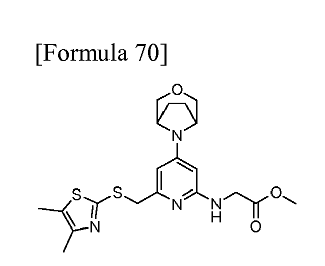

### {[6-1[(4,5-dimethyl-1,3-thiazol-2-yl)thio]methyl}-4-(3-oxa-8-azabicyclo[3,21]octo-8-y l)-2-pyridinyl]amino}acetic acid methyl ester

The title compound was obtained by the same method as in Example 25, methods equivalent thereto or combinations of these with usual methods using [6-{[(4,5-dimethyl-1,3-thiazo1-2-yl)thio]methyl)-4-(3-oxa-8-azabicydo[3,2,1]octo-8-yl)

-2-pyridinyl]carbamic acid tert-butyl ester obtained in Reference Example 2-20 and methyl bromoacetate.
¹ H-NMR(CDCl₃)δ:1.94-2.12(4H,m),2.28(6H,s),3.47(2H,d,J=10.9Hz),3.73-3.79(5H,m), 3.96-4.03(2H,m),4.11(2H,d,J=5.5Hz),4.19(2H,s),4.78-4.79(1H,m),5.56(1H,d,J=2.0Hz), 6.16(1H,d,J=2.0Hz).
mass:435(M+1)⁺.

The compounds of Example 39 and Example 40 were obtained by the same method as in Example 25, methods equivalent thereto or combinations of these with usual methods using the corresponding Boc protected compounds instead of [6- {[(4,5-dimethyl-1,3-oxazol-2-yl)thio]methyl}-4-(3-oxa-8-azabicyclo[3,2,1]octo-8-yl) -2-pyridinyl]carbamic acid tert-butyl ester obtained in Reference Example 2-21 and using 2,2,2-trifluoroethyl trifluoromethanesulfonate instead of bromoacetonitrile.

### Example 39

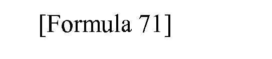

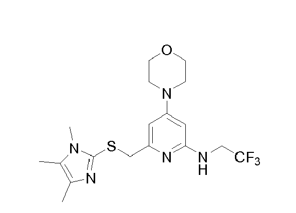

### 4-(4-morpholinyl)-N-(2,2,2-trifluoroethyl)-6-{[1,4,5-trimethyl-1H-imidazol-2-yl)thio] methyl}-2-pyridinamine

The title compound was obtained by the same method as in Example 25, methods equivalent thereto or combinations of these with usual methods using (4-(4-morpholinyl)-6-{[(1,4,5-trimethyl-1H-imidazol-2-yl)thio]methyl}-2-pyridinyl)car bamic acid tert-butyl ester obtained in Reference Example 2-25.
¹ H-NMR(CDCl₃)δ:2.06(3H,s),2.16(3H,s),3.12-3.14(4H,brm),3.23(3H,s),3.77(4H,t,J=4. 9Hz),3.94(2H,s),3.98-4.06(2H,m),4.65(1H,brs),5.67(1H,d,J=1.5Hz),5.95(1H,brs).
mass:416(M+1)⁺.

### Example 40

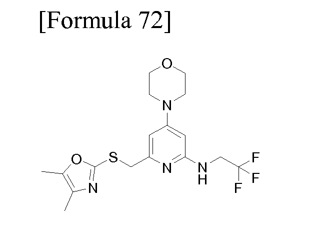

### 6-{[(4,5-dimethyl-1,3-oxazol-2-yl)thio]methyl}-4-(4-morpholinyl)-N-(2,2,2-trifluoroeth yl)-2-pyridinamine

The title compound was obtained by the same method as in Example 25, methods equivalent thereto or combinations of these with usual methods using [6-{[(4,5-dimethyl-1,3-oxazol-2-yl)thio]methyl}-4-(4-morpholinyl)-2-pyridinyl]carbam ic acid tert-butyl ester obtained in Reference Example 2-26.
¹ H-NMR(CDCl₃)δ:2.08(3H,s),2.24(3H,s),3.22(4H,t,J=5.1Hz),3.79(4H,t,J=5.1Hz),3.97-4.08(2H,m),4.26(2H,s),4.55(1H,brs),5.68(1H,d,J=2.0Hz),6.34(1H,d,J=2.0Hz).
mass:403(M+1)⁺.

The compounds of Example 41 and Example 42 were obtained by the same method as in Example 25, methods equivalent thereto or combinations of these with usual methods using the corresponding Boc protected compounds instead of [6-{[(4,5-dimethyl-1,3-oxazol-2-yl)thio]methyl}-4-(3-oxa-8-azabicyclo[3,2,1]octo-8-yl) -2-pyridinyl]carbamic acid tert-butyl ester obtained in Reference Example 2-21 and using 2-methoxyethyl p-toluenesulfonic acid instead of bromoacetonitrile.

### Example 41

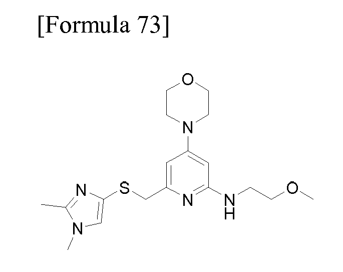

### 6-{[(1,2-dimethyl-1H-imidazol-4-yl)thio]methyl}-N-(2-methoxyethyl)-4-(4-morpholiny 1)-2-pyridinamine

The title compound was obtained by the same method as in Example 25, methods equivalent thereto or combinations of these with usual methods using [6-{[(1,2-dimethyl-1H-imidazol-4-yl)thio]methyl}-4-(4-morpholinyl)-2-pyridinyl]carba mic acid tert-butyl ester obtained in Reference Example 2-27.
¹ H-NMR(CDCl₃)δ:2.35(3H,s),3.22(4H,t,J=5.0Hz),3.37(3H,s),3.41(2H,q,J=5.4Hz),3.50 (3H,s),3.58(2H,t,J=5.4Hz),3.79(4H,t,J=5.0Hz),3.91(2H,s),5.64(1H,brs),6.20(1H,d,J=2.0 Hz),6.79(1H,s).
mass:378(M+1)⁺.

### Example 42

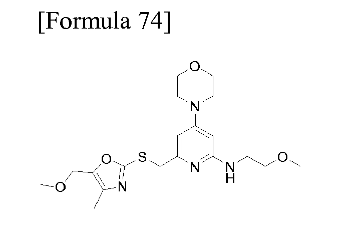

### N-(2-methoxyethyl)-6-({[5-(methoxymethyl)-4-methyl-1,3-oxazol-2-yl]thio}methyl)-4-(4-morpholinyl)-2-pyridinamine

The title compound was obtained by the same method as in Example 25, methods equivalent thereto or combinations of these with usual methods using [6-({[5-(methoxymethyl)-4-methyl-1,3-oxazol-2-yl]thio}methyl)-4-(4-morpholinyl)-2-p yridinyl]carbamic acid tert-butyl ester obtained in Reference Example 2-28.
¹ H-NMR(CDCl₃)δ:2.15(3H,s),3.24-3.26(4H,br),3.34(3H,s),3.37(3H,s),3.42(2H,q,J=5.4 Hz),3.57(2H,t,J=5.4Hz),3.80(4H,t,J=5.0Hz),4.30(2H,s),4.35(2H,s),5.65(1H,brs),6.30(1 H,brs).
mass:409(M+1)⁺.

### Example 43

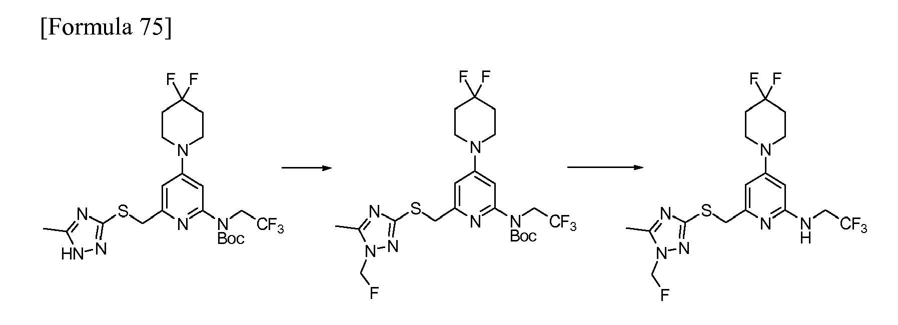

### 4-(4,4-difluoro-1-piperidinyl)-6-({[1-(fluoromethyl)-5-methyl-1H-1,2,4-triazo1-3-yl]thi o}methyl)-N-(2,2,2-trifluoroethyl)-2-pyridinamine

In N,N-dimethylformamide (1.00 mL) was dissolved (4-(4,4-difluoro-1-piperidinyl)-6-{[(5-methyl-1H-1,2,4-triazol-3-yl)thio]methyl}-2-pyri dinyl)(2,2,2-trifluoroethyl)carbamic acid tert-butyl ester (60.0 mg) obtained in Reference Example 2-40. To the solution were added potassium carbonate (32.0 mg) and fluoromethyl triflate (47.0 µL), and the mixture was stirred at room temperature for 1 hour. A saturated ammonium chloride aqueous solution was added to the reaction solution, and the mixture was extracted with ethyl acetate. The organic layer was washed with a saturated saline solution and dried over anhydrous magnesium sulfate. Insoluble matters were filtered out, and the filtrate was under reduced pressure. The resultant residue was dissolved in trifluoroacetic acid (1.00 mL), and the solution was stirred at room temperature for 2 hours. The reaction solution was under reduced pressure, the resultant residue was dissolved in chloroform and washed with a saturated aqueous sodium hydrogencarbonate solution, and the organic layer was then dried over anhydrous sodium sulfate. Insoluble matters were filtered out, the filtrate was under reduced pressure, and the resultant residue was purified by NH silica gel column chromatography (hexane/ethyl acetate=98/2 to 3/7) to yield the title compound (23.0 mg) as a pale yellow oil.
¹ H-NMR(CDCl₃)δ:1.94-2.04(4H,m),2.52(3H,s),3.42-3.45(4H,m),4.01-4.09(2H,m),4.24 (2H,s),4.48(1H,brs),5.71(1H,d,J=2.3Hz),5.89(1H,s),6.02(1H,s),6.41(1H,d,J=2.3Hz).
mass:455(M+1)⁺.

The compounds of Example 44 to Example 46 were obtained by the same method as in Example 43, methods equivalent thereto or combinations of these with usual methods using the corresponding Boc protected compounds instead of (4-(4,4-difluoro-1-piperidinyl)-6-{[(5-methyl-1H-1,2,4-triazol-3-yl)thio]methyl}-2-pyri dinyl)(2,2,2-trifluoroethyl)carbamic acid tert-butyl ester obtained in Reference Example 2-40 and using the corresponding alkyl iodides instead of fluoromethyl triflate.

### Example 44

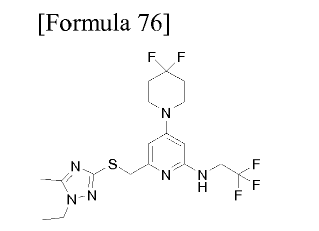

### 4-(4,4-difluoro-1-piperidinyl)-6-{[(1-ethyl-5-methyl-1H-1,2,4-triazol-3-yl)thio]methyl} -N-(2,2,2-trifluoroethyl)-2-pyridinamine

The title compound was obtained by the same method as in Example 43, methods equivalent thereto or combinations of these with usual methods using (4-(4,4-difluoro-1-piperidinyl)-6-{[(5-methyl-1H-1,2,4-triazol-3-yl)thio]methyl}-2-pyri dinyl)(2,2,2-trifluoroethyl)carbamic acid tert-butyl ester obtained in Reference Example 2-40 and ethyl iodide.
¹ H-NMR(CDCl₃)δ:1.42(3H,t,J=7.2Hz),1.94-2.04(4H,m),2.41(3H,s),3.43-3.45(4H,m),4. 01-4.09(4H,m),4.24(2H,s),4.53(1H,brs),5.71(1H,d,J=2.0Hz),6.42(1H,d,J=2.0Hz).
mass:451(M+1)⁺.

### Example 45

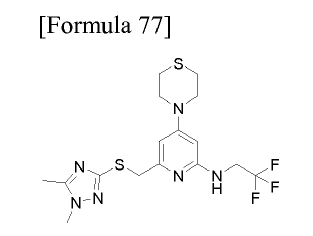

### 6-{[(1,5-dimethyl-1H-1,2,4-triazol-3-yl)thio]methyl}-4-(4-thiomorpholinyl)-N-(2,2,2-tri fluoroethyl)-2-pyridinamine

The title compound was obtained by the same method as in Example 43, methods equivalent thereto or combinations of these with usual methods using [6- {[(5-methyl-1H-1,2,4-triazol-3-yl)thio]methyl}-4-(4-thiomorpholinyl)-2-pyridinyl](2 ,2,2-trifluoroethyl)carbamic acid tert-butyl ester obtained in Reference Example 2-50 and methyl iodide.
¹ H-NMR(CDCl₃)δ:2.40(3H,s),2.59-2.62(4H,m),3.67-3.69(4H,m),3.74(3H,s),3.99-4.07( 2H,m),4.21(2H,s),4.58(1H,t,J=6.6Hz),5.63(1H,d,J=2.2Hz),6.32(1H,d,J=2.2Hz).
mass:419(M+1)⁺.

### Example 46

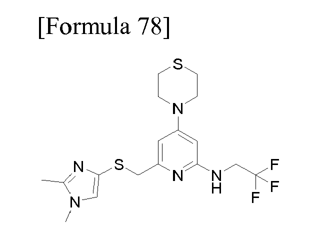

### 6-{[(1,2-dimethyl-1H-imidazol-4-yl)thio]methyl}-4-(4-thiomorpholinyl)-N-(2,2,2-triflu oroethyl)-2-pyridinamine

The title compound was obtained by the same method as in Example 43, methods equivalent thereto or combinations of these with usual methods using [6-{[(2-methyl-1H-imidazol-4-yl)thio]methyl}-4-(4-thiomorpholinyl)-2-pyridinyl](2,2,2 -trifluoroethyl)carbamic acid tert-butyl ester obtained in Reference Example 2-51 and methyl iodide.
¹ H-NMR(CDCl₃)δ:2.35(3H,s),2.58-2.60(4H,m),3.49(3H,s),3.67-3.69(4H,m),3.89(2H,s ),3.99-4.07(2H,m),4.55(1H,s),5.62(1H,d,J=2.2Hz),6.17(1H,d,J=2.2Hz),6.74(1H,s)
mass:418(M+1)⁺.

### Example 47

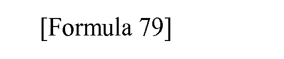

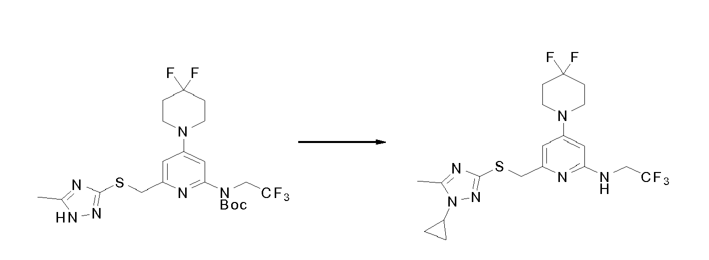

### 6-{[(1-cyclopropyl-5-methyl-1H-1,2,4-triazol-3-yl)thio]methyl}-4-(4,4-difluoro-1-piper idinyl)-N-(2,2,2-trifluoroethyl)-2-pyridinamine

In methylene chloride (1.00 mL) was dissolved (4-(4,4-difluoro-1-piperidinyl)-6-{[(5-methyl-1H-1,2,4-triazol-3-yl)thio]methyl}-2-pyri dinyl)(2,2,2-trifluoroethyl)carbamic acid tert-butyl ester (72.0 mg) obtained in Reference Example 2-40. To the solution were added pyridine (27.0 µL), copper (II) acetate (30.0 mg) and cyclopropyl bismuth (92.0 mg), and the mixture was stirred at 50°C for 3 days. The reaction solution was left standing to cool to room temperature and then under reduced pressure, trifluoroacetic acid (1.00 mL) was added to the resultant residue, and the mixture was stirred at room temperature for 1 hour. The reaction solution was under reduced pressure, and the resultant residue was dissolved in chloroform and washed with a saturated aqueous sodium hydrogencarbonate solution. The organic layer was washed with a saturated saline solution and dried over anhydrous magnesium sulfate. Insoluble matters were filtered out, the filtrate was under reduced pressure, and the resultant residue was purified by NH silica gel thin layer chromatography (hexane/ethyl acetate=3/1) to yield the title compound (12.0 mg) as a colorless oil.
¹ H-NMR(CDCl3)δ:1.05-1.10(2H,m),1.11-1.16(2H,m),1.94-2.02(4H,m),2.48(3H,s),3.28 -3.32(1H,m),3.42-3.45(4H,m),4.00-4.09(2H,m),4.21(2H,s),4.47(1H,brs),5.70(1H,d,J=2. 0Hz),6.41(1H,d,J=2.0Hz).
mass:463(M+1)⁺.

### Example 48

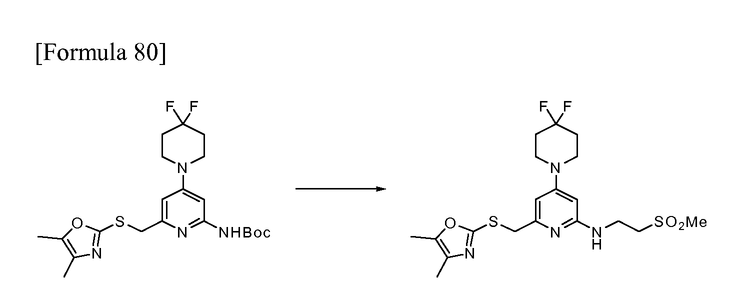

### 4-(4,4-difluoro-1-piberidinyl)-6-{[4,5-dimethyl-1,3-oxazol-2-yl)thio]methyl}-N-[2-(me thylsulfonyl)ethyl-2-pyridinamine

In N,N-dimethylformamide (1.00 mL) was dissolved (4-(4,4-difluoro-1-piperidinyl)-6-{[(4,5-dimethyl-1,3-oxazol-2-yl)thio]methyl}-2-pyridi nyl)carbamic acid tert-butyl ester (47.0 mg) obtained in Reference Example 2-31. To the solution was added 60% sodium hydride (12.0 mg) at 0°C, and the mixture was stirred for 5 minutes. Methyl vinyl sulfone (27.0 µL) was added, and the mixture was stirred for 2 hours. A saturated aqueous ammonium chloride solution was added to the reaction solution, and the mixture was extracted with ethyl acetate. The organic layer was washed with a saturated saline solution and dried over anhydrous magnesium sulfate. Insoluble matters were filtered out, the filtrate was under reduced pressure, and the resultant residue was purified by silica gel thin layer chromatography (hexane/ethyl acetate=4/1) to give a protector. The obtained protector was dissolved in trifluoroacetic acid (1.00 mL), and the solution was stirred at room temperature for 1 hour. The reaction solution was under reduced pressure, and the resultant residue was dissolved in chloroform and washed with a saturated aqueous sodium hydrogencarbonate solution. The organic layer was dried over anhydrous magnesium sulfate, insoluble matters were filtered out, and the filtrate was under reduced pressure. The resultant residue was purified by NH silica gel thin layer chromatography (ethyl acetate) to give the title compound (8.00 mg) as a colorless oil.
¹ H-NMR(CDCl₃)δ:1.94-2.04(4H,m),2.06(3H,s),2.21(3H,s),2.95(3H,s),3.38(2H,t,J=6.1 Hz),3.42-3.48(4H,m),3.87(2H,t,J=6.1Hz),4.24(2H,s),4.72(1H,brs),5.69(1H,d,J=2.0Hz),6 .31(1H,d,J=2.0Hz).
mass:461(M+1)⁺.

### Example 49

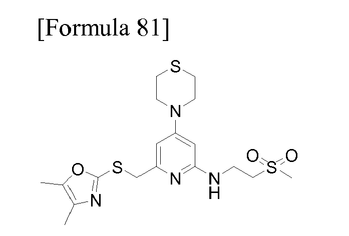

### 6-{[4,5-dimethyl-1,3-oxazol-2-yl)thio]methyl}-N-[2-(methylsulfonyl)ethyl-4-(4-thiom orpholinyl)-2-pyridinamine

The title compound was obtained by the same method as in Example 48, methods equivalent thereto or combinations of these with usual methods using [6-{[(4,5-dimethyl-1,3-oxazol-2-yl)thio]methyl}-4-(4-thiomorpholinyl)-2-pyridinyl]car bamic acid tert-butyl ester obtained in Reference Example 2-30.
¹ H-NMR(CDCl₃)δ:2.06-2.13(3H,m),2.18-2.22(3H,m),2.60-2.63(4H,m),2.95(3H,s),3.39 (2H,t,J=6.1Hz),3.65-3.72(4H,m),3.85(2H,t,J=6.0Hz),4.24(2H,s),4.84(1H,brs),5.62(1H,d ,J=2.0Hz),6.24(1H,d,J=2.0Hz).
mass:443(M+1)⁺.

### Example 50

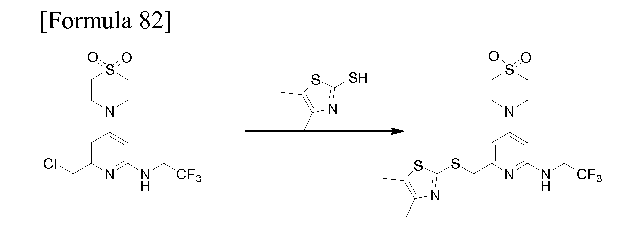

### 6-{[(4,5-dimethyl-1,3-thiazol-2-yl)thio]methyl}-4-(1,1-dioxide)-4-thiomorpholinyl)-N-( 2,2,2-trifluoroethyl)-2-pyridinamine

In N,N-dimethylformamide (2.00 mL) was dissolved 6-(chloromethyl)-4-(1,1-dioxide-4-thiomorpholinyl)-N-(2,2,2-trifluoroethyl)-2-pyridina mine (106 mg) obtained in Reference Example 2-61. To the solution were added potassium carbonate (62.0 mg) and 4,5-dimethyl-2-mercaptothiazole (52.0 mg), and the mixture was stirred overnight at room temperature. Water was added to the reaction solution, and the mixture was extracted with ethyl acetate. The organic layer was washed with water and a saturated saline solution and dried over anhydrous sodium sulfate. Insoluble matters were filtered out, the filtrate was under reduced pressure, and the resultant residue was purified by silica gel column chromatography (hexane/ethyl acetate=1/0 to 1/1) to yield the title compound (81.0 mg) as a white solid.
¹ H-NMR(DMSO-d₆)δ:2.22(3H,s),2.27-2.29(3H,m),3.05-3.10(4H,m),3.77-3.83(4H,m), 4.05-4.15(2H,m),4.20(2H,s),5.98(1H,d,J=2.0Hz),6.44(1H,d,J=2.0Hz),6.77(1H,t,J=6.8H z).
mass:467(M+1)⁺.

The compounds of Example 51 and Example 52 were obtained by the same method as in Example 50, methods equivalent thereto or combinations of these with usual methods using the corresponding pyrazole or oxazole derivatives instead of 6-(chloromethyl)-4-(1,1-dioxide-4-thiomorpholinyl)-N-(2,2,2-trifluoroethyl)-2-pyridina mine obtained in Reference Example 2-61.

### Example 51

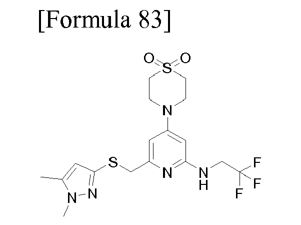

### 6-{[(1,5-dimethyl-1H-pyrazol-3-yl)thio]methyl}-4-(1,1-dioxide-4-thiomorpholinyl)-N-( 2,2,2-trifluoroethyl)-2-pyridinamine

The title compound was obtained by the same method as in Example 50, methods equivalent thereto or combinations of these with usual methods using 1,5-dimethyl-1H-pyrazol-3-thiol obtained in Reference Example 1-34.
¹ H-NMR(CDCl₃)δ:2.21(3H,s),2.95-3.03(4H,m),3.72(3H,s),3.85-3.92(4H,m),4.00(2H,s ),4.00-4.25(2H,m),4.66(1H,brs),5.70(1H,d,J=2.2Hz),5.99(1H,s),6.39(1H,d,J=2.2Hz).
mass:450(M+1)⁺.

### Example 52

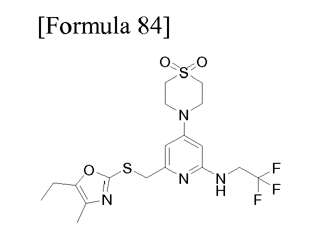

### 4-(1,1-dioxide-4-thiomorpholinyl)-6-{[(5-ethyl-4-methyl-1,3-oxazol-2-yl)thio]methyl}-N-(2,2,2-trifluoroethyl)-2-pyridinamine

The title compound was obtained by the same method as in Example 50, methods equivalent thereto or combinations of these with usual methods using 5-ethyl-4-methyl-1,3-oxazol-2-thiol obtained in Reference Example 1-17.
¹ H-NMR(DMSO-d₆)δ:1.09(3H,t,J=7.3Hz),1.98(3H,s),2.57(2H,q,J=7.3Hz),3.05-3.10(4 H,m),3.75-3.80(4H,m),3.98-4.10(2H,m),4.19(2H,s),5.96(1H,d,J=1.8Hz),6.44(1H,d,J=1. 8Hz),6.76(1H,t,J=6.8Hz).
mass:465(M+1)⁺.

### Example 53

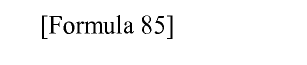

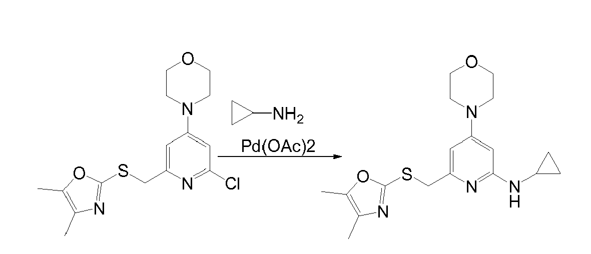

### N-cyclopropyl-6-{[(4,5-dimethyl-1,3-oxazol-2-yl)thio]methyl}-4-(4-morpholinyl)-2-pyr idinamine

In toluene (1.00 mL) was dissolved 4-(2-chloro-6-{[(4,5-dimethyl-1,3-oxazol-2-yl)thio]methyl}-4-pyridinyl)morpholine (40.0 mg) obtained in Reference Example 2-57. To the solution were added 2,2'-bis(diphenylphosphino)-1,1'-binaphthyl (46.2 mg), palladium (II) acetate (8.30 mg), cyclopropylamine (1.00 mL) and cesium carbonate (91.0 mg), and the mixture was stirred overnight under nitrogen atmosphere at 100°C. The temperature of the reaction solution was returned to room temperature, the reaction solution was Celite-filtered, and the filtrate was under reduced pressure. The resultant residue was purified by NH silica gel thin layer chromatography (hexane/ethyl acetate=1/1) to give the title compound (7.80 mg) as a colorless solid.
¹ H-NMR(CDCl₃)δ:0.53-0.57(2H,m),0.72-0.76(2H,m),2.05(3H,d,J=0.8Hz),2.19(3H,d,J =0.8Hz),2.42-2.47(1H,m),3.26(4H,t,J=4.5Hz),3.82(4H,t,J=4.5Hz),4.21(2H,s),4.98(1H,b rs),6.00(1H,d,J=2.0Hz),6.30(1H,d,J=2.0Hz).
mass:361(M+1)⁺.

### Example 54

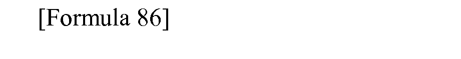

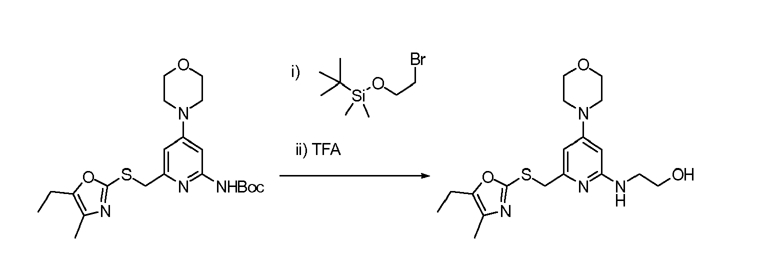

### 2-{[6-{[(5-ethyl-4-methyl-1,3-oxazol-2-yl)thio]methyl}-4-(4-morpholinyl)-2-pyridinyl] amino}ethanol

In N,N-dimethylformamide (2.00 mL) was dissolved [6-{[(5-ethyl-4-methyl-1,3-oxazol-2-yl)thio]methyl}-4-(4-morpholinyl)-2-pyridinyl]car bamic acid tert-butyl ester (100 mg) obtained in Reference Example 2-24. To the solution was added 60% sodium hydride (18.0 mg) at 0°C, and the mixture was stirred at room temperature for 30 minutes. To the reaction solution was added 2-bromoethoxy-tert-butyldimethylsilane (75.0 µL), and the mixture was stirred overnight at the same temperature. Water was added to the reaction solution, the mixture was then diluted with ethyl acetate, and the organic layer was washed with water and a saturated saline solution and dried over anhydrous sodium sulfate. Insoluble matters were filtered out, the filtrate was under reduced pressure, and the resultant residue was purified by silica gel column chromatography (hexane/ethyl acetate=1/1) to give a protector. The obtained protector was dissolved in trifluoroacetic acid (1.00 mL) at room temperature, and the solution was stirred at the same temperature for 1 hour. The reaction solution was under reduced pressure, the resultant residue was dissolves in chloroform, washed with a saturated aqueous sodium hydrogencarbonate solution and a saturated saline solution, and dried over anhydrous sodium sulfate. Insoluble matters were filtered out, the filtrate was under reduced pressure, and the resultant residue was purified by NH silica gel column chromatography (hexane/ethyl acetate=1/1) to yield the title compound (42.0 mg) as a white solid.
¹ H-NMR(CDCl₃)δ:1.17(3H,t,J=7.5Hz),2.06(3H,s),2.56(2H,q,J=7.5Hz),3.18-3.21(4H, m),3.45-3.50(2H,m),3.76-3.80(6H,m),4.22(3H,s),4.72(1H,brs),5.65(1H,d,J=2.0Hz),6.31 (1H,d,J=2.0Hz).
mass:379(M+1)⁺.

### Example 55

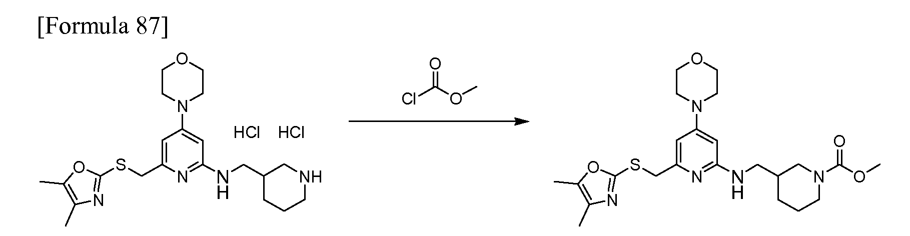

### 3-({[6-{[(4,5-dimethyl-1,3-oxazol-2-yl)thio]methyl}-4-(4-morpholinyl)-2-pyridinyl]ami no}methyl)-1-piperidine carboxylic acid methyl eater

In chloroform (2.00 mL) was dissolved 6-{[(4,5-dimethyl-1,3-oxazol-2-yl)thio]methyl}-4-(4-morpholinyl)-N-(3-piperidinylmet hyl)-2-pyridinamine dihydrochloride (14.0 mg) obtained in Reference Example 2-59. To the solution were added potassium carbonate (20.0 mg) and methyl chloroformate (4.00 µL), and the mixture was stirred overnight at room temperature. Water was added to the reaction solution, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by NH silica gel thin layer chromatography (hexane/ethyl acetate=1/2) to yield the title compound (6.60 mg) as a colorless oil.
¹ H-NMR(CDCl₃)δ:1.18-1.98(5H,m),2.05(3H,q,J=0.8Hz),2.18(3H,q,J=0.8Hz),2.75(1H, dd,J=9.8,12.9Hz),2.86-3.00(1H,m),3.01-3.29(6H,m),3.68(3H,s),3.74-3.86(5H,m),3.98-4 .05(1H,m),4.23(2H,s),4.67(1H,brs),5.57(1H,d,J=1.8Hz),6.26(1H,d,J=1.4Hz).
mass:476(M+1)⁺.

### Example 56

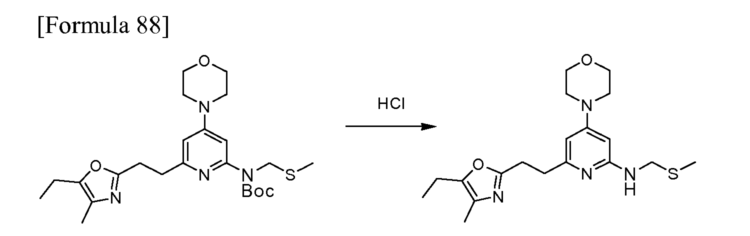

### 6-[2-(5-ethyl-4-methyl-1,3-oxazol-2-yl)ethyl]-N-[(methylthio)methyl]-4-(4-morpholinyl )-2-pyridinamine

A 4N hydrochloric acid-dioxane solution (0.50 mL) was added to [6-[2-(5-ethyl-4-methyl-1,3-oxazol-2-yl)ethyl]-4-(4-morpholinyl)-2-pyridinyl] [(methylt hio)methyl]carbamic acid tert-butyl ester (35.0 mg) obtained in Reference Example 2-75, and the mixture was stirred at room temperature for 45 minutes. The reaction solution was under reduced pressure, a saturated aqueous sodium hydrogencarbonate solution was added to the resultant residue, and the mixture was extracted with chloroform. The organic layer was washed with a saturated saline solution and dried over anhydrous sodium sulfate. Insoluble matters were filtered out, the filtrate was under reduced pressure, and the resultant residue was purified by NH silica gel column chromatography (hexane/ethyl acetate=9/1 to 1/2) to yield the title compound (11.0 mg) as a colorless oil.
¹ H-NMR(CDCl₃)δ:1.17(3H,t,J=7.4Hz),2.05(3H,s),2.15(3H,s),2.56(2H,q,J=7.4Hz),2.93 -3.09(4H,m),3.20-3.24(4H,m),3.79-3.83(4H,m),4.50(2H,d,J=6.7Hz),4.80-4.95(1H,m),5. 70(1H,d,J=2.0Hz),6.08(1H,d,J=2.0Hz).
mass:377(M+1)⁺.

### Example 57

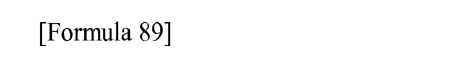

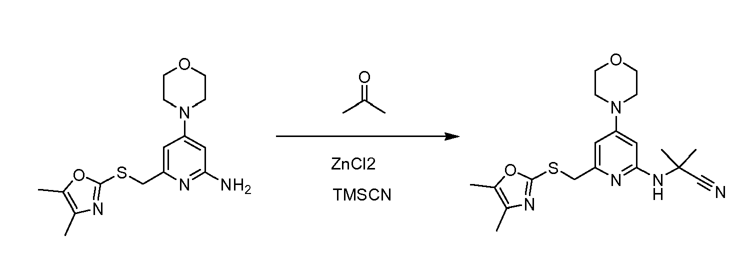

### 2-{[6-{[(4,5-dimethyl-1,3-oxazol-2-yl)thio]methyl}-4-(4-morpholinyl)-2-pyridinyl]ami no}-2-methylpropanenitrile

In acetonitrile (1.20 mL) was dissolved 6-{[(4,5-dimethyl-1,3-oxazol-2-yl)thio]methyl}-4-(4-morpholinyl)-2-pyridinamine (100 mg) obtained in Reference Example 2-56. To the solution were added acetone (46.0 µL), zinc (II) chloride (8.50 mg) and trimethylsilyl cyanide (84.0 µL), and the mixture was stirred overnight at 90°C. The temperature of the reaction solution was returned to room temperature, a saturated aqueous sodium hydrogencarbonate solution was added, and the mixture was extracted with ethyl acetate. The organic layer was washed with a saturated saline solution and dried over anhydrous sodium sulfate. Insoluble matters were filtered out, the filtrate was under reduced pressure, and the resultant residue was purified by NH silica gel column chromatography (hexane/ethyl acetate=1/1) to yield the title compound (27.8 mg) as a yellow solid.
¹ H-NMR(DMSO-d₆)δ:1.64(6H,s),1.98(3H,d,J=0.8Hz),2.19(3H,d,J=0.8Hz),3.09-3.15(4 H,m),3.65-3.71(4H,m),4.23(2H,s),5.85(1H,d,J=2.0Hz),6.44(1H,d,J=1.6Hz),6.71(1H,s).
mass:388(M+1)⁺.

### Industrial Applicability

Since an alkylaminopyridine derivative according to the present invention represented by the formula (I) or a pharmaceutically acceptable salt thereof has a potent antagonistic action to NPY, it is useful for treatment and/or prevention of various diseases associated with NPY, for example, cardiovascular diseases such as hypertension, arteriosclerosis, nephropathy, cardiac diseases and angiospasm; diseases of central nervous system such as bulimia, depression, epilepsy, anxiety, alcoholism and dementia; metabolic diseases such as obesity, diabetes mellitus and hormone abnormality, or glaucoma.

## Claims

1. A compound represented by formula (I): 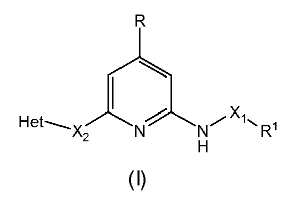 or a pharmaceutically acceptable salt thereof, wherein:
R¹ represents a group selected from the group consisting of:
hydrogen,
cyano,
trifluoromethyl,
halogen,
alkoxycarbonyl,
alkoxycarbonylamino,
lower alkylsulfonyl,
lower alkylsulfanilyl,
lower alkoxy, and
hydroxy;
R is a group represented by formula (II): 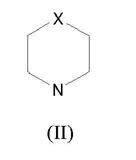 wherein X represents O, S or SO₂, or
a group represented by formula (III): 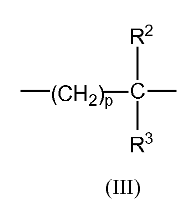 wherein R² and R³ are same or different hydrogen or halogen atoms; and p is an integer of 0 or 1, and
the group represented by the formula (II) is optionally crosslinked via a methylene or ethylene chain;
said R is optionally substituted with a group selected from the group consisting of: phenyl,
lower alkyl,
lower alkoxy,
hydroxy, and
halogen;
X₁ represents lower (C₁₋₄) alkylene,
said lower alkylene is optionally substituted with one or two same or different lower alkyl groups,
when the two same or different lower alkyl groups are bonded to the same carbon atom in the lower alkylene, these may together form a 3-7 membered cycloalkyl group, and when R¹ is lower alkoxycarbonylamino, nitrogen constituting the lower alkoxycarbonylamino and X₁ may together form a 5- or 6-membered aliphatic ring containing nitrogen;
X₂ represents C₂₋₆ lower alkylene, and one of carbon atoms constituting said lower alkylene is optionally substituted with an oxygen or sulfur atom, and the lower alkylene may be substituted with hydroxy;
Het represents a 5-membered heteroaromatic ring which has at least one nitrogen atom and, in addition, one or two hetero atoms selected from the group constituting of nitrogen, sulfur and oxygen, and
when said Het group is substituted with one or two same or different lower alkyl optionally substituted with halogen or lower alkoxy, or cycloalkyl groups, or when said Het is substituted with two alkyl groups, the two alkyl groups together may form 5-7 membered cycloalkyl.

2. The compound according to claim 1, or the pharmaceutically acceptable salt thereof, wherein X₂ is a C₂ or C₃ lower alkylene group optionally substituted with a hydroxy group or a C₁ or C₂ lower alkylene group in which one carbon atom which constitutes lower alkylene group, is replaced by sulfur atom.

3. The compound according to claim 1, or the pharmaceutically acceptable salt thereof, wherein Het is a group selected from the group consisting of thiazolyl, imidazolyl, oxazolyl, triazolyl and pyrazolyl groups.

4. The compound according to claim 1, or the pharmaceutically acceptable salt thereof, wherein X₁ is lower alkylene optionally substituted with lower alkyl.

5. The compound according to claim 1, or the pharmaceutically acceptable salt thereof, wherein R¹ is a group selected from the group consisting of cyano, trifluoromethyl, alkoxycarbonyl, lower alkylsulfonyl and lower alkoxy groups.

6. The compound according to claim 1, or the pharmaceutically acceptable salt thereof, wherein R is a group selected from the group consisting of formula (II-1):

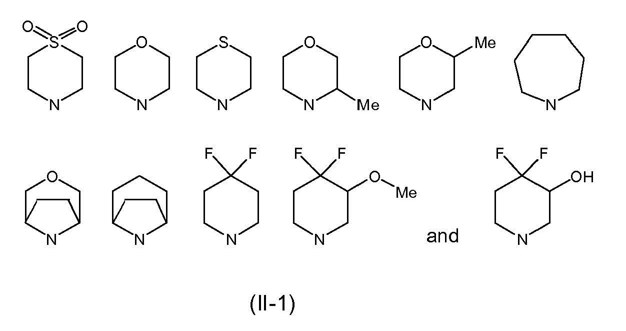

7. The compound according to claim 1, or the pharmaceutically acceptable salt thereof, wherein X₂ is a group selected from the group consisting of -CH₂, -CH₂-, -S-CH₂- and -CH₂-CH(OH)-.

8. The compound according to claim 1, or the pharmaceutically acceptable salt thereof, wherein R is a group selected from the group consisting of formula (II-2):

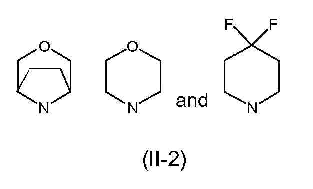

9. The compound according to claim 1, or the pharmaceutically acceptable salt thereof, wherein the compounds represented by the formula (I) is:
{[6-{[(4,5-dimethyl-1,3-thiazol-2-yl)thio]methyl}-4-(3-oxa-8-azabicyclo[3,2,1]octo-8-y 1)-2-pyridinyl]amino}acetonitrile,
6-{[(4,5-dimethyl-1,3-oxazol-2-yl)thio]methyl}-4-(3-oxa-8-azabicyclo[3,2,1]octo-8-yl)-N-(2,2,2-trifluoroethyl)-2-pyridinamine,
6-{[(1,5-dimethyl-1H-pyrazol-3-yl)thio]methyl}-4-(3-oxa-8-azabicyclo[3,2,1]octo-8-yl) -N-(2,2,2-trifluoroethyl)-2-pyridinamine,
6-{[(4,5-dimethyl-1,3-thiazol-2-yl)thio]methyl}-4-(3-oxa-8-azabicyclo[3,2,1]octo-8-yl)-N-(2,2,2-trifluoroethyl)-2-pyridinamine,
4-(4,4-difluoro-1-piperidinyl)-6-{[(1,5-diethyl-1H-1,2,4-triazol-3-yl)thio]methyl}-N-( 2,2,2-trifluoroethyl)-2-pyridinamine,
4-(4,4-difluoro-1-piperidinyl)-6-{[(5-ethyl-1-methyl-1H-1,2,4-triazol-3-yl)thio]methyl} -N-(2,2,2-trifluoroethyl)-2-pyridinamine,
4-(4,4-difluoro-1-piperidinyl)-6-{(1,2-diethyl-1H-imidazol-4-yl)thiol]methyl-N-(2,2 ,2-trifluoroethyl)-2-pyridinamine,
6-{(5-cyclopropyl-1-methyl-1H-1,2,4-triazol-3-yl)thio]methyl}-4-(4,4-difluoro-1-piper idinyl)-N-(2,2,2-trifluoroethyl)-2-pyridinamine,
4-(4,4-difluoro-1-piperidinyl)-6-{[(1,5-diethyl-1H-pyrazol-3-yl)thio]methyl}-N-(2,2,2 -trifluoroethyl)-2-pyridinamine,
[(4-(4,4-difluoro-1-piperidinyl)-6-{[(1,2-dimethyl-1H-imidazol-4-yl)thio]methyl}-2-per idinyl)amino]acetic acid ethyl ester,
6- {[(4,5-dimethyl-1,3-oxazol-2-yl)thio]methyl}-4-(1,1-dioxide-4-thiomorpholinyl)-N-(2 ,2,2-trifluoroethyl)-2-pyridinamine,
4-(1,1-dioxide-4-thiomorpholinyl)-6-[(4,5,6,7-tetrahydro-1,3-benzoxazo1-2-ylthio)meth yl]-N-(2,2,2-trifluoroethyl)-2-pyridinamine,
4-(1-azepanyl)-6-{[(1,2-dimethyl-1H-imidazol-4-yl)thio]methyl}-N-(2,2,2-trifluoroethy 1)-2-pyridinamine,
6-[2-(5-ethyl-4-methyl-1,3-oxazol-2-yl)ethyl]-N-(2-methoxyethyl)-4-(3-methyl-4-morp ho linyl)-2-pyridinamine,
6-[2-(5-ethyl-4-methyl-1,3-oxazol-2-yl)ethyl]-N-(2-methoxyethyl)-4-(2-methyl-4-morp ho linyl)-2-pyridinamine,
1-{2-[2-(5-ethyl-4-methyl-1,3-oxazol-2-yl)ethyl]-6-[(2-methoxyethyl)amino]-4-pyridin yl-4,4-difluoro-piperidinol,
4-(4,4-difluoro-3-methoxy-1-piperidinyl)-6-[2-(5-ethyl-4-methyl-1,3-oxazol-2-yl)ethyl] -N-(2-methoxyethyl)-2-pyridinamine,
4-(8-azabicyclo[3,2,1]octo-8-yl)-6-[2-(5-ethyl-4-methyl-1,3-oxazol-2-yl)ethyl]-N-(2-me thoxyethyl)-2-pyridinamine,
1-{4-(4,4-difluoro-1-piperidinyl)-6-[(2,2,2-trifluoroethyl)amino]-2-pyridinyl}-2-(4,5-di methyl-1,3-oxazol-2-yl)ethanol,
1- {4-(4,4-difluoro-1-piperidinyl)-6-[(2,2,2-trifluoroethyl)amino]-2-pyridinyl}-2-(5-ethy 1-4-methyl-1,3-oxazol-2-yl)ethanol,
2-(5-ethyl-4-methyl-1,3-oxazol-2-yl)-1-{4-(3-oxa-8-azabicyclo[3,2,1]octo-8-yl)-6-[(2,2, 2-trifluoroethyl)amino]-2-pyridinyl} ethanol,
2-(4,5-dimethyl-1,3-thiazol-2-yl)-1-{4-(3-oxa-8-azabicyclo[3,2,1]octo-8-yl)-6-[(2,2,2-tr ifluoroethyl)amino]-2-pyridinyl} ethanol,
2-(5-ethyl-4-methyl-1,3-oxazol-2-yl)-1-{4-(4-morpholinyl)-6-[(2,2,2-trifluoroethyl)ami no]-2-pyridinyl}ethanol,
2-(4,5-dimethyl-1,3-oxazol-2-yl)-1-{4-(4-thiomorpholinyl)-6-[(2,2,2-trifluoroethyl)ami no]-2-pyridinyl}ethanol,
{[6-{[(4,5-dimethyl-1,3-oxazol-2-yl)thio]methyl}-4-(3-oxa-8-azabicyclo[3,2,1]octo-8-y 1)-2-pyridinyl]amino}acetonitrile,
({4-(3-oxa-8-azabicyclo[3,2,1]octo-8-yl)-6-[(4,5,6,7-tetrahydro-1,3-benzoxazol-2-ylthio )methyl]-2-pyridinyl}amino)acetonitrile,
{[[6-[2-(5-ethyl-4-methyl-1,3-thiazo1-2-yl)ethyl]-4-(3-oxa-8-azabicyclo[3,2,1]octo-8-yl)-2-pyridinyl]amino}acetonitrile,
[(4-(4-morpholinyl)-6-{[(1,4,5-trimethyl-1H-imidazol-2-yl)thio]methyl}-2-pyridinyl)a mino]acetonitrile,
{[6-{[(4,5-dimethyl-1,3-thiazol-2-yl)thio]methyl}-4-(4-morpholinyl)-2-pyridinyl]amino }acetonitrile,
{[6-{[(4,5-dimethyl-1,3-oxazol-2-yl)thio]methyl}-4-(4-morpholinyl)-2-pyridinyl]amino }acetonitrile,
{[6-{[(5-ethyl-4-methyl-1,3-oxazol-2-yl)thio]methyl}-4-(4-morpholinyl)-2-pyridinyl]a mino} acetonitrile,
({4-(4,4-difluoro-1-piperidinyl)-6-[2-(1,5-dimethyl-1H-pyrazol-3-yl)ethyl]-2-pyridinyl} amino)acetonitrile,
({4-(4,4-difluoro-1-piperidinyl)-6-[2-(4,5-dimethyl-1,3-oxazol-2-yl)ethyl]-2-pyridinyl}a mino)acetonitrile,
[(4-(4-thiomorpholinyl)-6-{[(1,4,5-trimethyl-1H-imidazol-2-yl)thio]methyl}-2-pyridiny 1)amino]acetonitrile,
2-{[6-[2-(5-ethyl-4-methyl-1,3-oxazol-2-yl)ethyl]-4-(4-morpholinyl)-2-piperidinyl]ami no}propanenitrile,
3-{[6-[2-(5-ethyl-4-methyl-1,3-oxazol-2-yl)ethyl]-4-(4-morpholinyl)-2-pyridinyl]amino }propanenitrile,
6-{[(4,5-dimethyl-1,3-oxazol-2-yl)thio]methyl}-N-(3-methoxypropyl)-4-(4-morpholinyl )-2-pyridinamine,
{[6-{[(4,5-dimethyl-1,3-thiazol-2-yl)thio]methyl}-4-(3-oxa-8-azabicyclo[3,2,1]octo-8-y 1)-2-pyridinyl]amino}acetic acid methyl ester,
4-(4-morpholinyl)-N-(2,2,2-trifluoroethyl)-6-{[(1,4,5-trimethyl-1H-imidazol-2-yl)thio] methyl}-2-pyridinamine,
6-{[(4,5-dimethyl-1,3-oxazol-2-yl)thio]methyl}-4-(4-morpholinyl)-N-(2,2,2-trifluoroeth yl)-2-pyridinamine,
6-{[(1,2-dimethyl-1H-imidazol-4-yl)thio]methyl}-N-(2-methoxyethyl)-4-(4-morpholiny 1)-2-pyridinamine,
N-(2-methoxyethyl)-6-({[5-(methoxymethyl)-4-methyl-1,3-oxazol-2-yl]thio}methyl)-4-(4-morpholinyl)-2-pyridinamine,
4-(4,4-difluoro-1-piperidinyl)-6-({[1-(fluoromethyl)-5-methyl-1H-1,2,4-triazol-3-yl]thi o}methyl)-N-(2,2,2-trifluoroethyl)-2-pyridinamine,
4-(4,4-difluoro-1-piperidinyl)-6-{[(1-ethyl-5-methyl-1H-1,2,4-triazol-3-yl)thio]methyl} -N-(2,2,2-trifluoroethyl)-2-pyridinamine,
6-{[(1,5-dimethyl-1H-1,2,4-triazol-3-yl)thio]methyl}-4-(4-thiomorpholmyl)-N-(2,2,2-tri fluoroethyl)-2-pyridinamine,
6-{[(1,2-dimethyl-1H-imidazol-4-yl)thio]methyl}-4-(4-thiomorpholinyl)-N-(2,2,2-triflu oroethyl)-2-pyridinamine,
6-{[(1-cyclopropyl-5-methyl-1H-1,2,4-triazol-3-yl)thio]methyl}-4-(4,4-difluoro-1-piper idinyl)-N-(2,2,2-trifluoroethyl)-2-pyridinamine,
4-(4,4-difluoro-1-piperidinyl)-6-{[(4,5-dimethyl-1,3-oxazol-2-yl)thio]methyl}-N-[2-(me thylsulfonyl)ethyl]-2-pyridinamine,
6-{[(4,5-dimethyl-1,3-oxazol-2-yl)thio]methyl}-N-[2-(methylsulfonyl)ethyl]-4-(4-thiom orpholinyl)-2-pyridinamine,
6-{[(4,5-dimethyl-1,3-thiazol-2-yl)thio]methyl}-4-(1,1-dioxide)-4-thiomorpholinyl)-N-( 2,2,2-trifluoroethyl)-2-pyridinamine,
6-{[(1,5-dimethyl-1H-pyrazol-3-yl)thio]methyl}-4-(1,1-dioxide-4-thiomorpholinyl)-N-( 2,2,2-trifluoroethyl)-2-pyridinamine,
4-(1,1-dioxide-4-thiomorpholinyl)-6-{[(5-ethyl-4-methyl-1,3-oxazol-2-yl)thio]methyl}-N-(2,2,2-trifluoroethyl)-2-pyridinamine,
N-cyclopropyl-6-{(4,5-dimethyl-1,3-oxazol-2-yl)thio]methyl}-4-(4-morpholinyl)-2-pyr idinamine,
2-{[6-{[(5-ethyl-4-methyl-1,3-oxazol-2-yl)thio]methyl}-4-(4-morpholinyl)-2-pyridinyl] amino}ethanol,
3-({[6-{[(4,5-dimethyl-1,3-oxazol-2-yl)thio]methyl)-4-(4-morpholinyl)-2-pyridinyl]ami no}methyl)-1-piperidine carboxylic acid methyl ester,
6-[2-(5-ethyl-4-methyl-1,3-oxazol-2-yl)ethyl]-N-[(methylthio)methyl]-4-(4-morpholinyl )-2-pyridinamine or
2-{[6-{[(4,5-dimethyl-1,3-oxazol-2-yl)thio]methyl}-4-(4-morpholinyl)-2-pyridinyl]ami no}-2-methylpropanenitrile.

10. The compound according to claim 1, or a pharmaceutically acceptable salt thereof, wherein a compound represented by the formula (I) is:
{[6-{[(4,5-dimethyl-1,3-thiazol-2-yl)thio]methyl}-4-(3-oxa-8-azabicyclo[3,2,1]octo-8-y 1)-2-pyridinyl]amino}acetonitrile,
4-(4,4-difluoro-1-piperidinyl)-6-{[(1,5-dimethyl-1H-1,2,4-triazol-3-yl)thio]methyl}-N-( 2,2,2-trifluoroethyl)-2-pyridinamine,
4-(4,4-difluoro-1-piperidinyl)-6-{[(5-ethyl-1-methyl-1H-1,2,4-triazol-3-yl)thio]methyl} -N-(2,2,2-trifluoroethyl)-2-pyridinamine,
4-(4,4-difluoro-1-piperidinyl)-6-{[(1,5-dimethyl-1H-pyrazol-3-yl)thio]methyl}-N-(2,2,2 -trifluoroethyl)-2-pyridinamine,
6-{[(4,5-dimethyl-1,3-oxazol-2-yl)thio]methyl}-4-(1,1-dioxide-4-thiomorpholinyl)-N-(2 ,2,2-trifluoroethyl)-2-pyridinamine,
1-{4-(4,4-difluoro-1-piperidinyl)-6-[(2,2,2-trifluoroethyl)amino]-2-pyridinyl}-2-(4,5-di methyl-1,3-oxazol-2-yl)ethanol,
2-(5-ethyl-4-methyl-1,3-oxazol-2-yl)-1-{4-(3-oxa-8-azabicyclo[3,2,1]octo-8-yl)-6-[(2,2, 2-trifluoroethyl)amino]-2-pyridinyl}ethanol,
2-(5-ethyl-4-methyl-1,3-oxazol-2-yl)-1-{4-(4-morpholinyl)-6-[(2,2,2-trifluoroethyl)ami no]-2-pyridinyl}ethanol,
({[6-{[(4,5-dimethyl-1,3-oxazol-2-yl)thio]methyl}-4-(3-oxa-8-azabicyclo[3,2,1]octo-8-yl)-2-pyridinyl]amino}acetonitrile,
({4-(3-oxa-8-azabicyclo[3,2,1]octo-8-yl)-6-[(4,5,6,7-tetrahydro-1,3-benzoxazol-2-ylthio )methyl]-2-pyridinyl}amino)acetonitrile,
{[6-{[(4,5-dimethyl-1,3-thiazol-2-yl)thio]methyl}-4-(4-morpholinyl)-2-pyridinyl]amino }acetonitrile,
{[6-{[(4,5-dimethyl-1,3-oxazol-2-yl)thio]methyl}-4-(4-morpholinyl)-2-pyridinyl]amino }acetonitrile,
({4-(4,4-difluoro-1-piperidinyl)-6-[2-(1,5-dimethyl-1H-pyrazol-3-yl)ethyl]-2-pyridinyl} amino)acetonitrile,
4-(4,4-difluoro-1-piperidinyl)-6-({[1-(fluoromethyl)-5-methyl-1H-1,2,4-triazol-3-yl]thi o}methyl)-N-(2,2,2-trifluoroethyl)-2-pyridinamine,
4-(4,4-difluoro-1-piperidinyl)-6-{[(1-ethyl-5-methyl-1H-1,2,4-triazol-3-yl)thio]methyl}
-N-(2,2,2-trifluoroethyl)-2-pyridinamine,
6-{[(1,5-dimethyl-1H-1,2,4-triazol-3-yl)thio]methyl}-4-(4-thiomorpholinyl)-N-(2,2,2-tri fluoroethyl)-2-pyridinamine,
6-{[(1-cyclopropyl-5-methyl-1H-1,2,4-triazol-3-yl)thio]methyl}-4-(4,4-difluoro-1-piper idinyl)-N-(2,2,2-trifluoroethyl)-2-pyridinamine,
6-{[(4,5-dimethyl-1,3-thiazol-2-yl)thio]methyl}-4-(1,1-dioxide-4-thiomorpholinyl)-N-( 2,2,2-trifluoroethyl)-2-pyridinamine or
2-{[6-{[(4,5-dimethyl-1,3-oxazol-2-yl)thio]methyl}-4-(4-morpholinyl)-2-pyridinyl]ami no}-2-methylpropanenitrile.

11. A neuropeptide Y receptor antagonist comprising the compound according to any one of claims 1 to 10 or the pharmaceutically acceptable salt thereof as an active ingredient.

12. A pharmaceutical composition comprising the compound according to any one of claims 1 to 10 or the pharmaceutically acceptable salt thereof as an active ingredient.

13. An agent for treating bulimia, obesity or diabetes mellitus, comprising the compound according to any one of claims 1 to 10 or the pharmaceutically acceptable salt thereof as an active ingredient.
